(19) Europäisches Patentamt
European Patent Office
Office européen des brevets

(11) EP 3 344 659 B1

(12) **EUROPEAN PATENT SPECIFICATION**

(45) Date of publication and mention of the grant of the patent:
**26.03.2025 Bulletin 2025/13**

(21) Application number: **16840684.1**

(22) Date of filing: **18.07.2016**

(51) International Patent Classification (IPC):
***C07K 19/00*** (2006.01) ***A61P 7/02*** (2006.01)
***C07K 16/18*** (2006.01) ***A61K 39/44*** (2006.01)
***A61K 47/60*** (2017.01) ***C07K 7/08*** (2006.01)

(52) Cooperative Patent Classification (CPC):
**A61K 47/56; A61K 31/137; A61K 31/436; A61K 31/4545; A61K 38/05; A61K 39/44; A61K 47/55; A61K 47/65; A61K 47/6843; C07K 7/08; C07K 16/2866; C07K 16/36; C12N 9/6459; C12Y 304/21068;** C07K 2317/52;
(Cont.)

(86) International application number:
**PCT/CN2016/090296**

(87) International publication number:
**WO 2017/036255 (09.03.2017 Gazette 2017/10)**

(54) **MOLECULAR CONSTRUCTS FOR PREVENTING THE FORMATION OF BLOOD CLOT AND/OR TREATING THROMBOSIS**

MOLEKULARE KONSTRUKTE ZUR VERHINDERUNG DER BILDUNG VON BLUTGERINNSELN UND/ODER ZUR BEHANDLUNG VON THROMBOSEN

CONSTRUCTIONS MOLÉCULAIRES DESTINÉES À PRÉVENIR LA FORMATION D'UN CAILLOT SANGUIN ET/OU À TRAITER LA THROMBOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2015 US 201561213012 P**
**18.01.2016 PCT/CN2016/071184**
**15.03.2016 US 201662308349 P**

(43) Date of publication of application:
**11.07.2018 Bulletin 2018/28**

(73) Proprietor: **Immunwork Inc.**
**Taipei City 115 (TW)**

(72) Inventors:
- **Chang, Tse-Wen**
  **Taipei, Taiwan 115 (TW)**
- **Chu, Hsing-Mao**
  **Taipei, Taiwan 115 (TW)**
- **Lin, Chun-Yu**
  **Taipei City 115 (TW)**
- **Tian, Wei-Ting**
  **Taipei City 115 (TW)**

(74) Representative: **Lang, Christian**
**LangPatent Anwaltskanzlei IP Law Firm**
**Ingolstädter Straße 5**
**80807 München (DE)**

(56) References cited:
**WO-A1-2008/017122** **WO-A1-2011/045668**
**WO-A1-2013/123591** **WO-A1-2016/112870**
**WO-A2-2011/029008**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHAUDHARY V K ET AL: "A RAPID METHOD OF CLONING FUNCTIONAL VARIABLE-REGION ANTIBODY GENESIN ESCHERICHIA COLI AS SINGLE-CHAIN IMMUNOTOXINS", PROCEEDINGS OF THE NATIONAL ACADEMY OF SCIENCES OF THE UNITED STATES OF AMERICA, NATIONAL ACADEMY OF SCIENCES, US, vol. 87, no. 3, 1 February 1990 (1990-02-01), pages 1066 - 1070, XP000095668, ISSN: 0027-8424, DOI: 10.1073/PNAS.87.3.1066**
- **PETER K ET AL: "Construction and functional evaluation of single-chain antibody fusion protein with fibrin targeting and thrombin ohibition after activation by factor Xa", CIRCULATION, AMERICAN HEART ASSOCIATION, US, vol. 101, 14 March 2000 (2000-03-14), pages 1158 - 1164, XP002204646, ISSN: 0009-7322**


Remarks:

The complete document including Reference Table(s) and the Sequence Listing(s) can be downloaded from the EPO website

(52) Cooperative Patent Classification (CPC): (Cont.)
C07K 2317/622; C07K 2319/00; C07K 2319/035; C07K 2319/30

## Description

## BACKGROUND OF THE INVENTION

1. Field of the Invention

The present disclosure relates to the field of pharmaceuticals; more particularly, to multi-functional molecular constructs, e.g., those having targeting and effector elements for delivering the effector (e.g., therapeutic drug) to targeted sites.

2. Description of the Related Art

The continual advancement of a broad array of methodologies for screening and selecting monoclonal antibodies (mAbs) for targeted antigens has helped the development of a good number of therapeutic antibodies for many diseases that were regarded as untreatable just a few years ago. According to Therapeutic Antibody Database, approximately 2,800 antibodies have been studied or are being planned for studies in human clinical trials, and approximately 80 antibodies have been approved by governmental drug regulatory agencies for clinical uses. The large amount of data on the therapeutic effects of antibodies has provided information concerning the pharmacological mechanisms how antibodies act as therapeutics.

One major pharmacologic mechanism for antibodies acting as therapeutics is that, antibodies can neutralize or trap disease-causing mediators, which may be cytokines or immune components present in the blood circulation, interstitial space, or in the lymph nodes. The neutralizing activity inhibits the interaction of the disease-causing mediators with their receptors. It should be noted that fusion proteins of the soluble receptors or the extracellular portions of receptors of cytokines and the Fc portion of IgG, which act by neutralizing the cytokines or immune factors in a similar fashion as neutralizing antibodies, have also been developed as therapeutic agents.

Several therapeutic antibodies that have been approved for clinical applications or subjected to clinical developments mediate their pharmacologic effects by binding to receptors, thereby blocking the interaction of the receptors with their ligands. For those antibody drugs, Fc-mediated mechanisms, such as antibody-dependent cellular cytotoxicity (ADCC) and complement-mediated cytolysis (CMC), are not the intended mechanisms for the antibodies.

Some therapeutic antibodies bind to certain surface antigens on target cells and render Fc-mediated functions and other mechanisms on the target cells. The most important Fc-mediated mechanisms are antibody-dependent cellular cytotoxicity (ADCC) and complement-mediated cytolysis (CMC), which both will cause the lysis of the antibody-bound target cells. Some antibodies binding to certain cell surface antigens can induce apoptosis of the bound target cells.

The concept and methodology for preparing antibodies with dual specificities germinated more than three decades ago. In recent year, the advancement in recombinant antibody engineering methodologies and the drive to develop improved medicine has stimulated the development bi-specific antibodies adopting a large variety of structural configurations.

For example, the bi-valent or multivalent antibodies may contain two or more antigen-binding sites. A number of methods have been reported for preparing multivalent antibodies by covalently linking three or four Fab fragments via a connecting structure. For example, antibodies have been engineered to express tandem three or four Fab repeats.

Several methods for producing multivalent antibodies by employing synthetic crosslinkers to associate, chemically, different antibodies or binding fragments have been disclosed. One approach involves chemically cross-linking three, four, and more separately Fab fragments using different linkers. Another method to produce a construct with multiple Fabs that are assembled to one-dimensional DNA scaffold was provided. Those various multivalent Ab constructs designed for binding to target molecules differ among one another in size, half-lives, flexibility in conformation, and ability to modulate the immune system. In view of the foregoing, several reports have been made for preparing molecular constructs with a fixed number of effector elements or with two or more different kinds of functional elements (e.g., at least one targeting element and at least one effector element). For example, Chaudhary V K et al. (in "A rapid method of cloning functional variable-region antibody genes in Escherichia coli as single-chain immunotoxins"; Proc Natl Acad Sci U S A., 1990 Feb; 87(3): 1066-1070) discloses an antibody OVB3, in which the light chain and heavy chain sequences are linked by a peptide linker. Document WO 2011/029008 A2 teaches a polypeptide having a plurality of lysine residues for linking cancer-associated peptides. Document WO 2011/045668 A1 discloses a salmon calcitonin peptide (sCT) comprising three K residues for linking bone targeting moiety (BP) via linker NHS-PEG-MAL. Further, Peter K et al. (in "Construction and functional evaluation of a single-chain antibody fusion protein with fibrin targeting and thrombin inhibition after activation by factor Xa"; CIRCULATION, 2000 March; 101: 1158-1164) discloses a fusion protein comprising an anti-fibrin scFv and an inhibitor of thrombin, wherein the anti-fibrin scFv and the inhibitor of thrombin are linked by a linker $(G_4S)_3$ through the formation of amide bonds. Document WO 2008/017122 A1 teaches a dendrimer comprising lysine residues for attaching functional moieties; and document WO 2013/123591 A1 teaches a multimer of a single domain antibody

(sdAb), wherein the sdAb has binding affinity to prothrombin, and is linked together by one or more disulfide bonds. However, it is often difficult to build a molecular construct with a particular combination of the targeting and effector elements either using chemical synthesis or recombinant technology. Accordingly, there exists a need in the related art to provide novel molecular platforms to build a more versatile molecule suitable for covering applications in a wide range of diseases.

## SUMMARY

### < I > Peptide Core-Based Multi-Arm Linkers

The present disclosure is directed to a linker unit that has at least two different functional elements linked thereto. For example, the linker unit may have linked thereto two different effector elements, one targeting element and one effector element, or one effector element and a polyethylene glycol (PEG) chain for prolonging the circulation time of the linker unit. The present linker unit is designed to have at least two different functional groups such that the functional elements can be linked thereto by reacting with the respective functional groups. Accordingly, the present linker unit can serve as a platform for preparing a molecular construct with two or more functional elements.

According to the present invention, a linker unit as defined in claim 1 is provided. The linker unit comprises a center core and a plurality of linking arms. The center core is a polypeptide core comprising a plurality of lysine (K) resides, in which each K residue and a next K residue are separated by a filler sequence comprising glycine (G) and serine (S) residues, and the number of K residues ranges from 2 to 15. Optionally, the filler sequence consists of 2 to 20 amino acid residues. The filler sequence may have the sequence of GS, GGS, GSG, or SEQ ID NOs: 1-16. The center core comprises 2-15 units of the sequence of $G_{1-5}SK$; preferably, the center core comprises the sequence of $(GSK)_{2-15}$. Each of the linking arms is linked to the K residues of the center core. The amino acid residue at the N- or C-terminus of the center core has an azide group or an alkyne group; alternatively or additionally, the amino acid residue at the N- or C-terminus of the center core is a cysteine (C) residue, in which the thiol group of the amino acid residue is linked with a coupling arm having an azide group, an alkyne group, a tetrazine group, a cyclooctene group, or a cyclooctyne group at the free terminus of the coupling arm.

According to the present disclosure, the linker unit further comprises a plurality of first elements. The linker unit is produced by linking the plurality of first elements to the plurality of linking arms, in which each of the first elements is linked to one of the linking arms via forming an amide bound between the linking arm and the first element. In other embodiments, the linker unit is produced by linking the plurality of first elements to the plurality of linking arms, in which each of the first elements is linked to one of the linking arms via thiol-maleimide reaction, copper catalyzed azide-alkyne cycloaddition (CuAAC) reaction, strained-promoted azide-alkyne click chemistry (SPAAC) reaction, or inverse electron demand Diels-Alder (iEDDA) reaction occurred between the linking arm and the first element.

In the present disclosure, the linking arm is a PEG chain having 2 to 20 repeats of EG units and the coupling linking arm is a PEG chain having 2 to 12 repeats of EG units.

Regarding amino acid residues having the azide group, non-limiting examples of said amino acid residues include L-azidohomoalanine (AHA), 4-azido-L-phenylalanine, 4-azido-D-phenylalanine, 3-azido-L-alanine, 3-azido-D-alanine, 4-azido-L-homoalanine, 4-azido-D-homoalanine, 5-azido-L-ornithine, 5-azido-d-ornithine, 6-azido-L-lysine, and 6-azido-D-lysine. As to the amino acid residues having the alkyne group, illustrative examples thereof include L-homopropargylglycine (L-HPG), D-homopropargylglycine (D-HPG), and beta-homopropargylglycine (β-HPG).

When the amino acid residues at the N- or C-terminus of the center core is the cysteine residue, the cyclooctene group at the free terminus of the coupling arm may be, a trans-cyclooctene (TCO) group, while the cyclooctyne group at the free terminus of the coupling arm may be a dibenzocyclooctyne (DBCO), difluorinated cyclooctyne (DIFO), bicyclononyne (BCN), or dibenzocyclooctyne (DICO) group. Alternatively, the tetrazine group at the free terminus of the coupling arm includes, but is not limited to, 1,2,3,4-tetrazine, 1,2,3,5-tetrazine, and 1,2,4,5-tetrazine, and derivatives thereof, such as, 6-methyl tetrazine.

According to the present disclosure, the first element is a single-chain variable fragment (scFv) specific for fibrin.

Optionally, the linker unit further comprises a second element that is different from the first elements. In some examples, the linker unit is produced by linking the second element to the center core, in which the second element has an azide or alkyne group, so that it is linked to the center core or the coupling arm by coupling with the corresponding alkyne or azide group of the center core or the coupling arm via CuAAC reaction. Alternatively, in some examples, the linker unit is produced by linking the second element to the center core, in which the second element having an azide or cyclooctyne group is linked to the center core or the coupling arm by coupling with the corresponding cyclooctyne or azide group of the center core or the coupling arm via SPAAC reaction. Still alternatively, in certain examples, the linker unit is produced by linking the second element to the center core, in which the second element having a tetrazine or cyclooctene group is linked to the center core or the coupling arm by coupling with the corresponding cyclooctene or tetrazine group of the center core or the coupling arm via iEDDA reaction. According to the examples of the present disclosure, the second element is a

tissue plasminogen activator or an inhibitor of Factor Xa or thrombin. Non-limiting examples of the tissue plasminogen activators include, alteplase, reteplase, tenecteplase, and lanoteplase. The inhibitor of Factor Xa is selected from the group consisting of, apixaban, edoxaban, and rivaroxaban. The inhibitor of thrombin can be argatroban or melagatran.

< **II** > **Molecular Constructs with Targeting and Effector Moieties**

The present disclosure is further directed to a molecular construct comprising two linker units coupling to each other either directly or indirectly, in which the core of one linker unit is configured to be linked with at least one targeting element while the core of the other linker unit is configured to be linked with at least one effector element. The present molecular construct is advantageous in that the two linker units are coupled to each other via an iEDDA reaction, a SPAAC reaction, or a CuAAC reaction. This design allows for a facile synthesis of a molecular construct with a complex structure. According to the principles and spirits of the present disclosure, the two linker units respectively carrying different numbers and/or types of functional elements can be independently prepared, and then conjugated together. In this way, it becomes feasible for a skilled artisan to construct libraries of molecular constructs respectively carrying different functional elements, and then select and combine two molecular constructs (or linker units) from the libraries to generate a desired constructs, depending on the needs and/or intended applications. Moreover, the number of functional elements per linker unit may be controlled by adjusting the number of specific functional group(s) of the core.

According to the present invention, a molecular construct as defined in claim 10 is provided. The molecular construct comprises a first linker unit and a second linker unit. Specifically, the first linker unit comprises (1) a first center core, (2) one or more linking arms (hereinafter, the first linking arms) linked to the first center core, (3) one or more elements (hereinafter, the first elements) linked to the first linking arm(s), and (4) optionally a coupling arm (hereinafter, the first coupling arm) linked to the first center core; the second linker unit comprises (1) a second center core, (2) one or more linking arms (hereinafter, the second linking arms) linked to the second center core, (3) one or more elements (hereinafter, the second elements) linked to the second linking arm(s), and (4) optionally a coupling arm (hereinafter, the second coupling arm) linked to the second center core. The first and second linker units are coupled to each other via iEDDA, SPAAC, or CuAAC reaction occurred between any of the followings: the first and second center cores, the first coupling arm and the second center core, the first and second coupling arms, or the first center core and the second coupling arm.

According to the present disclosure, both the first and second center cores have a plurality of lysine (K) residues. Each of the linking arms is linked to K residues of the center core. After being linked to the center core, the linking arm thus has an NHS, a maleimide, an azide, an alkyne, a tetrazine, a cyclooctene, or a cyclooctyne group at the free terminus thereof.

In the presence of the NHS group, the first element and the second element are respectively linked to the first and second linking arms via forming an amide bond between the element (i.e., the first element and the second element) and the linking arm (i.e., the first linking arm or the second linking arm). In the case where the linking arm has a maleimide, an azide, an alkyne, a tetrazine, a cyclooctene, or a cyclooctyne group at its free terminus, the first element and the second element are respectively linked to the first and second linking arms via the thiol-maleimide, CuAAC, iEDDA, or SPAAC reaction occurred between the element (i.e., the first element and the second element) and the linking arm (i.e., the first linking arm or the second linking arm).

According to the present disclosure, each of the linking arms is a PEG chain having 2-20 repeats of EG units; and each of the coupling arms is a PEG chain having 2-12 repeats of EG units.

According to some embodiments of the present disclosure, the present polypeptide core comprises a plurality of lysine (K) residues; optionally, 2 to 15 K residues. Also, each K residue and the next K residue are separated by a filler sequence comprising glycine (G) and serine (S) residues; optionally, the filler sequence consists of 2 to 20 amino acid residues. In various examples, the filler sequence may have the sequence of GS, GGS, GSG, or SEQ ID NOs: 1-16. In some examples, the polypeptide comprises 2-15 units of the sequence of $G_{1-5}SK$, for example, $(GSK)_{2-15}$.

The center core may comprise a cysteine residue at its N- or C-terminus. In these instances, the coupling arm is linked to the cysteine residue of the center core via the thiol-maleimide reaction. The coupling arm linked to the cysteine residue has an azide, an alkyne, a cyclooctene, a cyclooctyne, or a tetrazine group at the free-terminus thereof.

The first and second linker units may be coupled via various configurations, which are described in detail below, depending on the presence or absence of the first and second coupling arms..

When the first and second linker units respectively comprise the coupling arms, then one of the coupling arms (say, for example, the first coupling arm) has a tetrazine group at the free-terminus thereof, and the other coupling arm (in this case, the second coupling arm) has a cyclooctene group at the free-terminus thereof, such that the two linker units are coupled via the iEDDA reaction occurred between the two coupling arms (i.e., the first and second coupling arms). Preferably, the tetrazine group is 1,2,3,4-tetrazine, 1,2,3,5-tetrazine, and 1,2,4,5-tetrazine, or derivatives thereof, such as, 6-methyl tetrazine; and the cyclooctene group is TCO. The same rule also applies in the case where the free termini of both coupling arms respectively have an azide group and an alkyne group; in this instance, the two linker units are coupled via the CuAAC reaction occurred between the two coupling arms (i.e., the first and second coupling arms). Alternatively, one of

the coupling arms (for example, the first coupling arm) has an azide group, and the other coupling arm (in this case, the second coupling arm) has a cyclooctyne group (preferably, DBCO, DIFO, BCN, or DICO); accordingly, the two coupling arm can be coupled via the SPAAC reaction.

When only one linker unit has the coupling arm (as an example, the first linker unit with the first coupling arm), the center core of the other linker unit (for example, the second center core) is a polypeptide core. In this case, the first amino acid residue at the N- or C-terminus of one of the second center core is an amino acid residue having an azide group or an alkyne group. In some examples, the amino acid residue having the azide or alkyne group would undergo CuAAC reaction with the corresponding alkyne or azide group of the first coupling arm of the first linker unit, thereby coupling the first and second linker units. Alternatively, the first amino acid residue at the N- or C-terminus of one of the second center core is an amino acid residue having an azide group, which can be linked to the coupling arm of the first linker unit having a cyclooctyne group (preferably, DBCO, DIFO, BCN, or DICO) at the free-terminus via the SPAAC reaction.

It is also possible that the first and second linker units are coupled without the presence of any coupling arms (that is, the first and second coupling arms). In other words, the first and second coupling arms are directly linked with each other. This configuration mostly occurs between two polypeptide cores. Specifically, one of the two center cores (say, for example, the first center core) has an amino acid residue having an azide group at the N- or C-terminus thereof, while the other center core (such as the second center core) has an amino acid residue having an alkyne group at the N- or C-terminus thereof. In this way, the azide group of the first center core reacts with the alkyne group of the second center core, thereby coupling the first and second linker units.

Non-limiting examples of amino acid residues having the azide group include, L-azidohomoalanine (AHA), 4-azido-L-phenylalanine, 4-azido-D-phenylalanine, 3-azido-L-alanine, 3-azido-D-alanine, 4-azido-L-homoalanine, 4-azido-D-homoalanine, 5-azido-L-ornithine, 5-azido-d-ornithine, 6-azido-L-lysine, and 6-azido-D-lysine. Illustrative examples of amino acid residues having the alkyne group include, but are not limited to, L-homopropargylglycine (L-HPG), D-homopropargylglycine (D-HPG), and beta-homopropargylglycine (β-HPG).

According to the present disclosure, the first and second center cores may be the same or different.

According to the present disclosure, the first element is an scFv specific for fibrin, and the second element is the inhibitor of Factor Xa or thrombin. The inhibitor of Factor Xa is selected from the group consisting of, apixaban, edoxaban, and rivaroxaban. The inhibitor of thrombin can be argatroban or melagatran.

According to other examples of the present disclosure, the linker units comprise an scFv specific for fibrin as the first element, and a tissue plasminogen activator as the second element. Non-limiting examples of the tissue plasminogen activators include, alteplase, reteplase, tenecteplase, and lanoteplase.

## BRIEF DESCRIPTION OF THE DRAWINGS

The present description will be better understood from the following detailed description read in light of the accompanying drawings briefly discussed below.

Figure 1A to Figure 1I are schematic diagrams illustrating linker units according to certain examples of the present disclosure.

Figure 2A to Figure 2D are schematic diagrams illustrating T-E molecular constructs according to some examples of the present disclosure.

Figure 3 is a schematic diagram that illustrates libraries for constructing molecular constructs according to some examples of the present disclosure.

Figure 4A and Figure 4B are schematic diagrams that illustrate molecular constructs according to some examples of the present disclosure.

Figure 5 shows the mass spectrometry MALDI-TOF result of a peptide core-based linker-unit carrying one linking arm with TCO group and five PEG6 linking arms with maleimide groups, according to one working example of the present disclosure.

Figure 6 shows the mass spectrometry MALDI-TOF result of the apixaban-$PEG_3$-S-S-$PEG_3$-apixaban synthetized according to one working example of the present disclosure.

Figure 7 shows the mass spectrometry MALDI-TOF result of the argatroban-$PEG_3$-S-S-$PEG_3$-argatroban synthetized according to one working example of the present disclosure.

Figures 8A, 8B, and 8C respectively show the results of SDS-PAGE, MALDI-TOF and ELISA analysis of purified 102-10 scFv specific for human fibrin, according to one working example of the present disclosure.

Figure 9A and Figure 9B respectively show the results of phage titer analysis and single colony ELISA analysis of phage-displayed scFvs specific for human fibrin, according to one working example of the present disclosure.

Figure 10A and Figure 10B respectively show the results of SDS-PAGE and MALDI-TOF analysis of purified reteplase, according to one working example of the present disclosure.

Figure 11 shows the result of mass spectrometric analysis of TCO-conjugated reteplase, according to one working example of the present disclosure.

Figure 12 shows the result of SDS-PAGE analysis of a targeting linker-unit with one free tetrazine functional group and a set of three scFvs specific for human fibrin, according to one working example of the present disclosure.

Figure 13 shows the MS result on a single linker-unit molecular construct with three scFvs specific for human fibrin (as targeting elements) and one reteplase molecule (as the effector element), according to one working example of the present disclosure.

Figure 14 shows the result of inhibition assay of apixaban-$PEG_3$-SH molecule, according to one working example of the present disclosure.

Figure 15 shows the result of SDS-PAGE analysis of purified recombinant 2-chain (reteplase)-hgG4.Fc fusion protein, according to one working example of the present disclosure.

Figure 16 shows the result of SDS-PAGE analysis of purified recombinant 2-chain (reteplase)-hIgG4.Fc-(scFv α fibrin) fusion protein, according to one working example of the present disclosure.

Figure 17 shows the result of SDS-PAGE analysis of purified recombinant 2-chain (reteplase)-(scFv α fibrin) fusion protein, according to one working example of the present disclosure.

Figure 18 shows the result of SDS-PAGE analysis of purified recombinant 2-chain (TNK-tPA)-IgG4.Fc fusion protein, according to one working example of the present disclosure.

Figure 19 shows the assay results of protease activity of recombinant 2-chain (reteplase)-hIgG4.Fc, 2-chain (reteplase)-hIgG4.Fc-(scFv α fibrin) and (reteplase)-(scFv α fibrin), according to one working example of the present disclosure.

In accordance with common practice, the various described features/elements are not drawn to scale but instead are drawn to best illustrate specific features/elements relevant to the present invention. Also, like reference numerals and designations in the various drawings are used to indicate like elements/parts, where possible.

## DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

For convenience, certain terms employed in the specification, examples and appended claims are collected here. Unless otherwise defined herein, scientific and technical terminologies employed in the present disclosure shall have the meanings that are commonly understood and used by one of ordinary skill in the art.

Unless otherwise required by context, it will be understood that singular terms shall include plural forms of the same and plural terms shall include the singular. Specifically, as used herein and in the claims, the singular forms "a" and "an" include the plural reference unless the context clearly indicated otherwise. Also, as used herein and in the claims, the terms "at least one" and "one or more" have the same meaning and include one, two, three, or more. Furthermore, the phrases "at least one of A, B, and C", "at least one of A, B, or C" and "at least one of A, B and/or C," as use throughout this specification and the appended claims, are intended to cover A alone, B alone, C alone, A and B together, B and C together, A and C together, as well as A, B, and C together.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques. Ranges can be expressed herein as from one endpoint to another endpoint or between two endpoints. All ranges disclosed herein are inclusive of the endpoints, unless specified otherwise.

This present disclosure pertains generally to molecular constructs, in which each molecular construct comprises a targeting element (T) and an effector element (E), and these molecular constructs are sometimes referred to as "T-E molecules", "T-E pharmaceuticals" or "T-E drugs" in this document.

As used herein, the term "targeting element" refers to the portion of a molecular construct that directly or indirectly binds to a target of interest (e.g., a receptor on a cell surface or a protein in a tissue) thereby facilitates the transportation of the present molecular construct into the interested target. In some example, the targeting element may direct the molecular construct to the proximity of the target cell. In other cases, the targeting element specifically binds to a molecule present on the target cell surface or to a second molecule that specifically binds a molecule present on the cell surface. In some cases, the targeting element may be internalized along with the present molecular construct once it is bound to the interested target, hence is relocated into the cytosol of the target cell. A targeting element may be an antibody or a ligand for a cell surface receptor, or it may be a molecule that binds such antibody or ligand, thereby indirectly targeting the present molecular construct to the target site (e.g., the surface of the cell of choice). The localization of the effector (therapeutic agent) in the diseased site will be enhanced or favored with the present molecular constructs as compared to the therapeutic without a targeting function. The localization is a matter of degree or relative proportion; it is not meant for absolute or total localization of the effector to the diseased site.

According to the present invention, the term "effector element" refers to the portion of a molecular construct that elicits a biological activity (e.g., inducing immune responses, exerting cytotoxic effects and the like) or other functional activity (e.g., recruiting other hapten tagged therapeutic molecules), once the molecular construct is directed to its target site. The "effect" can be therapeutic or diagnostic. The effector elements encompass those that bind to cells and/or extracellular immunoregulatory factors. The effector element comprises agents such as proteins, nucleic acids, lipids, carbohydrates, glycopeptides, drug moieties (both small molecule drug and biologics), compounds, elements, and isotopes, and fragments thereof.

Although the terms, first, second, third, etc., may be used herein to describe various elements, components, regions, and/or sections, these elements (as well as components, regions, and/or sections) are not to be limited by these terms. Also, the use of such ordinal numbers does not imply a sequence or order unless clearly indicated by the context. Rather, these terms are simply used to distinguish one element from another. Thus, a first element, discussed below, could be termed a second element without departing from the teachings of the examples.

Here, the terms "link," "couple," and "conjugates" are used interchangeably to refer to any means of connecting two components either via direct linkage or via indirect linkage between two components.

The term "polypeptide" as used herein refers to a polymer having at least two amino acid residues. Typically, the polypeptide comprises amino acid residues ranging in length from 2 to about 200 residues; preferably, 2 to 50 residues. Where an amino acid sequence is provided herein, L-, D-, or beta amino acid versions of the sequence are also contemplated. Polypeptides also include amino acid polymers in which one or more amino acid residues are an artificial chemical analogue of a corresponding naturally occurring amino acid, as well as to naturally occurring amino acid polymers. In addition, the term applies to amino acids joined by a peptide linkage or by other, "modified linkages," e.g., where the peptide bond is replaced by an $\alpha$-ester, a $\beta$-ester, a thioamide, phosphoramide, carbomate, hydroxylate, and the like.

In certain examples, conservative substitutions of the amino acids comprising any of the sequences described herein are contemplated. In various examples, one, two, three, four, or five different residues are substituted. The term "conservative substitution" is used to reflect amino acid substitutions that do not substantially alter the activity (e.g., biological or functional activity and/or specificity) of the molecule. Typically, conservative amino acid substitutions involve substitution one amino acid for another amino acid with similar chemical properties (e.g., charge or hydrophobicity). Certain conservative substitutions include "analog substitutions" where a standard amino acid is replaced by a non-standard (e.g., rare, synthetic, etc.) amino acid differing minimally from the parental residue. Amino acid analogs are considered to be derived synthetically from the standard amino acids without sufficient change to the structure of the

parent, are isomers, or are metabolite precursors.

In certain examples, polypeptides comprising at least 80%, preferably at least 85% or 90%, and more preferably at least 95% or 98% sequence identity with any of the sequences described herein are also contemplated.

"Percentage (%) amino acid sequence identity" with respect to the polypeptide sequences identified herein is defined as the percentage of polypeptide residues in a candidate sequence that are identical with the amino acid residues in the specific polypeptide sequence, after aligning the sequences and introducing gaps, if necessary, to achieve the maximum percent sequence identity, and not considering any conservative substitutions as part of the sequence identity. Alignment for purposes of determining percentage sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, sequence comparison between two polypeptide sequences was carried out by computer program Blastp (protein-protein BLAST) provided online by Nation Center for Biotechnology Information (NCBI). The percentage amino acid sequence identity of a given polypeptide sequence A to a given polypeptide sequence B (which can alternatively be phrased as a given polypeptide sequence A that has a certain % amino acid sequence identity to a given polypeptide sequence B) is calculated by the formula as follows:

$$\frac{X}{Y} \times 100\ \%$$

where X is the number of amino acid residues scored as identical matches by the sequence alignment program BLAST in that program's alignment of A and B, and where Y is the total number of amino acid residues in A or B, whichever is shorter.

As used herein, the term "terminus" with respect to a polypeptide refers to an amino acid residue at the N- or C-end of the polypeptide. With regard to a polymer, the term "terminus" refers to a constitutional unit of the polymer (e.g., the polyethylene glycol of the present disclosure) that is positioned at the end of the polymeric backbone. In the present specification and claims, the term "free terminus" is used to mean the terminal amino acid residue or constitutional unit is not chemically bound to any other molecular.

The term "antigen" or "Ag" as used herein is defined as a molecule that elicits an immune response. This immune response may involve a secretory, humoral, and/or cellular antigen-specific response. In the present disclosure, the term "antigen" can be any of a protein, a polypeptide (including mutants or biologically active fragments thereof), a polysaccharide, a glycoprotein, a glycolipid, a nucleic acid, or a combination thereof.

In the present specification and claims, the term "antibody" is used in the broadest sense and covers fully assembled antibodies, antibody fragments that bind with antigens, such as antigen-binding fragment (Fab/Fab'), $F(ab')_2$ fragment (having two antigen-binding Fab portions linked together by disulfide bonds), variable fragment (Fv), single chain variable fragment (scFv), bi-specific single-chain variable fragment (bi-scFv), nanobodies, unibodies and diabodies. "Antibody fragments" comprise a portion of an intact antibody, preferably the antigen-binding region or variable region of the intact antibody. Typically, an "antibody" refers to a protein consisting of one or more polypeptides substantially encoded by immunoglobulin genes or fragments of immunoglobulin genes. The well-known immunoglobulin genes include the kappa, lambda, alpha, gamma, delta, epsilon, and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. A typical immunoglobulin (antibody) structural unit is known to comprise a tetramer. Each tetramer is composed of two identical pairs of polypeptide chains, with each pair having one "light" chain (about 25 kDa) and one "heavy" chain (about 50-70 kDa). The N-terminus of each chain defines a variable region of about 100 to 110 or more amino acids primarily responsible for antigen recognition. The terms variable light chain ($V_L$) and variable heavy chain ($V_H$) refer to these light and heavy chains, respectively. According to examples of the present disclosure, the antibody fragment can be produced by modifying the nature antibody or by *de novo* synthesis using recombinant DNA methodologies. In certain examples of the present disclosure, the antibody and/or antibody fragment can be bispecific, and can be in various configurations. For example, bispecific antibodies may comprise two different antigen binding sites (variable regions). In various examples, bispecific antibodies can be produced by hybridoma technique or recombinant DNA technique. In certain examples, bispecific antibodies have binding specificities for at least two different epitopes.

The term "specifically binds" as used herein, refers to the ability of an antibody or an antigen-binding fragment thereof, to bind to an antigen with a dissociation constant (Kd) of no more than about $1\times10^{-6}$ M, $1\times10^{-7}$ M, $1\times10^{-6}$ M, $1\times10^{-9}$ M, $1\times10^{-10}$ M, $1\times10^{-11}$ M, $1\times10^{-12}$ M, and/or to bind to an antigen with an affinity that is at least two-folds greater than its affinity to a nonspecific antigen.

The present disclosure is based, at least on the construction of the T-E pharmaceuticals that can be delivered to target cells, target tissues or organs at increased proportions relative to the blood circulation, lymphoid system, and other

cells, tissues or organs. When this is achieved, the therapeutic effect of the pharmaceuticals is increased, while the scope and severity of the side effects and toxicity is decreased. It is also possible that a therapeutic effector is administered at a lower dosage in the form of a T-E molecule, than in a form without a targeting component. Therefore, the therapeutic effector can be administered at lower dosages without losing potency, while lowering side effects and toxicity.

## PART I Multi-Arm Linkers for Treating Specific Diseases

### *I-(i) Peptide Core for Use in Multi-arm Linker*

The present disclosure pertains to a linker unit that comprises, (1) a center core that comprises 2-15 lysine (K) residues, and (2) 2-15 linking arms respectively linked to the K residues of the center core. The present center core is characterized in having or being linked with an azide group, an alkyne group, a tetrazine group, or a strained alkyne group at its N- or C-terminus.

In the preparation of the present linker unit, a PEG chain having a N-hydroxysuccinimidyl (NHS) group at one terminus and a functional group (e.g., an NHS, a maleimide, an azide, an alkyne, a tetrazine, or a strained alkyne group) at the other terminus is linked to the K residue of the center core by forming an amide bond between the NHS group of the PEG chain and the amine group of the K residue. In the present disclosure, the PEG chain linked to the K residue is referred to as a linking arm, which has a functional group at the free-terminus thereof.

According to the examples of the present disclosure, the center core is a polypeptide that has 8-120 amino acid residues in length and comprises 2 to 15 lysine (K) residues, in which each K residue and the next K residue are separated by a filler sequence.

According to examples of the present disclosure, the filler sequence comprises glycine (G) and serine (S) residues; preferably, the filler sequence consists of 2-15 residues selected from G, S, and a combination thereof. For example, the filler sequence can be,

GS,
GGS,
GSG,
GGGS (SEQ ID NO: 1),
GSGS (SEQ ID NO: 2),
GGSG (SEQ ID NO: 3),
GSGGS (SEQ ID NO: 4),
SGGSG (SEQ ID NO: 5),
GGGGS (SEQ ID NO: 6),
GGSGGS (SEQ ID NO: 7),
GGSGGSG (SEQ ID NO: 8),
SGSGGSGS (SEQ ID NO: 9),
GSGGSGSGS (SEQ ID NO: 10),
SGGSGGSGSG (SEQ ID NO: 11),
GGSGGSGGSGS (SEQ ID NO: 12),
SGGSGGSGSGGS (SEQ ID NO: 13),
GGGGSGGSGGGGS (SEQ ID NO: 14),
GGGSGSGSGSGGGS (SEQ ID NO: 15), or
SGSGGGGGSGGSGSG (SEQ ID NO: 16).

The filler sequence placed between two lysine residues may be variations of glycine and serine residues in somewhat random sequences and/or lengths. Longer fillers may be used for a polypeptide with fewer lysine residues, and shorter fillers for a polypeptide with more lysine residues. Hydrophilic amino acid residues, such as aspartic acid and histidine, may be inserted into the filler sequences together with glycine and serine. As alternatives for filler sequences made up with glycine and serine residues, filler sequences may also be adopted from flexible, soluble loops in common human serum proteins, such as albumin and immunoglobulins.

Basically, the filler sequences between lysine residues cover peptides with glycine and serine residues. However, they can alternatively be peptides composed of amino acids excluding one with amine group in its side chain. Those amino acids are predominantly, but not necessarily entirely hydrophilic amino acids. The amino acids are not necessarily naturally occurring amino acids.

According to the present disclosure, the center core comprises 2-15 units of the sequence of $G_{1-5}SK$. Alternatively, the polypeptide comprises the sequence of $(GSK)_{2-15}$; that is, the polypeptide comprises at least two consecutive units of the sequence of GSK. For example, the present center core may comprise the amino acid sequence of the

following,

Ac-CGGSGGSGGSKGSGSK (SEQ ID NO: 17),
Ac-CGGSGGSGGSKGSGSKGSK (SEQ ID NO: 18), or
Ac-CGSKGSKGSKGSKGSKGSKGSKGSKGSKGSK (SEQ ID NO: 19),

in which Ac represents the acetyl group.

As described above, the present center core is characterized in having or being linked with an azide group, an alkyne group, a tetrazine group, or a strained alkyne group at its N- or C-terminus. According to some examples of the present disclosure, the present center core comprises, at its N- or C-terminus, an amino acid residue having an azide group or an alkyne group. The amino acid residue having an azide group can be, L-azidohomoalanine (AHA), 4-azido-L-phenylalanine, 4-azido-D-phenylalanine, 3-azido-L-alanine, 3-azido-D-alanine, 4-azido-L-homoalanine, 4-azido-D-homoalanine, 5-azido-L-ornithine, 5-azido-d-ornithine, 6-azido-L-lysine, or 6-azido-D-lysine. For example, the present center core may have the sequence of,

Ac-$(GSK)_{2\text{-}7}$-$(G2\text{-}4S)_{1\text{-}8}$-$A^{AH}$,
Ac-$A^{AH}$-$(SG_{2\text{-}4})_{1\text{-}8}$-$(GSK)_{2\text{-}7}$,
Ac-$A^{AH}$-$(SG_{2\text{-}4})_{0\text{-}7}$-$(GSK)_{2\text{-}6}$-$(G_{2\text{-}4}S)_{1\text{-}8}$-C, or
Ac-C-$(SG_{2\text{-}4})_{0\text{-}7}$-$(GSK)_{2\text{-}6}$-$(G_{2\text{-}4}S)_{1\text{-}8}$-$A^{AH}$,

in which Ac represents the acetyl group, and $A^{AH}$ represents the AHA residue.

Exemplary amino acid having an alkyne group includes, but is not limited to, L-homopropargylglycine (L-HPG), D-homopropargylglycine (D-HPG), or beta-homopropargylglycine (β-HPG). In this case, the present center core may have the sequence of,

Ac-$(GSK)_{2\text{-}7}$-$(G_{2\text{-}4}S)_{1\text{-}8}$-$G^{HP}$,
Ac-$G^{HP}$-$(SG_{2\text{-}4})_{1\text{-}8}$-$(GSK)_{2\text{-}7}$,
Ac-$G^{HP}$-$(SG_{2\text{-}4})_{0\text{-}7}$-$(GSK)_{2\text{-}6}$-$(G_{2\text{-}4}S)_{1\text{-}8}$-C, or
Ac-C-$(SG_{2\text{-}4})_{0\text{-}7}$-$(GSK)_{2\text{-}6}$-$(G_{2\text{-}4}S)_{1\text{-}8}$-$G^{HP}$,

in which Ac represents the acetyl group, and $G^{HP}$ represents the HPG residue.

It is noted that many of the amino acids containing an azide or alkyne group in their side chains and PEGylated amino acids are available commercially in t-boc (tert-butyloxycarbonyl)- or Fmoc (9-fluorenylmethyloxycarbonyl)-protected forms, which are readily applicable in solid-phase peptide synthesis.

According to some working examples of the present disclosure, the center core may comprise the sequence of,

Ac-$G^{HP}$GGSGGSGGSKGSGSK (SEQ ID NO: 21),
Ac-$G^{HP}$GGSGGSGGSKGSGSKGSK (SEQ ID NO: 22),
Ac-$A^{AH}$GGSGGSGGSKGSGSKGSK (SEQ ID NO: 23),
Ac-$G^{HP}$GGSGGSGGSKGSGSKGSGSC (SEQ ID NO: 24),
Ac-C-$Xaa_2$-K-$Xaa_2$-K-$Xaa_2$-K (SEQ ID NO: 25), or
Ac-C-$Xaa_6$-K-$Xaa_6$-K-$Xaa_6$-K-$Xaa_6$-K-$Xaa_6$-K (SEQ ID NO: 26),

in which Xaa is a PEGylated amino acid having specified repeats of EG unit, Ac represents the acetyl group, $A^{AH}$ represents the AHA residue, and $G^{HP}$ represents the HPG residue; SEQ ID NOs: 25 and 26 are only provided for illustration but not within the scope of the claims.

Alternatively, the present center core is linked with a coupling arm, which has a functional group (e.g., an azide group, an alkyne group, a tetrazine group, or a strained alkyne group) at the free-terminus thereof (that is, the terminus that is not linked to the center core). In these cases, the present center core comprises a cysteine residue at its N- or C-terminus. To prepare a linker unit linked with a coupling arm, a PEG chain having a maleimide group at one terminus and a functional group at the other terminus is linked to the cysteine residue of the center core via thiol-maleimide reaction occurred between the maleimide group of the PEG chain and the thiol group of the cysteine residue. In the present disclosure, the PEG chain linked to the cysteine residue of the center core is referred to as the coupling arm, which has a functional group at the free-terminus thereof.

Preferably, the coupling arm has a tetrazine group or a strained alkyne group (e.g., a cyclooctene or cyclooctyne group) at the free-terminus thereof. These coupling arms have 2-12 EG units. According to the examples of the present disclosure, the tetrazine group is 1,2,3,4-tetrazine, 1,2,3,5-tetrazine, 1,2,4,5-tetrazine, or derivatives thereof. The strained alkyne group may be a cyclooctene or a cyclooctyne group. According to the working examples of the present disclosure,

the cyclooctene group is a trans-cyclooctene (TCO) group; example of cyclooctyne group includes, but is not limited to, dibenzocyclooctyne (DBCO), difluorinated cyclooctyne (DIFO), bicyclononyne (BCN), and dibenzocyclooctyne (DICO). According to some examples of the present disclosure, the tetrazine group is 6-methyl-tetrazine.

Alternatively, the center core has an azide or alkyne group at one terminus and a coupling arm with tetrazine or strained alkyne group at the other terminus.

The polypeptide may also be synthesized using recombinant technology by expressing designed gene segments in bacterial or mammalian host cells. It is preferable to prepare the polypeptide as recombinant proteins if the core has high numbers of lysine residues with considerable lengths. As the length of a polypeptide increases, the number of errors increases, while the purity and/or the yield of the product decrease, if solid-phase synthesis was adopted. To produce a polypeptide in bacterial or mammalian host cells, a filler sequence ranges from a few amino acid residues to 10-20 residues may be placed between two K residues. Further, since AHA and HPG are not natural amino acids encoded by the genetic codes, the N-terminal or C-terminal residue for those recombinant polypeptides is cysteine. After the recombinant proteins are expressed and purified, the terminal cysteine residue is then reacted with short bifunctional cross-linkers, which have maleimide group at one end, which reacts with SH group of cysteine residue, and alkyne, azide, tetrazine, or strained alkyne at the other end.

The synthesis of a polypeptide using PEGylated amino acids involves fewer steps than that with regular amino acids such as glycine and serine resides. In addition, PEGylated amino acids with varying lengths (i.e., numbers of repeated ethylene glycol units) may be employed, offering flexibility for solubility and spacing between adjacent amino groups of lysine residues. Other than PEGylated amino acids, the center cores may also be constructed to comprise artificial amino acids, such as D-form amino acids, homo-amino acids, N-methyl amino acids, etc. Preferably, the PEGylated amino acids with varying lengths of polyethylene glycol (PEG) are used to construct the center core, because the PEG moieties contained in the amino acid molecules provide conformational flexibility and adequate spacing between conjugating groups, enhance aqueous solubility, and are generally weakly immunogenic. The synthesis of PEGylated amino acid-containing center core is similar to the procedures for the synthesis of regular polypeptides.

Optionally, for stability purpose, the present center core has an acetyl group to block the amino group at its N-terminus.

As could be appreciated, the number of the linking arms linked to the center core is mainly determined by the number of lysine resides comprised in the center core. Since there are at least two lysine residues comprised in the present center core, the present linker unit may comprise a plurality of linking arms.

Reference is now made to Figure 1A. As illustrated, the linker unit 10A comprises a center core 11a comprising one HPG ($G^{HP}$) residue and four lysine (K) residues respectively separated by filler sequences (denoted by the dots throughout the drawings). The filler sequences between the HPG residue and K residue or between any two K residues may comprise the same or different amino acid sequences. In this example, four linking arms 20a-20d are linked to the lysine residues by forming an amide linkage between the NHS group and the amine group of the lysine residue, respectively. As could be appreciated, certain features discussed above regarding the linker unit 10A or any other following linker units are common to other linker units disclosed herein, and hence some or all of these features are also applicable in the following examples, unless it is contradictory to the context of a specific example. However, for the sake of brevity, these common features may not be explicitly repeated below.

Figure 1B provides a linker unit 10B according to another example of the present disclosure. The center core 11b comprises one cysteine (C) residue and six lysine (K) residues respectively separated by the filler sequences. In this example, the linker unit 10B comprises six linking arms 20a-20f that are respectively linked to the lysine residues. According to the examples of the present disclosure, the linking arm is a PEG chain having 2-20 repeats of EG units.

Unlike the linker unit 10A of Figure 1A, the linker unit 1B further comprises a coupling arm 60. As discussed above, a PEG chain having a maleimide group at one end and a functional group at the other end is used to form the coupling arm 60. In this way, the coupling arm 60 is linked to the cysteine residue of the center core 11b via thiol-maleimide reaction. In this example, the functional group at the free terminus of the coupling arm 60 is a tetrazine group 72. According to the examples of the present disclosure, the coupling arm is a PEG chain having 2-12 repeats of EG units.

According to the examples of the present disclosure, the linking arm linked to the K residue of the center core has a functional group (i.e., a maleimide, an NHS, an azide, an alkyne, a tetrazine, or a strained alkyne group) at its free terminus. Preferably, when the free terminus of the linking arm is an azide, alkyne, or cyclooctyne group, then the amino acid residue at the N- or C-terminus of the center core is a cysteine residue, and the free terminus of the coupling arm is a tetrazine or cyclooctene group. Alternatively, when the free terminus of the linking arm is a tetrazine group or cyclooctene group, then the amino acid residue at the N- or C-terminus of the center core has an azide or alkyne group, or the amino acid residue at the N- or C-terminus of the center core is a cysteine residue, and the free terminus of the coupling arm is an azide, the alkyne, or the cyclooctyne group

Depending on the functional group (i.e., a maleimide, an NHS, an azide, an alkyne, a tetrazine, or a strained alkyne group) present at the free terminus of the linking arm, it is feasible to design a functional element (such as, a targeting element, an effector element, or an element for improving the pharmacokinetic property) with a corresponding

functional group, so that the functional element may linked to the free terminus of the linking arm via any of the following chemical reactions,

(1) forming an amide bond therebetween: in this case, the linking arm has an NHS group at the free terminus, and the functional element has an amine group;
(2) the thiol-maleimide reaction: in this case, the linking arm has a maleimide group at the free terminus, and the functional element has an thiol group;
(3) the Copper(I)-catalyzed alkyne-azide cycloaddition reaction (CuAAC reaction, or the "click" reaction for short): one of the free terminus of the linking arm and the functional element has an azide group, while the other has an alkyne group; the CuAAC reaction is exemplified in Scheme 1;
(4) the inverse electron demand Diels-Alder (iEDDA) reaction: one of the free terminus of the linking arm and the functional element has a tetrazine group, while the other has a cyclooctene group; the iEDDA reaction is exemplified in Scheme 2; or
(5) the strained-promoted azide-alkyne click chemistry (SPAAC) reaction: one of the free terminus of the linking arm and the functional element has an azide group, while the other has an cyclooctyne group; the SPAAC reaction is exemplified in Scheme 3.

## <<Scheme 1 CuAAC reaction>>

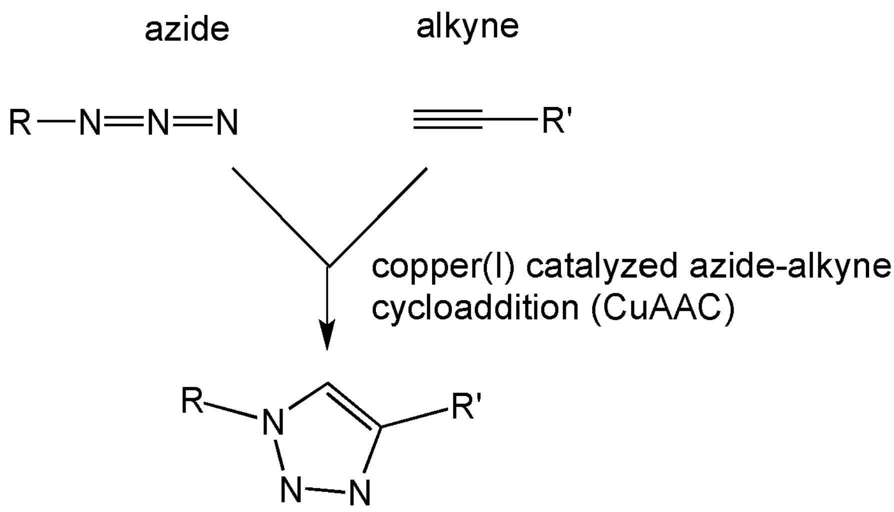


The CuAAC reaction yields 1,5 di-substituted 1,2,3-triazole. The reaction between alkyne and azide is very selective and there are no alkyne and azide groups in natural biomolecules. Furthermore, the reaction is quick and pH-insensitive. It has been suggested that instead of using copper (I), such as cuprous bromide or cuprous iodide, for catalyzing the click reaction, it is better to use a mixture of copper (II) and a reducing agent, such as sodium ascorbate to produce copper (I) in situ in the reaction mixture. Alternatively, the second element can be linked to the N- or C-terminus of the present center core via a copper-free reaction, in which pentamethylcyclopentadienyl ruthenium chloride complex is used as the catalyst to catalyze the azide-alkyne cycloaddition.

## <<Scheme 2 iEDDA Reaction>>

Tetrazine Trans-cyclooctene (TCO)

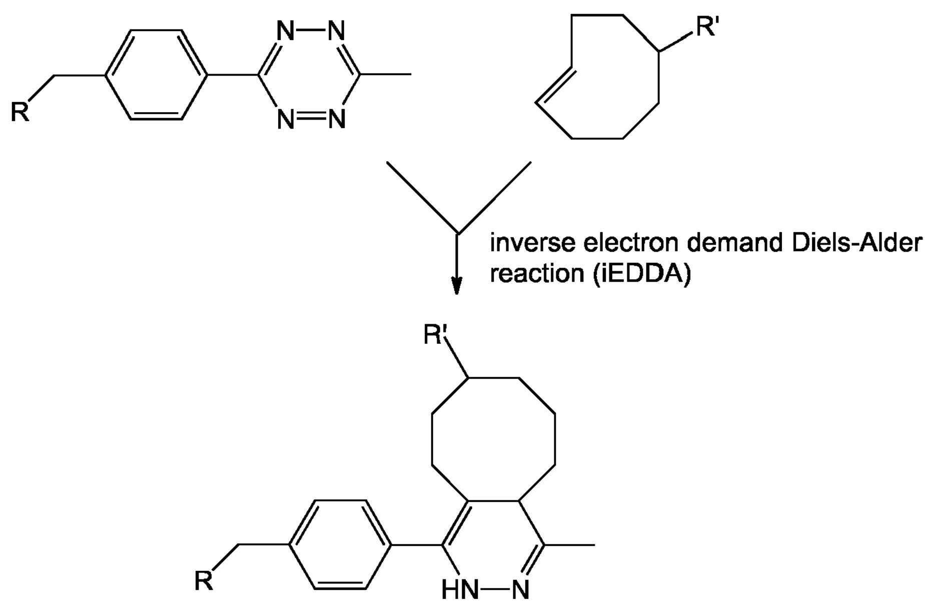


**<<Scheme 3 SPAAC reaction>>**

dibenzocyclooctyl (DBCO)

azide

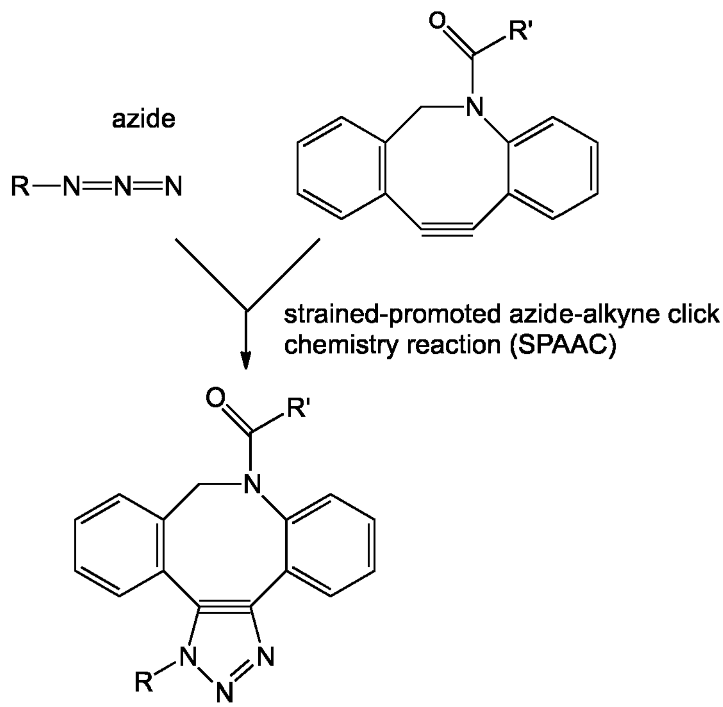


For the sake of illustration, the functional elements linked to the linking arms are referred to as the first elements. As could be appreciated, the number of the first elements carried by the present linker unit depends on the number of K residues of the center core (and thus, the number of the linking arms). Accordingly, one of ordinary skill in the art may adjust the number of the first elements of the linker unit as necessary, for example, to achieve the desired targeting or therapeutic effect.

An example of a linker unit 10C having the first elements is illustrated Figure 1C. Other than the features disused hereafter, Figure 1C is quite similar to Figure 1B. First, there are five K residues in the center core 11d, and accordingly, five linking arms 20a-20e are linked thereto, respectively. Second, the linker unit 10C has five first elements 30a-30e linked to each of the linking arms 20a-20e. As disused below, the optional tetrazine group 72 allows for the conjugation with an

additional functional element, another molecular construct (see, Part II below).

In order to increase the intended or desired effect (e.g., the therapeutic effect), the present linker unit may further comprise a second element in addition to the first element. For example, the second element can be either a targeting element or an effector element. In some examples of the present disclosure, the first element is an effector element, while the second element may be another effector element, which works additively or synergistically with or independently of the first element. Still optionally, the first and second elements exhibit different properties; for example, the first element is a targeting element, and the second element is an effector element, and vice versa. Alternatively, the first element is an effector element, and the second element is an element capable of improving the pharmacokinetic property of the linker unit, such as solubility, clearance, half-life, and bioavailability. The choice of a particular first element and/or second element depends on the intended application in which the present linker unit (or multi-arm linker) is to be used. Examples of these functional elements are discussed below in Part I-(iii) of this specification.

Structurally, the second element is linked to the azide, alkyne, tetrazine, or strained alkyne group at the N- or C-terminus of the center core. Specifically, the second element may be optionally conjugated with a short PEG chain (preferably having 2-12 repeats of EG units) and then linked to the N- or C-terminal amino acid residue having an azide group or an alkyne group (e.g., AHA residue or HPG residue). Alternatively, the second element may be optionally conjugated with the short PEG chain and then linked to the coupling arm of the center core.

According to some examples of the present disclosure, the center core comprises an amino acid having an azide group (e.g., the AHA residue) at its N- or C-terminus; and accordingly, a second element having an alkyne group is linked to the N- or C-terminus of the center core via the CuAAC reaction. According to other examples of the present disclosure, the center core comprises an amino acid having an alkyne group (e.g., the HPG residue) at its N- or C-terminus; and a second element having an azide group is thus capable of being linked to the N- or C-terminus of the center core via the CuAAC reaction.

Figure 1D provides an example of the present linker unit 10D carrying a plurality of first elements and one second element. In this example, the center core 11c comprises one HPG ($G^{HP}$) residue and five lysine (K) residues. Five linking arms 20a-20e are respectively linked to the five K residues of the center core 11c; and five first elements 30a-30e are respectively linked to said five linking arms 20a-20e via the thiol-maleimide reaction. In addition to the first elements, the linker unit 10D further comprises one second element 50 that is linked to one end of a short PEG chain 62. Before being conjugated with the center core 11c, the other end of the short PEG chain 62 has an azide group. In this way, the azide group may react with the HPG residue that having an alkyne group via CuAAC reaction, so that the second element 50 is linked to the center core 11c. The solid dot 40 depicted in Figure 1D represents the chemical bond resulted from the CuAAC reaction occurred between the HPG residue and the azide group.

Alternatively, the second element is linked to the center core via a coupling arm. According to certain examples of the present disclosure, the coupling arm has a tetrazine group, which can be efficiently linked to a second element having a TCO group via the iEDDA reaction. According to other examples of the present disclosure, the coupling arm has a TCO group, which is capable of being linked to a second element having a tetrazine group via the iEDDA reaction. In the iEDDA reaction, the strained cyclooctene that possess remarkably decreased activation energy in contrast to terminal alkynes is employed, and thus eliminates the need of an exogenous catalyst.

Reference is now made to Figure 1E, in which the center core 11d of the linker unit 10E comprises a terminal cysteine (C) residue and five lysine (K) residues. As depicted in Figure 1E, five linking arms 20a-20e are respectively linked to the five K residue of the center core 11d, and then five first elements 30a-30e are respectively linked to the five linking arms 20a-20e via thiol-maleimide reactions. The cysteine residue is linked to the coupling arm 60, which, before being conjugated with the second element, comprises a tetrazine group or a TCO group at its free-terminus. In this example, a second element 50 linked with a short PEG chain 62 having a corresponding TCO or tetrazine group can be linked to the coupling arm 60 via the iEDDA reaction. The ellipse 70 as depicted in Figure 1E represents the chemical bond resulted from the iEDDA reaction occurred between the coupling arm 60 and the short PEG chain 62.

According to other examples of the present disclosure, before the conjugation with a second element, the coupling arm has an azide group. As such, the coupling arm can be linked to the second element having a strained alkyne group (e.g., the DBCO, DIFO, BCN, or DICO group) at the free-terminus of a short PEG chain via SPAAC reaction (see, scheme 3), and *vice versa.*

Reference is now made to Figure 1F, in which the linker unit 10F has a structure similar to the linker unit 10E of Figure 1E, except that the coupling arm 60 comprises an azide or a strained alkyne group (e.g., the DBCO, DIFO, BCN, or DICO group), instead of the tetrazine or TCO group. Accordingly, the second element 50 linked with a short PEG chain 62 may have a corresponding strained alkyne (e.g., DBCO, DIFO, BCN, or DICO) or azide group, so that it can be linked to the coupling arm 60 via the SPAAC reaction. The diamond 90 as depicted in Figure 1F represents the chemical bond resulted from the SPAAC reaction occurred between the coupling arm 60 and the short PEG chain 62.

Scheme 4 is an exemplary illustration of the process of preparing the present linker unit. In step 1, the center core comprising the amino acid sequence of $(GSK)_3$ and a L-azidohomoalanine (AHA) residue at the C-terminus thereof is prepared. In step 2, three linking arms are respectively linked to the lysine (K) residues of the center core via forming an

amide bond between the NHS group and the amine group; the linking arm linked to the center core has a maleimide (Mal) group at the free-terminus thereof. In step 3, three anti-fibrin scFvs (scFv α fibrin) as the first element are respectively linked to the linking arms via the thiol-maleimide reaction. Meanwhile, in step 4, one tPA analogue as the second element is linked with a short PEG chain that has 4 repeats of EG units and a DBCO group at the free terminus. Finally, in step 5, the second element is linked to the AHA residue of the center core via the SPAAC reaction.

### <<Scheme 4 Preparation of linker unit linked with two different scFvs via linking arm and C-terminal amino acid residue>>

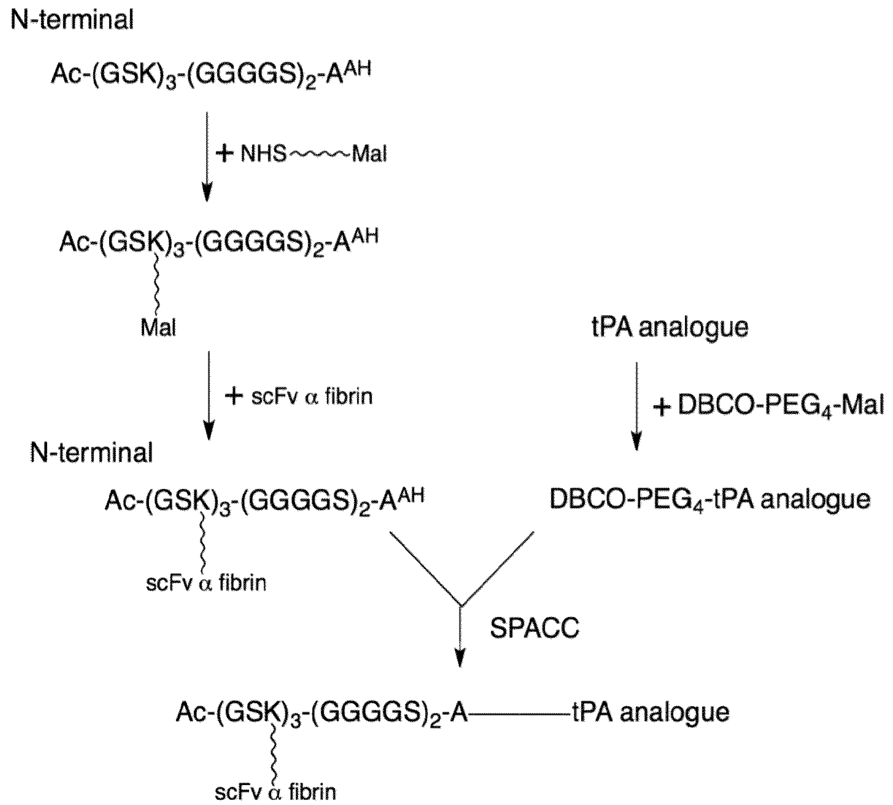


Figure 1G provides an alternative example of the present linker unit (linker unit 10G), in which five first elements 30 are respectively linked to the lysine residues via the linking arms 20, and the AHA ($A^{AH}$) residue of the center core 11e is linked with a PEG chain 80 via the CuAAC reaction. The solid dot 40 depicted in Figure 1G represents the chemical bond resulted from the CuAAC reaction occurred between the AHA residue and the PEG chain 80.

Figure 1H provides another example of the present disclosure, in which the N-terminus of the center core 11d is a cysteine residue that is linked to a coupling arm 60. A PEG chain 80 can be efficiently linked to the coupling arm 60 via the iEDDA reaction. The ellipse 70 of the linker unit 10H represents the chemical bond resulted from the iEDDA reaction occurred between the coupling arm 60 and the PEG chain 80.

Figure 1I provides an alternative example of the present linker unit, in which the linker unit 101 has a structure similar to the linker unit 10G of Figure 1G, except that the PEG chain 80 is linked to the coupling arm 60 via the SPAAC reaction. The diamond 90 depicted in Figure 1I represents the chemical bond resulted from the SPAAC reaction occurred between the coupling arm 60 and the PEG chain 80.

In the preferred examples of this disclosure, the linking arms have a maleimide group in the free terminus for conjugating with first elements having the sulfhydryl group via the thiol-maleimide reaction. Also, there is one cysteine residue or an amino acid residue with an azide or alkyne group at a terminus of the peptide core for attaching a coupling arm for linking a second element.

It is conceivable for those skilled in the arts that variations may be made. A conjugating group, other than maleimide, such as azide, alkyne, tetrazine, or strained alkyne may be used for the free terminus of the linking arms, for linking with first elements with a CuAAC, iEDDA, or SPAAC reaction. Also the cysteine residue (or an amino acid residue with an azide or alkyne group) of the peptide core needs not to be at the N- or C-terminus. Furthermore, two or more of such residues may be incorporated in the peptide core to attach multiple coupling arms for linking a plural of second elements.

### *I-(ii) Functional Elements Suitable for Use in Multi-arm Linker*

In the case where the linker unit (or multi-arm linker) comprises only the first element but not the second element(s), the first element is an effector element that may elicit a therapeutic effect in a subject. On the other hand, when the present linker unit comprises two elements, then one of the elements is an effector element, while the other may be another effector element, a targeting element, or an element capable of enhancing one or more pharmacokinetic properties of the linker unit (e.g., solubility, clearance, half-life, and bioavailability). For example, the linker unit may have two different kinds of effector element, or one effector element and one targeting element or one pharmacokinetic property-enhancing element.

According to some examples of the present disclosure, the present linker unit is useful in preventing the formation of blood clot. In these examples, the present linker unit comprises the first element of an scFv specific for fibrin as the targeting element, and the second element of Factor Xa inhibitors or thrombin inhibitors as the effector element. Preferably, the inhibitor of Factor Xa is selected from the group consisting of, apixaban, edoxaban, and rivaroxaban; and the inhibitor of thrombin is argatroban or melagatran.

Linker units for use in the treatment of thrombosis may comprise the first element of an scFv specific for fibrin as the targeting element, and the second element of tissue plasminogen activators (such as alteplase, reteplase, tenecteplase and lanoteplase) used as the effector element.

Compared with previously known therapeutic constructs, the present linker unit discussed in Part I is advantageous in two points:

(1) The number of the functional elements may be adjusted in accordance with the needs and/or applications. The present linker unit may comprise two elements (i.e., the first and second elements) in accordance with the requirements of the application (e.g., the disease being treated, the route of administration of the present linker unit, and the binding avidity and/or affinity of the antibody carried by the present linker unit). For example, when the present linker unit is directly delivered into the tissue/organ (e.g., the treatment of eye), one element acting as the effector element may be enough, thus would eliminate the need of a second element acting as the targeting element. However, when the present linker unit is delivered peripherally (e.g., oral, enteral, nasal, topical, transmucosal, intramuscular, intravenous, or intraperitoneal injection), it may be necessary for the present linker unit to simultaneously comprise a targeting element that specifically targets the present linker unit to the lesion site; and an effector element that exhibits a therapeutic effect on the lesion site.

(2) The first element is provided in the form of a bundle. As described above, the number of the first element may vary with the number of lysine residue comprised in the center core. If the number of lysine residue in the center core ranges from 2 to 15, then at least two first elements may be comprised in each linker unit. Thus, instead of providing one single molecule (e.g., cytotoxic drug and antibody) as traditional therapeutic construct may render, the present linker unit is capable of providing more functional elements (either as targeting elements or as effector elements) at one time, thereby greatly improves the therapeutic effect.

In certain therapeutic applications, it is desirable to have a single copy of a targeting or effector element. For example, a single copy of a targeting element can be used to avoid unwanted effects due to overly tight binding. This consideration is relevant, when the scFv has a relatively high affinity for the targeted antigen and when the targeted antigen is a cell surface antigen on normal cells, which are not targeted diseased cells. As an example, in using scFv specific for CD3 or CD16a to recruit T cells or NK cells to kill targeted cells, such as thyroid gland cells in patients with Grave's disease,, a single copy of the scFv specific for CD3 or CD16a is desirable, so that unwanted effects due to cross-linking of the CD3 or CD16a may be avoided. Similarly, in using scFv specific for CD32 or CD16b to recruit phagocytic neutrophils and macrophages to clear antibody-bound viral or bacterial particles or their products, a single copy of scFv may be desirable. Also, in using scFv specific for transferrin receptor to carry effector drug molecules to the BBB for treating CNS diseases, a single copy of scFv specific for transferrin receptor is desirable. In still another example, it is desirable to have only one copy of long-chain PEG for enhancing pharmacokinetic properties. Two or more long PEG chains may cause tangling and affect the binding properties of the targeting or effector elements.

## PART II Joint-linker Molecular Constructs for Treating Specific Diseases

The present disclosure further pertains to a molecular construct comprising two linker units, in which one linker unit carries one or more targeting element, whereas the other linker unit carries one or more effector elements or pharmacokinetic property-enhancing elements. In the present disclosure, molecular constructs with both the targeting and effector moieties (whether a therapeutic or pharmacokinetic one) are referred to as joint-linker molecular constructs. According to various examples of the present disclosure, each of the linker unit comprised in such joint-linker molecular constructs is a peptide core-based multi-arm linkers discussed above in Part I of the present disclosure.

***II-(i) Structure of Joint-linker Molecular Construct***

According to some examples of the present disclosure, the molecular construct comprises two linker units, and the linker units are coupled to each other via either the CuAAC reaction (using copper or pentamethylcyclopentadienyl ruthenium chloride complex as catalyst), the SPAAC reaction, or the iEDDA reaction. In the examples, one of the linker units is linked with a plurality of first elements, which act as the targeting elements, and the other of the linker units is linked with a plurality of second elements, which act as the effector elements.

Reference is first made to Figures 2A-2D, which respectively depicts the linkage between the two linker units. Figure 2A depicts a molecular construct comprising two linker units (100A, 200A), which are coupled to each other via the iEDDA reaction. The first linker unit 100A comprises a first center core 110a, a linking arm 120 (as the first linking arm), and a coupling arm 130a (as the first coupling arm), in which the linking and coupling arms are respectively linked to the first center core 110a at one ends. Similarly, the second linker unit 200A comprises a second center core 210a, a linking arm 220 (as the second linking arm), and a coupling arm 230a (as the second coupling arm), in which the linking and coupling arms are respectively linked to the second center core 210a at one ends. One of the coupling arms 130a, 230a has a tetrazine group at its free terminus, while the other of the coupling arms 130a, and 230a has a TCO group. Specifically, if the coupling arm 130a has a tetrazine group 152 at its free terminus (i.e., the terminus not connected to the first center core 110a), then the coupling arm 230a would have a TCO group 154 at its free terminus (i.e., the terminus not connected to the second center core 210a), and *vice versa.* Accordingly, the two linker units (100A, 200A) are coupled to each other via the iEDDA reaction occurred between the respective free ends of the coupling arms 130a, 230a. The ellipse 156 as depicted in Figure 2A represents the chemical bond resulted from the iEDDA reaction occurred between the coupling arms 130a, 230a.

In the depicted example, each of the linking arms 120, 220 has a maleimide group at its free terminus. Accordingly, a first targeting element 140 and a first effector element 240, each has a thiol group are respectively linked to the linking arms 120, 220 via the thiol-maleimide reaction.

Figure 2B provides an alternative example of the present disclosure, in which both the first and second center cores 110b, 210b are polypeptide cores, and are respectively linked to a first targeting element 140 and a first effector element 240 via the linking arms 120, 220. The unique feature in this example is that, one of the center cores 110b, 210b comprises an amino acid residue having an azide group (e.g., the AHA residue) at it N- or C-terminus, while the other of the center cores 110b, 210b comprises an amino acid residue having an alkyne group (e.g., the HPG residue) at it N- or C-terminus, such configuration allows the center cores 110a, 210a to be directly linked to each other, that is, without connecting through any coupling arms as that depicted in Figure 2A. Specifically, if the center core 110b comprises the amino acid residue having the azide group 162 at its N- or C-terminus, then the center core 210b would comprises the amino acid residue having the alkyne group 164 at its N- or C-terminus, and *vice versa.* Accordingly, the linker units 100B, 200B can couple together directly via the CuAAC reaction occurred between the N- or C-terminal amino acid residues of the center cores 110b, 210b. The solid dot 166 as depicted in Figure 2B represents the chemical bond formed between the N- or C-terminal amino acid residues.

Figure 2C is another example of the present disclosure. The linker units 100C, 200C have the similar structures as the linker units 100A, 200A, except that the coupling arms 130b, 230b respectively have an azide group 162 and a DBCO group 172, instead of the azide group 152 and the alkyne group 154 as depicted in the linker units 100A, 200A of Figure 2A. Specifically, the center core 110a is linked with a coupling arm 130b (as the first coupling arm) having an azide group 162 at its free-terminus; and the center core 210a is linked with a coupling arm 230b (as the second coupling arm) having a DBCO group 172 at its free-terminus. The linker units 100C, 200C are then coupled via the SPAAC reaction occurred between the coupling arms 130b, 230b; and forming the chemical bond 182, depicted as a diamond.

As would be appreciated, two linker units can be coupled to each other via the CuAAC reaction occurred between the center core and the coupling arm. Reference is now made to Figure 2D, in which the center core 110b comprises a N- or C-terminal amino acid residue that has an azide group 162 (e.g., the AHA residue), and the center core 210a is linked with a coupling arm 230b having a TCO group 172 at its free-terminus. Accordingly, the linker units 100B and 200C can be coupled via the SPAAC reaction occurred between the center core 110b and the coupling arm 230b; and forming the chemical bond 182.

According to one example, the linker units 100B, 200C depicted in Figure 2D respectively comprise polypeptide cores.

Alternatively, the linker unit 200B that comprises a N- or C-terminal amino acid residue having an alkyne group 160b (e.g., the HPG residue), and the linker unit 100C comprising the coupling arm 130b with an azide group 160a at its free-terminus can be coupled together via the azide-alkyne cycloaddition occurred between the center core 210b and the coupling arm 130b.

Compared with other therapeutic construct, the present molecular construct is advantageous in at least the three following aspects:

(1) the linker unit comprising a specified number and/or type of targeting/effector element can be prepared independently, then proceed to be coupled together via the CuAAC reaction, the iEDDA reaction, or the SPAAC reaction;

(2) the number and kind of the targeting and/or effector elements may vary in accordance with the requirements of application (e.g., the disease being treating, and the binding avidity and/or affinity of the targeting and/or effector element). The combination of the targeting and effector elements may be adjusted according to specific needs and/or applications. Each of the present targeting and effector elements may vary with such factors like particular condition being treated, the physical condition of the patient, and/or the type of disease being treated. The clinical practitioner may combine the most suitable targeting element and the most suitable effector element so as to achieve the best therapeutic effect. According to examples of the present disclosure, the targeting element may be a growth factor, a peptide hormone, a cytokine, or an antibody fragment; and the effector element may be an immunomodulant, a chelator complexed with a radioactive nuclide, a cytotoxic drug, a cytokine, a soluble receptor, or an antibody; and

(3) compared with other coupling reactions, the CuAAC reaction, the iEDDA reaction, or the SPAAC reaction is more efficient in terms of coupling any two linker units.

Reference is now made to Figure 3, in which six libraries are illustrated, and are prepared independently. In this example, Libraries 1-6 respectively comprise a plurality of linker units 300A, 300B, 300C, 400A, 400B, and 400C that are linked with functional elements. Each linker units 300A, 300B, and 300C are similar in structures; in which each of the linker units 300A, 300B, and 300C comprises one center core 310, one coupling arm 330 linked thereto and has a tetrazine group 350 at its free terminus, and a specified number of the linking arm 320. For instance, Linker unit 300A comprises four linking arms 320, and accordingly, four targeting elements 340a can be respectively linked to the four linking arms 320. Similarly, two targeting elements 340b and five targeting elements 340c can be respectively linked to the linker units 300B and 300C. The targeting elements 340a, 340b, and 340c can be the same or different. As to the linker units 400A, 400B, and 400C, each of these linker units comprises one center core 410, one coupling arm 430 linked thereto and has a strained alkyne group 450 at its free terminus, and a specified number of the linking arm 420. As depicted, three effector elements 440a, five effector elements 440b, and eight effector elements 440c can be respectively linked to the linker units 400A, 400B, and 400C. The effector elements 440a, 440b, and 440c can be the same or different. The Libraries 1-6 may be prepared independently. One skilled artisan may select the first linker unit from Libraries 1, 2 and 3, and the second linker unit from Libraries 4, 5, and 6, then proceed to couple the first and second linker units via the iEDDA reaction occurred between the tetrazine group 350 and the strained alkyne group 450 so as to produce the molecular construct with the specified number of targeting and effector elements.

Based on the library concept, the present molecular construct can be produced with different configurations depending on the libraries selected. Figure 4A provides an example of the present molecular construct, in which each of the first and second center cores (310, 410) is linked with three linking arms (320, 420) and one coupling arm (330, 340). Three of the first targeting elements 340 are respectively linked to the linking arms 320; and three of the first effector elements 440 are respectively linked to the linking arms 420. The two linker units are coupled to each other via the iEDDA reaction occurred between two coupling arms 330, 430, and forming the chemical bond 356. By this configuration, equal numbers of multiple targeting and/or effector elements may be carried in one molecular construct.

Figure 4B provides another example of the present molecular construct, in which the first and second center cores respectively contain different numbers of amine groups (e.g., lysine residues), and accordingly, the molecular construct contains non-equal numbers of targeting and effector elements. In the depicted example, the first center core 310 is linked to one coupling arm 330, and two linking arms 320. The second center core 410 is linked to one coupling arm 430, and five linking arms 420. Accordingly, two targeting elements 340 are respectively linked to the linking arms 320; and five effector elements 440 are respectively linked to the linking arms 420. The ellipse 356 in Figure 4B represents the linkage between two coupling arms 330, 430.

Based on the concept, a linker unit may comprise a plurality of linking arms, which can be linked to a plurality of functional elements. For example, a linker unit may comprise 5-12 linking arms, which can be linked to 5-12 functional elements. This is especially useful when the functional elements are small molecules, such as cytotoxic drugs or toll-like receptor agonists. The linker unit carrying multiple molecules of a cytotoxic drug is herein referred to as a drug bundle.

Basically, the coupling arm of the present molecular construct described in above examples of the present disclosure that has an azide, alkyne, tetrazine, or strained alkyne group at the terminus is designed as a PEG chain having 2-12 repeats of EG units. The linking arm is designed as a PEG chain having 2-20 repeats of EG units.

Adopting a polypeptide as the center core provides versatility in the present molecular construct, in which multiple copies or types of targeting/effector elements may be present in one construct, accordingly, enhanced specificity of drug delivery and potency in the intended target sites are achieved. A large number of configurations can be adopted by employing the molecular construct comprising multiple linker units. A few examples are: a first linker unit carrying three scFvs targeting elements, and a second linker unit carrying 5 cytotoxic drugs; a first linker unit carrying three scFvs targeting elements, and a second linker unit carrying three scFvs effector elements; or a first linker unit carrying 2 bi-scFv

targeting elements, and a second linker unit carrying two scFvs effector elements.

Schemes 5-7 provide several working example respectively depicting the coupling and preparation of specified linker units.

Scheme 5 is a schematic diagram depicting the preparation of the present molecular construct in accordance with one example of the present disclosure, in which NHS represents NHS ester, Mal represents maleimide group, $A^{AH}$ represents L-azidohomoalanine (AHA) residue, AAH represents homopropargylglycine (HPG) residue, Ac represents acetyl group, and scFv represent single-chain variable fragment.

In step 1, the first center core comprising the amino acid sequence of $(GSK)_3$ and a L-azidohomoalanine (AHA) residue at the C-terminus thereof; and the second center core comprising the amino acid sequence of $(GSK)_5$ and a homopropargylglycine (HPG) residue at the C-terminus thereof, are respectively prepared. For the purpose of stabilizing the polypeptide, the N-terminuses of the first and second center cores are respectively modified with an acetyl group. In step 2, the linking arms are respectively linked to the lysine residues in the first and second center cores via forming an amide linkage there between; the linked arm linked to the center core has a maleimide group at the free-terminus. In step 3, the first targeting element (such as an scFv specific for fibrin) having a thiol group (e.g., a cysteine residue) is linked to the linking arm linked with the first center core via the thiol-maleimide reaction; similarly, the effector element (i.e., the drug, such as a Factor Xa inhibitor or thrombin inhibitor) having a thiol group is linked to the linking arm linked with the second center core via the thiol-maleimide reaction. In step 4, the two linker units are coupled via a CuAAC reaction occurred between the AHA and HPG residues.

**<<Scheme 5 Coupling of linker units via C-terminal amino acid residues>>**

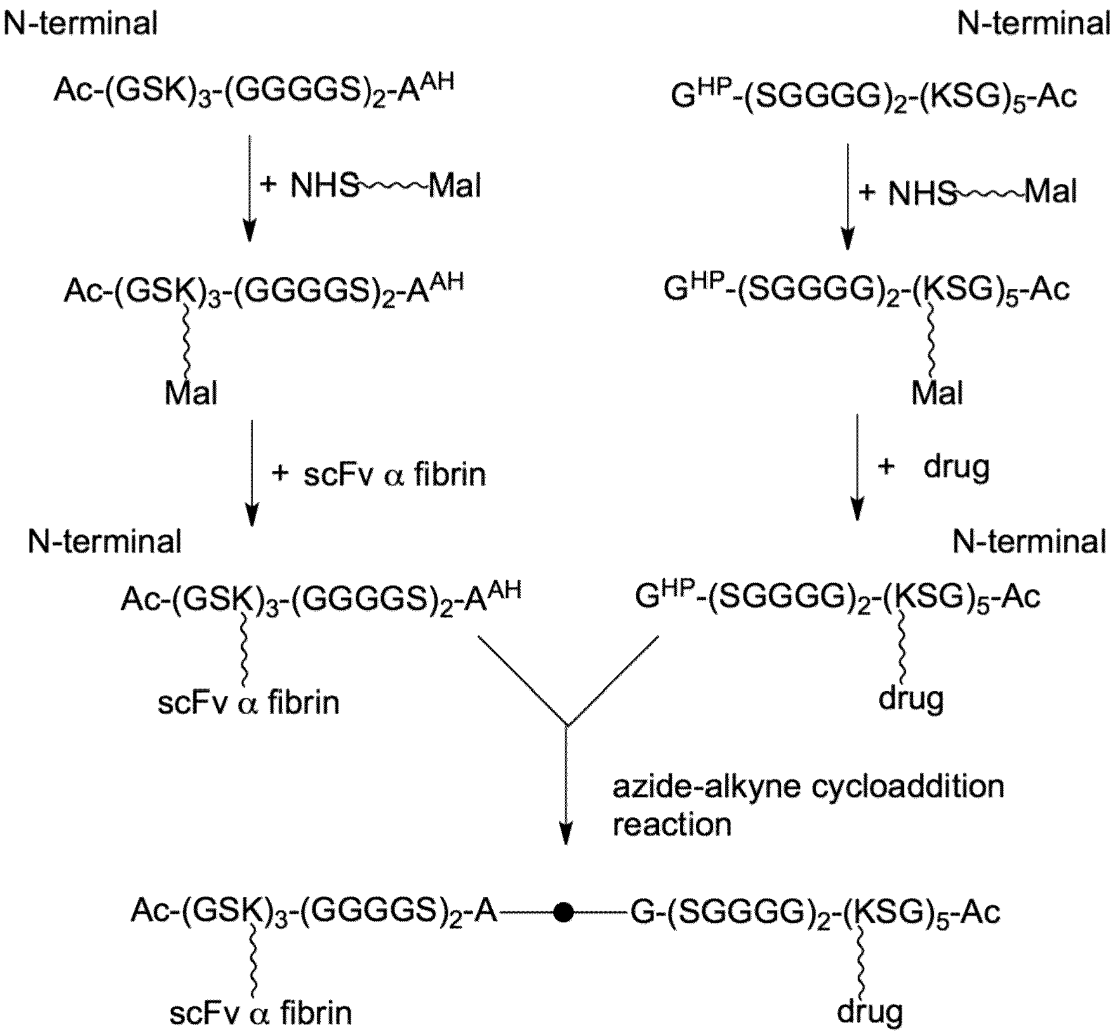


Optionally, the targeting/effector element can be linked to the center core in an alternative method. Scheme 6 is a scheme illustrating the coupling of the effector element with the polypeptide core, in which the linking arm is first linked to the center core, and then the effector element (i.e., the drug) is linked to the linking arm via the thiol-maleimide reaction.

**<<Scheme 6 Method of coupling of effector element with polypeptide core through linking to linking arms>>**

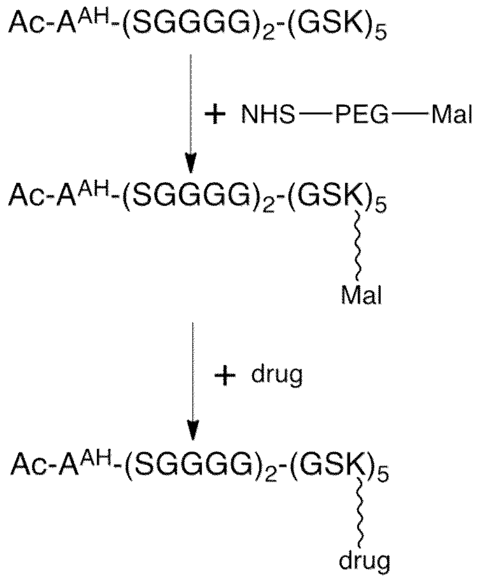


In the alternative method of scheme 7, the effector element (i.e., the drug) is coupled to the linking arm so as to produce a linking arm-effector conjugate (i.e., PEG-drug); next, the linking arm-effector conjugate is linked to the center core via forming an amide linkage between the lysine residues and the NHS esters.

**<<Scheme 7 Alternative method of coupling of effector element with polypeptide core by first conjugating with PEG chain and then linking to amino groups of lysine residues>>**

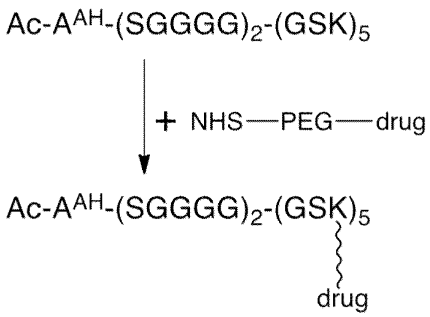


Alternatively, the linking arms for the joint-linker configuration may also be used to link bispecific scFv, which act as targeting elements or effector elements. These configurations will increase the specificity of targeting and/or the potency of the effector mechanisms.

When the targeting and effector elements are all scFv, and linking arms of 600 Daltons (12 EG units) are used, a molecular construct with a total of six scFvs has a molecular weight of about 170,000 Daltons. A molecular construct with seven scFvs has a molecular weight of about 200,000 Daltons, and a molecular construct with eight scFvs has a molecular weight of about 230,000 Daltons. Most of the molecular constructs of this invention have molecular weights smaller than 200,000 Daltons, and a few molecular constructs have molecular weights in 200,000-250,000 Daltons.

When four different sets of scFv are to be carried in one molecular construct, it is preferable to have one linker unit carrying a joined single-chain, bi-specific scFv (bi-scFv), such as scFv1-scFv2, and the other two linker units each carrying one scFv (i.e., scFv3 and scFv4 respectively). There are two ways to construct bi-specific scFv1-scFv2. In the "tandem" configuration, $V_L1$-$V_H1$-$V_L2$-$V_H2$ or $V_H1$-$V_L1$-$V_H2$-$V_L2$ is arranged; in the "diabody" configuration, $V_L2$-$V_L1$-$V_H1$-$V_H2$ or $V_H2$-$V_H1$-$V_L1$-$V_L2$ is arranged. Proper linkers with GGGGS (SEQ ID NO: 6) repeats or other sequences are placed between the immunoglobulin domains.

In our experience, a peptide or a PEG linker, which contain maleimide and azide groups may become polymerized upon long-term storage, due to the automatic coupling reaction between the maleimide and azide groups. Therefore, it is preferable that each linker unit is prepared freshly and independently, and processed to connecting the targeting or effector elements onto the linker units, and the coupling of the linker units through click reaction without delay. An alternative preferred embodiment is that the targeting elements and effector elements are both conjugated to linker units with alkyne groups, and the alkyne group in one of the linker units is then converted to azide with a short homo-bifunctional linker with azide at both ends. The linker units, one with alkyne and the other with azide, are then coupled via a click reaction.

### *II-(ii) Functional Elements Suitable for Use with Joint-linker Molecular Construct*

As discussed above, the present joint-linker comprises two linker units, in which the first linker unit carries one or more targeting elements, and the second linker unit carries one or more effector elements, and vice versa.

In constructing joint-linker molecular constructs for treating diseases/conditions associated with blood clots, one may use an scFv specific for fibrin as the targeting element. In the case where the prevention of blood clot formation is the main purpose, the present joint-linker molecular constructs may use Factor Xa inhibitors or thrombin inhibitors as the effector element. Illustrative examples of Factor Xa inhibitors include apixaban, edoxaban, and rivaroxaban. Non-limiting examples of thrombin inhibitors include argatroban and melagatran. For joint-linker molecular constructs aiming to treat thrombosis, tissue plasminogen activators, such as alteplase, reteplase, tenecteplase, and lanoteplase, can be used as the effector element.

## EXPERIMENTAL EXAMPLES

### Example 1: Synthesis of peptide 1 (SEQ ID NO: 18) as peptide core, and conjugation of SH group of cysteine residue with maleimide-$PEG_4$-tetrazine as conjugating arm

The synthesized peptide 1 (Chinapeptide Inc., Shanghai, China) was dissolved in 100 mM sodium phosphate buffer (pH 7.0) containing 50 mM NaCl and 5 mM EDTA at 2 mM final concentration. The dissolved peptide was reduced by 1 mM TCEP at 25°C for 2 hours. For conjugating the SH group of cysteine residue with maleimide-$PEG_4$-tetrazine (Conju-probe Inc.) to create a functional linking group tetrazine, the peptide and maleimide-$PEG_4$-tetrazine were mixed at a 1/5 ratio and incubated at pH 7.0 and 4°C for 24 hours. Tetrazine-conjugated peptides were purified by reverse phase HPLC on a Supelco C18 column (250 mm X 10 mm; 5 μm), using a mobile phase of acetonitrile and 0.1% trifluoroacetic acid, a linear gradient of 0% to 100% acetonitrile over 30 minutes, at a flow rate of 1.0 mL/min and a column temperature of 25°C.

The present tetrazine-peptide 1, as illustrated below, had a m.w. of 2,185.2 Daltons.

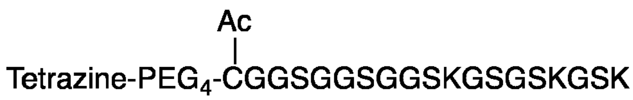

### Example 2: Synthesis of peptide 2 (SEQ ID NO: 26) as peptide core, and conjugation of the SH group of cysteine residue with maleimide-$PEG_3$-transcyclooctene (TCO) as a coupling arm

The synthesized peptide 2 (Chinapeptide Inc., Shanghai, China) was processed similarly. Briefly, the peptide was dissolved in 100 mM sodium phosphate buffer (pH 7.0) containing 50 mM NaCl and 5 mM EDTA at a final concentration of 2 mM. The dissolved peptide was reduced by 1 mM *tris*(2-carboxyethyl)phosphine (TCEP) at 25°C for 2 hours. For conjugating the SH group of the cysteine residue with maleimide-$PEG_3$-TCO (Conju-probe Inc.) to create a functional linking group TCO, the peptide and maleimide-$PEG_3$-TCO were mixed at a 1/7.5 ratio and incubated at pH 7.0 and 25°C for 18 hours. TCO-conjugated peptides were purified by reverse phase HPLC on a Supelco C18 column (250 mm X 10 mm; 5 μm), using a mobile phase of acetonitrile and 0.1% trifluoroacetic acid, a linear gradient of 0% to 100% acetonitrile over 30 minutes, at a flow rate of 1.0 mL/min and a column temperature of 25°C.

The identification of the synthesized TCO-peptide (illustrated below) was carried out by MALDI-TOF mass spectrometry. Mass spectrometry analyses were performed by the Mass Core Facility at the Institute of Molecular Biology (IMB), Academia Sinica, Taipei, Taiwan. Measurements were performed on a Bruker Autoflex III MALDI-TOF/TOF mass spectrometer (Bruker Daltonics, Bremen, Germany).

The thus-synthesized TCO-peptide 2, as illustrated below, had a m.w. of 2, 020.09 Daltons.

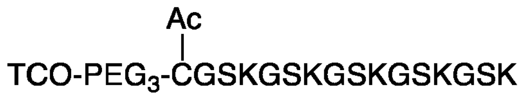

**Example 3: Synthesis of linker unit by conjugating NHS-$PEG_{12}$-maleimide to $NH_2$ groups of tetrazine-peptides 1**

Three linking arms of $PEG_{12}$-maleimide were attached to the peptide core tetrazine-peptide 1. The crosslinker, NHS-$PEG_{12}$-maleimide (succinimidyl-[(N-maleimido-propionamido)-dodecaethyleneglycol] ester, was purchased from Conju-probe Inc. The conjugation procedure was performed per the manufacturer's instruction; the peptide with lysine residues was dissolved in the conjugation buffer, phosphate buffered saline (PBS, pH 7.5) at 100 mM. NHS-$PEG_{12}$-maleimide crosslinker was added to the dissolved peptide at 1 mM final concentration (10-fold molar excess over 0.1 mM peptide solution). The reaction mixtures were incubated for 18 hours at room temperature. $PEG_{12}$-maleimide-conjugated tetrazine-peptide 1 was purified by reverse phase HPLC on a Supelco C18 column (250 mm X 4.6 mm; 5 μm), using a mobile phase of acetonitrile and 0.1% trifluoroacetic acid, a linear gradient of 0% to 100% acetonitrile over 30 minutes, at a flow rate of 1.0 ml/min and a column temperature of 25°C.

As illustrated below, the present $PEG_{12}$-maleimide-conjugated tetrazine-peptide 1 carried one coupling arm with a tetrazine group and three PEG linking arms with maleimide groups; it had a m.w. of 4,461 Daltons.

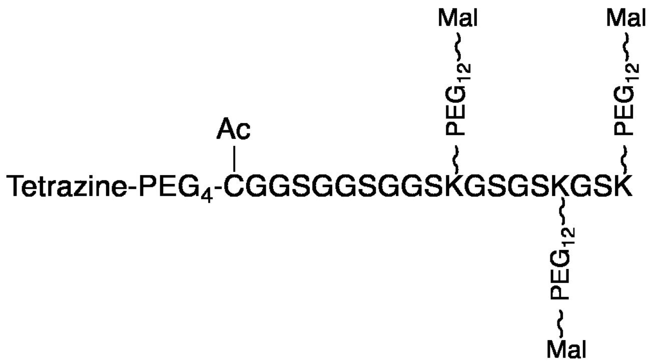

**Example 4: Synthesis of linker unit by conjugating NHS-$PEG_6$-Mal to $NH_2$ groups of TCO-peptide 2**

The procedure for conjugating NHS-$PEG_6$-Mal to $NH_2$ groups of TCO-peptide 2 was performed similarly as described in the previous Example. Briefly, NHS-$PEG_6$-maleimide crosslinker was added to the dissolved peptide at 40 mM final concentration (20-fold molar excess over 2 mM peptide solution). The reaction mixtures were incubated for 3 hours at room temperature.

The present $PEG_6$-maleimide-conjugated peptide 2, as illustrated below, had a m.w. of 4,478 Daltons; it was a peptide core-based linker unit carrying one TCO group and five PEG linking arms with maleimide groups (Figure 5).

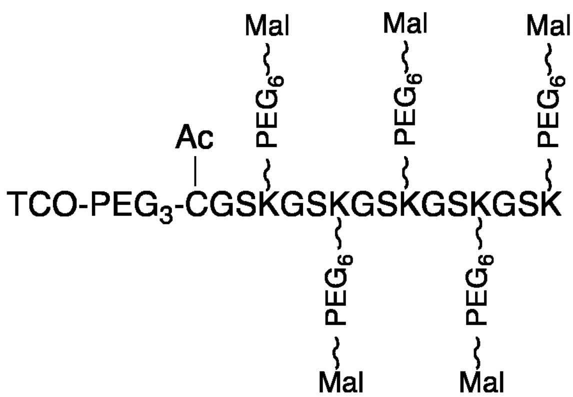

**Example 5: Conjugation of apixaban carboxylic acid molecule with $NH_2$-$PEG_3$-S-S-$PEG_3$-$NH_2$ crosslinker**

Apixaban carboxylic acid was purchased from KM3 Scientific Inc. (New Taipei City, Taiwan). The activated

carboxyl group of apixaban carboxylic acid molecule was reacted with a homo-bifunctional cleavable crosslinker, $NH_2$-$PEG_3$-S-S-$PEG_3$-$NH_2$ as shown in scheme 8.

Apixaban carboxylic acid was dissolved in 100% DMSO at a final concentration of 20 mM, and $NH_2$-$PEG_3$-S-S-$PEG_3$-$NH_2$, a homo-bifunctional cleavable crosslinker, was dissolved in PBS at a 10 mM final concentration. To activate the carboxyl group of apixaban carboxylic acid, 1-ethyl-3-(3-dimethylaminopropyl)carbodiimide hydrochloride (EDC) (KM3 Scientific Inc.) was added to the apixaban carboxylic acid solution at a molar ratio of 1:2 ([apixaban]:[EDC]) and then incubated for 15 minutes.

The activated apixaban carboxylic acid solution was added to the $NH_2$-$PEG_3$-S-S-$PEG_3$-$NH_2$ crosslinker at a 2 mM final concentration (5-fold molar excess over 0.4 mM $NH_2$-$PEG_3$-S-S-$PEG_3$-$NH_2$ crosslinker solution). The reaction mixture was incubated for 3 hours at room temperature.

## <<Scheme 8 Conjugation of two apixaban carboxylic acid molecules to an $NH_2$-$PEG_3$-S-S-$PEG_3$-$NH_2$ crosslinker>>

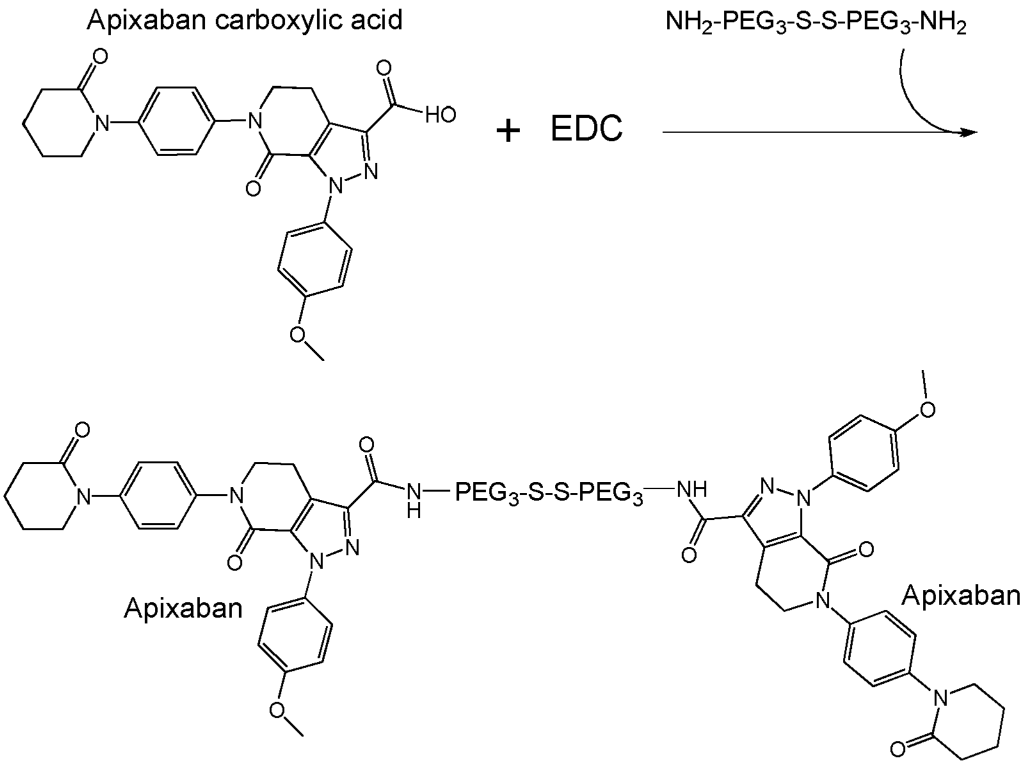


Apixaban-$PEG_3$-S-S-$PEG_3$-apixaban was purified by reverse phase HPLC on a Supelco C18 column (250 mm X 4.6 mm; 5 μm), using a mobile phase of acetonitrile and 0.1% trifluoroacetic acid, a linear gradient of 0% to 100% acetonitrile over 30 minutes, at a flow rate of 1.0 ml/min and a column temperature of 25°C.

The mass spectroscopic analysis of the thus-synthesized apixaban-$PEG_3$-S-S-$PEG_3$-apixaban (see, Figure 6) indicated that the molecular construct had m.w. of 1,301.64 and 1,323.68 Daltons, corresponding to $[M+H]^+$ and $[M+Na]^+$, respectively.

**Example 6: Conjugation of two argatroban molecules to an $NH_2$-$PEG_3$-S-S-$PEG_3$-$NH_2$ crosslinker**

Argatroban was purchased from KM3 Scientific Inc. (New Taipei City, Taiwan). The procedure for conjugating $NH_2$-$PEG_3$-S-S-$PEG_3$-$NH_2$ to COOH groups of argatroban molecule was performed similarly as described in the previous Example. Briefly, argatroban was dissolved in 100% DMSO at a final concentration of 20 mM. EDC solution was added to the argatroban solution to activate COOH group of argatroban and then incubated for 15 minutes. The activated argatroban solution was added to $NH_2$-$PEG_3$-S-S-$PEG_3$-$NH_2$ crosslinker solution at a 2 mM final concentration (5-fold molar excess over 0.4 mM $NH_2$-$PEG_3$-S-S-$PEG_3$-$NH_2$ crosslinker solution) (see, scheme 9).

## <<Scheme 9 Conjugation of argatroban molecule with an $NH_2$-$PEG_3$-S-S-$PEG_3$-$NH_2$ crosslinker>>

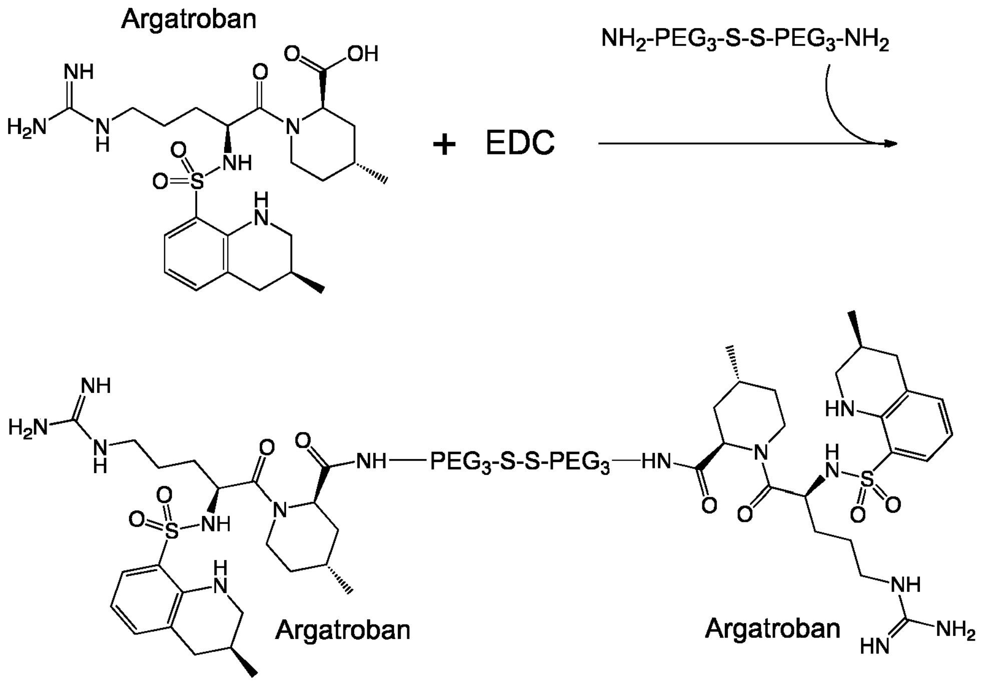


The MALDI-TOF result provided in Figure 7 shows that the thus-synthesized argatroban-$PEG_3$-S-S-$PEG_3$-argatroban had a m.w. of 1,378.61 Daltons.

**Example 7: Conjugation of apixaban-$PEG_3$-SH and argatroban-$PEG_3$-SH to maleimide-PEGs-conjugated TCO-peptide 2**

Prior to conjugation with the TCO-peptide 2 that had five maleimide-$PEG_6$ linking arms, apixaban-$PEG_3$-S-S-$PEG_3$-apixaban and argatroban-$PEG_8$-S-S-$PEG_8$-argatroban (prepared in the preceding Examples) were incubated with 4 mM TCEP at a molar ratio of 3:1 ([TCEP]:[drug-linker]) at room temperature for 90 minutes with gentle shaking to generate the apixaban-$PEG_3$-SH and argatroban-$PEG_3$-SH molecule with a free sulfhydryl group.

The thus-synthesized drug bundle, as illustrated below, was composed of a linker unit with a free TCO functional group and a set of five apixaban molecules as effector elements. The present molecular construct had a m.w. of 7,713 Daltons, corresponding to $[M+H]^+$.

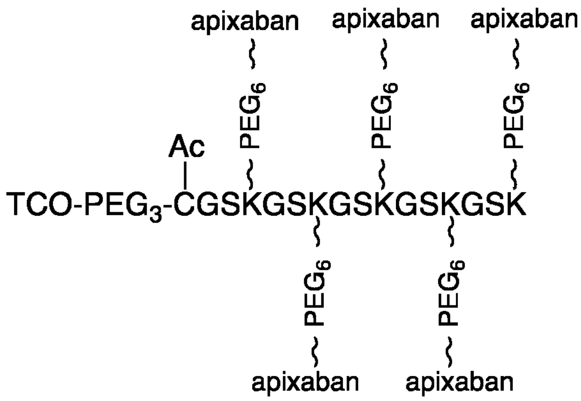


Another thus-synthesized drug bundle, as illustrated below, was composed of a linker unit with a free TCO functional group and a set of five argatroban molecules as effector elements. The present molecular construct had a m.w. of 8,112.8 Daltons, corresponding to $[M+H]^+$.

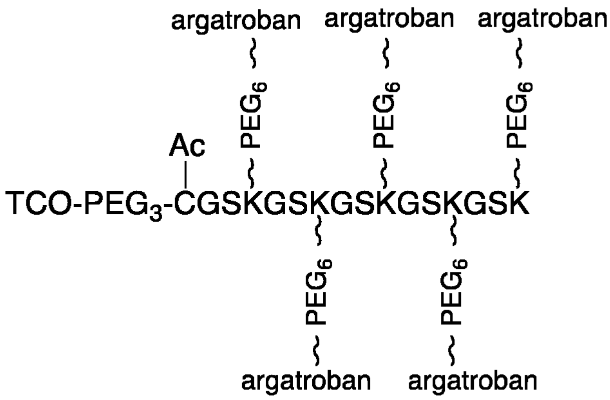

Example 8: Production of mouse scFv of mAb specific for human fibrin by Expi293F overexpression system

The $V_L$ and $V_H$ of the scFv specific for human fibrin were from mouse monoclonal antibody 102-10 (Japanese Patent Application Publication No.2012-72). The scFv derived from this antibody was designed to contain a flexible linker of GGGGSGGGGS and a terminal cysteine residue at the C-terminus. The cysteine residue provides a sulfhydryl group for conjugation with maleimide group present at the free ends of liking arms in various linker units. To produce the scFv of mAb specific for human fibrin, we used the $V_H$ and $V_L$ DNA sequences of mAb 102-10 with further codon optimization. DNA sequences encoding $V_L$-GSTSGSGKPGSGEGSTKG-$V_H$-$(GGGGS)_2$-C were synthesized. The amino acid sequence of the scFv of mAb 102-10 prepared for the experiments in the present invention is set forth in SEQ ID NO: 27.

For preparing scFv proteins using a mammalian expression system, we used the overexpression system based on Expi293F™ cell line for experimentation. The system employed ExpiFectamine™ 293 transfection kit (Life Technologies, Carlsbad, USA) consisting of the Expi293F™ cell line, the cationic lipid-based ExpiFectamine™ 293 Reagent and ExpiFectamine™ 293 transfection Enhancers 1 and 2, and the medium (Gibco, New York, USA).

The scFv-encoding sequence was placed in pG1K expression cassette. Expi293F cells were seeded at a density of $2.0 \times 10^6$ viable cells/ml in Expi293F expression medium and maintained for 18 to 24 hours prior to transfection to ensure that the cells were actively dividing at the time of transfection. On the day of transfection, $7.5\times10^8$ cells in 255ml medium in a 2-liter Erlenmeyer shaker flask were transfected by ExpiFectamine™ 293 transfection reagent. The transfected cells were incubated at 37°C for 16 to 18 hours post-transfection in an orbital shaker (125 rpm) and the cells were added ExpiFectamine™ 293 transfection enhancer 1 and enhancer 2 to the shaker flask, and incubated for another 5 to 6 days. Culture supernatants were harvested and scFv proteins in the media were purified using Protein L affinity chromatography. Figures 8A and 8B respectively show the results of SDS-PAGE and Mass spectrometric analysis of purified scFv of mAb specific for human fibrin. The scFv of mAb specific for human fibrin in SDS-PAGE migrated in two major bands of 26 and 30 kDa. The same protein solution was further analyzed by MALDI-TOF and showed that the 102-10 scFv specific for human fibrin had only the molecular weight of 26,838 Daltons, which is consistent with the calculated molecular weight.

**Example 9: ELISA analysis of purified mouse scFvs specific for human fibrin**

To prepare fibrinogen-coated plates, each plate was prepared according to procedures described in US patent application publication 2016/0011217A1. Briefly, 100 μl of human fibrinogen (Sigma) in PBS was added to 96-well flat-bottom plates (Nunc) at 1 μg/well, and the plate was sealed and allowed to stand at 4°C overnight.

The fibrin plate was prepared as follows. The fibrinogen solution was removed and then 100 μL of TBS containing 0.05 U/ml thrombin (Sigma), 2 mM $CaCl_2$ and 7 mM L-cysteine (Sigma) was added to the wells. The thrombin-treated plate was incubated at 37°C for 1 hour to allow fibrin formation. The thrombin solution was then removed and blocked with 10% skim milk at room temperature for 1 hour.

Then, 100 μl of the 102-10 scFv solution was added to the fibrinogen plate and the fibrin plate, which were then shaken at room temperature for 1 hour. After that, each plate was washed with TBS-T, and 50 μl of TMB (Thermo Fisher Scientific Inc., Waltham, USA) was added, and colorimetry was conducted. The reaction was stopped by adding 50 μl of 1N HCl. Then the absorbance (O.D.) was obtained by measuring the absorbance at 450 nm with a plate reader.

Figure 8C shows the ELISA result, indicating that the purified 102-10 scFv bound specifically to human fibrin, but not to fibrinogen.

**Example 10: Construction and selection of phage-displayed human scFvs specific for human fibrin**

The phage clones carrying the human scFv specific for human fibrin were obtained through a contractual arrangement with Dr. An-Suei Yang's laboratory at the Genomics Research Center, Academia Sinica, Taipei, Taiwan. The framework sequence of the GH2 scFv library was derived from a human IgG antibody fragment, G6 anti-VEGF Fab (Protein Bank Code 2FJG) and cloned into restriction sites *SfiI* and *NotI* of phagemid vector pCANTAB5E (GE Healthcare), carrying an ampicillin resistance, a lacZ promotor, a pelB leader sequence for secretion of scFv fragments into culture supernatants, and an E-tag applicable for detection. The $V_H$ and $V_L$ domains of the scFv template were diversified separately based on the oligonucleotide-directed mutagenesis procedure; the three CDRs in each of the variable domains were diversified simultaneously. The scFv library of over $10^9$ clones was used for selections on human fibrin.

The thrombin-treated fibrin plates (1 μg/100 μl per well) were prepared as described in the preceding Examples. The fibrin plates were used for panning anti-fibrin antibodies. In brief, the fibrin-coated wells were treated with blocking buffer (5% skim milk in PBST (phosphate buffered saline with 0.1% tween-20)) for 1 hour at room temperature. Recombinant phages in the blocking buffer diluted to $8\times10^{11}$ CFU/ml was added to the fibrin-coated wells for 1 hour with gentle shaking; CFU stands for colony-forming unit. The wells were then washed vigorously 10 times with PBST, followed by 6 times with PBS to remove nonspecific binding phages. The bound phages were eluted using 0.1 M HCl/glycine buffer at pH 2.2, and eluted fraction was neutralized immediately by 2 M Tris-base buffer at pH 9.0. *E. coli* strain ER2738 (OD600 = ~0.6) was used for phage infection at 37 °C for 30 minutes; non-infected *E. coli* was eliminated by treating with ampicillin for 30 minutes. After ampicillin treatment, helper phage M13KO7 carrying kanamycin resistance was added for another 1 hour incubation. The selected phages rescued by helper phage in the *E. coli* culture were amplified with vigorously shaking overnight at 37 °C in the presence of kanamycin. The amplified phages were precipitated in PEG/NaCl, and then resuspended in PBS for the next selection-amplification cycle. A total of three consecutive panning rounds were performed on human fibrin by repeating this selection-amplification procedure.

Phage-infected ER2738 colonies were enumerated by serial dilution series were counted and phage titers were calculated, yielding the output titer/ml (CFU/ml) per panning round. A 1000-fold increase in phage output title from 2.5E+06 CFU/well to 4.3E+09 CFU/well was obtained after three rounds of panning. The phage output/input titer ratios from each round are shown in Figure 9A. For each panning round, the phage output/input titer ratios are given on the y-axis. There was clear enrichment of the positive clones over the three rounds of panning. The third panning round resulted in a 500-fold on the ratios of phage output/input titer over the first round, as the binding clones became the dominant population in the library.

In a typical selection procedure, after three rounds of antigen-panning on human fibrin-coated wells in ELISA plates, approximately 80% of the bound phage particles bound to fibrin specifically in ELISA with coated fibrin.

**Example 11: Single colony ELISA analysis of human phage-displayed scFvs specific for human fibrin**

*E. coli* strain ER2738 infected with single-clonal phages each harboring a selected scFv gene in its phagemid was grown in the mid-log phase in 2YT broth (16 g/L tryptone, 10 g/l yeast extract, 5 g/l NaCl, pH 7.0) with 100 μg/ml ampicillin in deep well at 37 °C with shaking. After broth reaching an OD600 of 1.0, IPTG was added to final concentration of 1 μg/ml. The plates were incubated at 37 °C overnight with rigorously shaking. After overnight incubation at 37°C with vigorous shaking, the plates were centrifuged at 4,000 g for 15 minutes at 4°C.

For soluble scFv binding test, ELISA was carried out. In brief, 96-well Maxisorp 96-well plate (Nunc) was coated with fibrin (1 μg/100 μl PBS per well) or a negative control antigen human fibrinogen for 18 hours with shaking at 4°C. After treated with 300 μl of blocking buffer for 1 hour, 100 μl of secreted scFv in the supernatant was mixed with 100 μl of blocking buffer and then added to the coated plate for another 1 hour. Goat anti-E-tag antibody (conjugated with HRP, 1:4000, Cat. No. AB19400, Abcam) was added to the plate for 1 hour. TMB substrate (50 μl per well) was added to the wells and the absorbance at 450 nm was measured after reactions were stopped by adding 1N HCl (50 μl per well).

A total of 960 phage clones after the 3rd round of panning were subjected to the present analysis. Among them, six scFv clones that bound to fibrin with a differential of OD450 greater than 10 over fibrinogen were further characterized by DNA sequencing of their encoding scFv genes. Four different DNA sequences were identified. Figure 9B shows the ELISA result of an scFv clone D10. The amino acid sequence of an scFV clone D10, which binds to human fibrin with an OD450 of 1.09, is shown in as SEQ ID NO: 29.

**Example 12: Production of recombinant reteplase by Expi293F overexpression system**

The amino acid sequence of reteplase was from DrugBank. The recombinant protein was designed to contain a flexible linker of GGGGSGGGGS and a terminal cysteine residue at the C-terminus. The cysteine residue provides a sulfhydryl group for conjugation with maleimide group present at the free ends of linking arms in various linker units. The amino acid sequences of reteplase prepared for the experiments of the invention are set forth in SEQ ID NO: 28.

In this Example, the gene-encoding sequence was placed in pcDNA3 expression cassette. For preparing reteplase protein using a mammalian expression system, we used the overexpression system based on Expi293F™ cell line for experimentation as described in the above Examples.

Figures 10A and 10B respectively show results of SDS-PAGE and mass spectrometric analyses of purified reteplase. The recombinant reteplase in SDS-PAGE migrated in two major bands of 43 and 48 kDa. The same protein solution was further analyzed by MALDI-TOF and showed that the recombinant reteplase had only a molecular weight of 43,415 Daltons, which is consistent with the calculated molecular weight.

**Example 13: Preparation of TCO-conjugated reteplase**

For the conjugation of SH group of reteplase with Mal-$PEG_3$-TCO (Conju-probe, Inc.), the cysteine residue at the C-terminal end of the purified reteplase was reduced by incubating with 5 mM dithiothreitol (DTT) at room temperature for 4 hours with gentle shaking. The buffer of reduced proteins was exchanged to sodium phosphate buffer (100 mM sodium phosphate, pH7.0, 50 mM NaCl, and 5 mM EDTA) by using NAP-10 Sephadex G-25 column. After the reduction reaction and buffer exchange, conjugation was conducted overnight at room temperature in a reaction molar ratio of 10:1 ([Mal-$PEG_3$-TCO:[protein]]. The excess crosslinker was removed by a desalting column and the TCO-conjugated protein product was analyzed.

The results of mass spectroscopy MALDI-TOF analysis indicated that the sample of TCO-conjugated reteplase protein had a m.w. of 45,055 Daltons. The purity of TCO-conjugated reteplase protein was identified through Coomassie blue staining of 10% SDS-PAGE. Figure 11 shows mass spectrometric analysis of TCO-conjugated reteplase.

**Example 14: Conjugation of three scFvs specific for human fibrin to the three maleimide-$PEG_{12}$ linking arms based on tetrazine-peptide 1**

The DNA sequence encoding SEQ ID NO: 27 was synthesized and expressed as in the above Examples. Prior to conjugation with the tetrazine-peptide 1 that had three $PEG_{12}$-maleimide linking arms, the cysteine residue at the C-terminal end of the purified 102-10 scFv of mAb specific for human fibrin was reduced by incubating with 5 mM DTT at a molar ratio of 2:1 ([DTT]:[scFv]) at room temperature for 4 hours with gentle shaking. Subsequently, the buffer of the reduced 102-10 scFv was exchanged to maleimide-SH coupling reaction buffer (100 mM sodium phosphate, pH 7.0, 50 mM NaCl and 5 mM EDTA) by using an NAP-10 Sephadex G-25 column (GE Healthcare). After the reduction and buffer exchange, the conjugation to the tetrazine-peptide 1 having three $PEG_{12}$-maleimide linking arms was conducted overnight at 4°C at a molar ratio of 1:4 ([linker]:[Protein]).

The reaction mixture was applied to a size exclusion chromatography column S75. The $PEG_{12}$-maleimide-conjugated tetrazine-peptide 1 conjugated with three 102-10 scFvs specific for human fibrin was separated from the free scFv, free $PEG_{12}$-maleimide-conjugated tetrazine-peptide 1 and the $PEG_{12}$-maleimide-conjugated tetrazine-peptide 1 conjugated with one and two 102-10 scFvs specific for human fibrin by size exclusion chromatography column S75. The product (i.e., the $PEG_{12}$-maleimide-conjugated tetrazine-peptide 1 having a free tetrazine functional group and being conjugated with a set of three 102-10 scFvs specific for human fibrin) was purified and shown in the 10% SDS-PAGE analysis shown in Figure 12.

**Example 15: Analysis of a targeting linker unit containing three scFvs specific for human fibrin linked to the three maleimide-$PEG_{12}$ linking arms based on tetrazine-peptide 1 by MALDI-TOF**

The sample of the targeting linker unit of three102-10 scFvs specific human fibrin linked to the three maleimide-$PEG_{12}$ linking arms based on tetrazine-peptide 1 was analyzed by MALDI-TOF. The median of the experimental molecular weight was consistent with the median of theoretical molecular weight of three 102-10 scFvs specific for human fibrin conjugated to tetrazine-peptide 1 with three maleimide-$PEG_{12}$ linking arms. According to the mass spectrometric profile in Figure 13, the synthesized targeting linker unit had the median molecular weight of 84,974 Daltons.

Illustrated below is the synthesized targeting linker unit that was composed of a linker unit with a free tetrazine functional group and a set of three 102-10 scFvs specific for human fibrin as targeting elements.

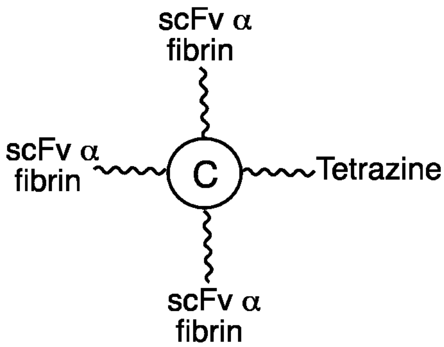


**Example 16: Preparation of molecular construct with three scFvs specific for human fibrin as targeting elements and one reteplase molecule as an effector element**

In this example, the targeting linker unit of the preceding examples and a TCO-conjugated reteplase protein was coupled via a tetrazine-TCO iEDDA reaction. Specifically, the targeting linker unit had three 102-10 scFvs specific for human fibrin and one free tetrazine group.

The procedure for tetrazine-TCO ligation was performed per the manufacturer's instructions (Jena Bioscience GmbH, Jena, Germany). Briefly, 100 μl of the targeting linker unit (0.3 mg/ml) was added to the solution containing the effector element at a molar ratio of 1:1.2 ([tetrazine]:[TCO]). The reaction mixture was incubated for 1 hour at room temperature.

Illustrated below is the present joint-linker molecular construct with three 102-10 scFvs specific for human fibrin as targeting elements and with a reteplase molecule as effector elements. In 8% SDS-PAGE analysis of the reaction mixture, a band of about 180 kDa in size was observed.

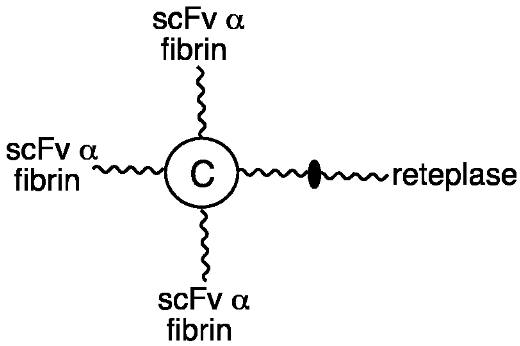


**Example 17: Preparation of molecular construct with three scFvs specific for human fibrin as targeting elements and five apixaban or argatroban molecules as an effector elements**

In this example, a joint-linker molecular construct with three 102-10 scFvs specific for human fibrin and a drug bundle of five apixaban molecules was constructed. The molecular construct was made by a TCO-tetrazine iEDDA reaction as described in the preceding Examples. Briefly, 100 μl of the targeting linker unit (0.3 mg/ml) was added to the solution containing the effector element at a molar ratio of 1:1.2 ([tetrazine]:[TCO]). The reaction mixture was incubated for 1 hour at room temperature.

The resultant joint-linker molecular construct, as illustrated below, had three 102-10 scFvs specific for human fibrin as targeting elements and with a drug bundle of five apixaban molecules as effector elements. In 10% SDS-PAGE analysis of the reaction mixture, a band of about 160 kDa in size was observed.

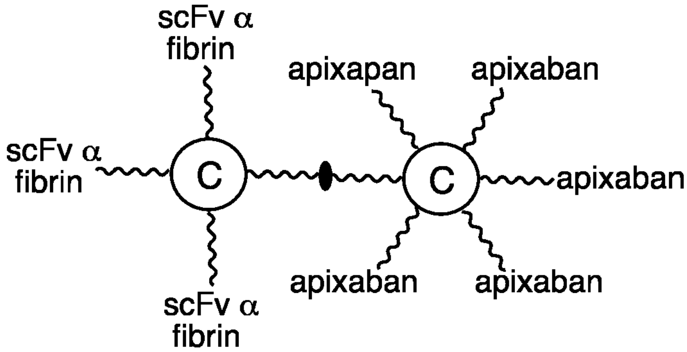


Illustrated below is the present joint-linker molecular construct with three 102-10 scFvs specific for human fibrin as targeting elements and with a drug bundle of five argatroban molecules as effector elements. In 10% SDS-PAGE analysis of the reaction mixture, a band of about 165 kDa in size was observed.

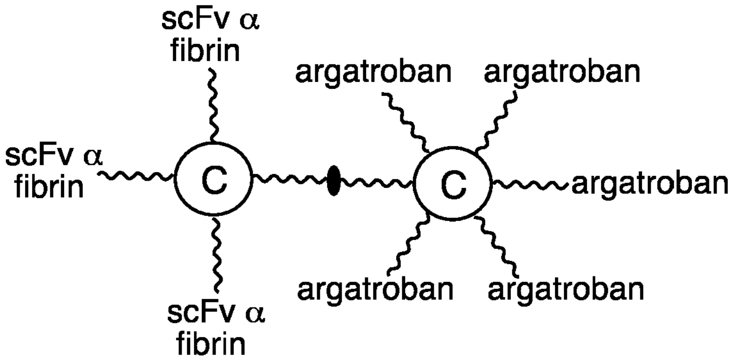


**Example 18: Inhibition assay of apixaban-PEG$_3$-SH molecule**

Factor Xa catalyzes the conversion of inactive prothrombin to active thrombin. Apixaban has been used as a Factor Xa inhibitor, indirectly to decrease clot formation induced by thrombin. The synthesis of the modified apixaban molecule (apixaban-PEG$_3$-SH) has been shown in the preceding examples. To examine the inhibitory activities of the modified apixaban molecule (apixaban-PEG$_3$-SH), Factor Xa inhibition assay (BioVision, Milpitas, USA) was performed. The Factor Xa inhibition assay utilizes the ability of Factor Xa to cleave a synthetic substrate thereby releasing a fluorophore, which can be detected by a fluorescence reader. In the presence of a Factor Xa inhibitor, the extent of cleavage reaction catalyzed by Factor Xa is reduced or completely abolished.

In this example, 50 μl of Factor Xa enzyme solution (provided by manufacturer) was added to the 96-well flat-bottom plate (Nunc). 10 μl of 1 μM apixaban-PEG$_3$-SH and apixaban carboxylic acid were added to the plate contained Factor Xa enzyme solution and incubated for 15 minutes at room temperature. Then, 40 μl of Factor Xa substrate solution (provided by manufacturer) was added to the plate and incubated at 37°C for 30 minutes. The fluorescence intensity of fluorophores (relative fluorescence units, RFU) was obtained by measuring the emission at 450 nm under the excitation at 350 nm with fluorescence plate reader.

Figure 14 shows the assay results of the inhibitory activity of apixaban-PEG$_3$-SH. In the presence of synthetic substrate, Factor Xa activity was measured in the absence of Factor Xa inhibitor (substrate only). The result indicates that the apixaban molecule conjugated with a connecting arm had a similar biological activity to inhibit action of factor Xa as the unmodified apixaban carboxylic acid. A Factor Xa inhibitor (GGACK Dihydrochloride, provided by manufacturer) is used as the control inhibitor.

**Example 19: Assay of biological activity of recombinant reteplase**

Reteplase is a recombinant human tissue plasminogen activator that catalyzes the conversion of plasminogen to plasmin; this process is involved in breakdown of blood clots. To investigate the biological activity of the recombinant reteplase, a chromogenic assay in 96-well flat-bottom plate was performed.

Briefly, 1 μl of 1 μM recombinant reteplase, 25 μl of 10 μM human plasminogen (Cat. No.7549-1, Biovision) and 62.5 μl of 100 mM Tris buffer at pH 8.5 were added and incubated in the well of the plate at 37°C for 30 minutes. Next, 1 μl of 50 mM chromogenic substrate D-Val-Leu-Lys-p-Nitroanilide dihydrochloride (Cat. No.V7127, Sigma), a synthetic plasmin

substrate, was added to the well and incubated at 25°C for 30 minutes. 31.5 μl of 10 % citric acid was then added to each well to stop the reaction. The recombinant reteplase catalyzes plasminogen to form plasmin, which in turn cleaves the chromogenic substrate to release yellow colored p-Nitroanilide, which was measured at 405 nm by a plate reader.

The result shows that recombinant reteplase exhibited a protease activity with an OD405 of 1.8, whereas the positive control protein, the commercially available tPA protein (Cat. No.T0831, Sigma) had an OD405 of 1.5.

**Example 20: Construction of a gene segment encoding 2-chain IgG4.Fc fusion protein containing reteplase**

The reteplase-CH2-CH3 (human γ4) recombinant chain was configured by fusing reteplase to the N-terminal of CH2 domain of IgG4.Fc through a flexible hinge region. The sequence of the recombinant chain in the IgG4.Fc fusion protein molecular construct is shown as SEQ ID NO: 30.

Illustrated below is the configuration of the prepared 2-chain (reteplase)-higG4.Fc molecular construct.

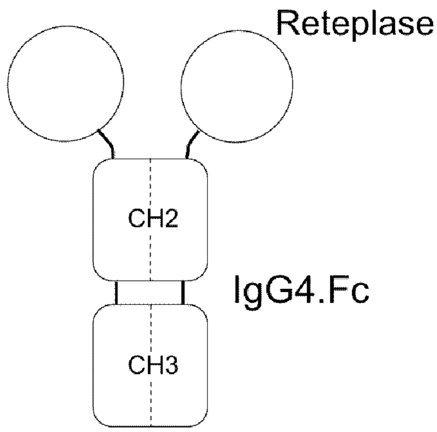


**Example 21: Expression and purification of recombinant 2-chain IgG4.Fc fusion protein containing reteplase**

In this Example, the gene-encoding sequence was placed in pcDNA3 expression cassette. Expi293F cells were seeded at a density of $2.0 \times 10^6$ viable cells/ml in Expi293F expression medium and maintained for 18 to 24 hours prior to transfection to ensure that the cells were actively dividing at the time of transfection. At the time of transfection, $7.5 \times 10^8$ cells in 255-ml medium in a 2-liter Erlenmeyer shaker flask were transfected by ExpiFectamine™ 293 transfection reagent. The transfected cells were incubated at 37°C for 16 to 18 hours post-transfection in an orbital shaker (125 rpm) and the cells were added ExpiFectamine™ 293 transfection enhancer 1 and enhancer 2 to the shaker flask, and incubated for 7 days. Culture supernatants were harvested and recombinant 2-chain (reteplase)-hlgG4.Fc fusion protein in the media was purified using Protein A chromatography. Following buffer exchange to PBS, the concentration of (reteplase)-hlgG4.Fc protein was determined and analyzed by 8% SDS-PAGE shown in Figure 15. The Fc-fusion molecular construct was revealed as the major band at about 74 kDa, consistent with the expected size.

**Example 22: Construction of a gene segment encoding 2-chain IgG4.Fc fusion protein containing reteplase and scFv specific for human fibrin**

The reteplase-CH2-CH3-scFv (human γ4) recombinant chain was configured by fusing reteplase to the N-terminal of CH2 domain of IgG4.Fc through a flexible hinge region, and the 102-10 scFv specific for human fibrin was fused to the C-terminal of CH3 domain through a flexible linker, $(GGGGS)_3$.

The scFvs had an orientation of $V_L$-linker-$V_H$. The $V_L$ and $V_H$ in the scFv were connected by a hydrophilic linker, GSTSGSGKPGSGEGSTKG. The sequence of the recombinant chain in the IgG4.Fc fusion protein molecular construct is shown as SEQ ID NO: 31. The expression of the constructed gene in Expi293F cells and the purification of the expressed fusion protein were performed as in preceding Examples. Characterization of the new construct was performed with SDS-PAGE. The 8% SDA-PAGE results in Figure 16 shows that the recombinant chain of the new construct has a size of about 100 kDa, consistent with the expected size.

Illustrated below is the configuration of the prepared 2-chain (reteplase)-hlgG4.Fc-(scFv α fibrin) molecular construct.

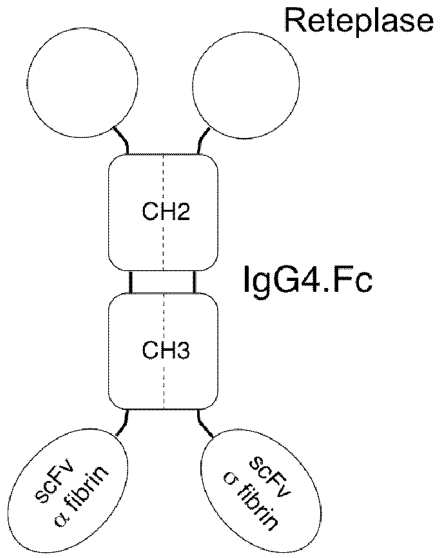


**Example 23: Construction of a gene segment encoding fusion protein containing reteplase and scFv specific for human fibrin**

The (reteplase)-scFv recombinant chain was configured by fusing reteplase to the N-terminal of the 102-10 scFv specific for human fibrin through a flexible linker, $(GGGGS)_3$.

The scFv had an orientation of $V_L$-linker-$V_H$. The $V_L$ and $V_H$ in the scFv were connected by a hydrophilic linker, GSTSGSGKPGSGEGSTKG. The sequence of the recombinant fusion protein molecular construct is shown as SEQ ID NO: 32. Characterization of the new construct was performed with SDS-PAGE. The 8% SDA-PAGE results in Figure 17 shows that the recombinant chain of the new construct has a size of about 72 kDa, consistent with the expected size.

Illustrated below is the configuration of the prepared (reteplase)-(scFv α fibrin) molecular construct.

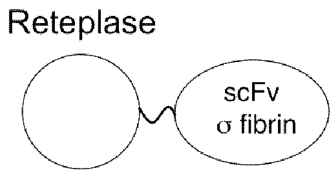


**Example 24: Construction of a gene segment encoding 2-chain IgG4.Fc fusion protein containing tenecteplase (TNK-tPA)**

The (TNK-tPA)-CH2-CH3 (human γ4) recombinant chain was configured by fusing TNK-tPA to the N-terminal of CH2 domain of IgG4.Fc through a flexible hinge region. The sequence of the recombinant chain in the IgG4.Fc fusion protein molecular construct is shown as SEQ ID NO: 33. The 8% SDA-PAGE assay results in Figure 18 shows that the recombinant chain of the new construct has a size of about 98 kDa, consistent with the expected size.

Illustrated below is the configuration of the prepared 2-chain (TNK-tPA)-hIgG4.Fc molecular construct.

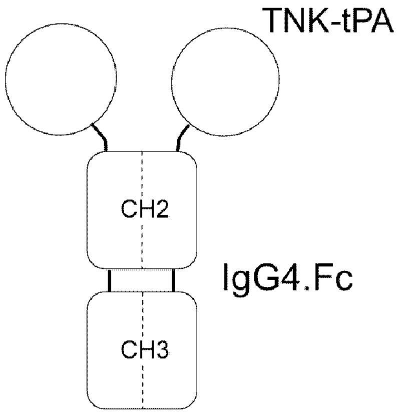


**Example 25: Construction of a gene segment encoding 2-chain IgG4.Fc fusion protein containing TNK-tPA and human scFv specific for human fibrin**

The (TNK-tPA)-CH2-CH3-scFv (human γ4) recombinant chain was configured by fusing TNK-tPA to the N-terminal of CH2 domain of IgG4.Fc through a flexible hinge region, and the human scFv D10 specific for human fibrin was fused to the C-terminal of CH3 domain through a flexible linker, $(GGGGS)_3$.

The scFvs had an orientation of $V_L$-linker-$V_H$. The $V_L$ and $V_H$ in the scFv were connected by a hydrophilic linker, GSTSGSGKPGSGEGSTKG. The sequence of the recombinant chain in the IgG4.Fc fusion protein molecular construct is shown as SEQ ID NO: 34.

To detect the recombinant 2-chain (TNK-tPA)-hIgG4.Fc-(scFv α fibrin) expressed at low level, In-gel digestion of protein isolated by gel electrophoresis and tandem mass spectrometric analysis of trypsin-digested 2-chain (TNK-tPA)-hIgG4.Fc-(scFv α fibrin) were performed for the identification of the molecular constructs. All mass spectrometry experiments were done using a Bruker Autoflex III MALI TOF/TOF mass spectrometer (Bremen, Germany) equipped with a 200 Hz Smart Bean Laser in positive ion mode with delayed extraction in the reflection mode. Data acquisition was done manually with Flex Control 3.4, and data processing was performed with Flex-Analysis 3.4 (both Bruker Dalton).

To identify the peptide by molecular mass searching of protein fragment in protein database with the Mascot search engine, the m/z values of two protein fragments in MS/MS spectrum, corresponding to 1,617.8223 and 1,053.5074 Daltons, were matched to the amino acid sequences of two TNK-tPA fragments, VYTAQNPSAQALGLGK and QYSQPQFR. The m/z value of a protein fragment in MS/MS spectrum, corresponding to 830.4496 Daltons, was matched to the amino acid sequence of a peptide fragment in the human IgG4.Fc region, GLPSSIEK. The m/z values of two protein fragments in MS/MS spectrum, corresponding to 737.3866 and 620.3044 Daltons, were matched to the amino acid sequences of two peptide fragments in the D10 scFv region, NTAYLR and MNSLR.

Illustrated below is the configuration of the prepared 2-chain (TNK-tPA)-hIgG4.Fc-(scFv α fibrin) molecular construct.

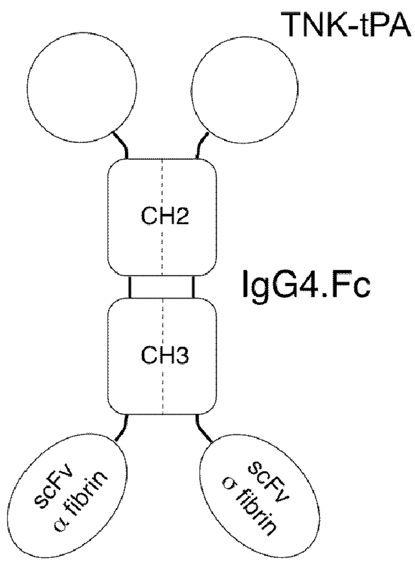

**Example 26: Construction of a gene segment encoding fusion protein containing TNK-tPA and scFv specific for human fibrin**

The (TNK-tPA)-(scFv α fibrin) recombinant chain was configured by fusing TNK-tPA to the N-terminal of the human scFv D10 specific for human fibrin through a flexible linker, $(GGGGS)_3$.

The scFvs had an orientation of $V_L$-linker-$V_H$. The $V_L$ and $V_H$ in the scFv were connected by a hydrophilic linker, GSTSGSGKPGSGEGSTKG. The sequence of the recombinant fusion protein molecular construct is shown as SEQ ID NO: 35. To detect the recombinant (TNK-tPA)-(scFv α fibrin) expressed at low level, In-gel digestion of protein isolated by gel electrophoresis and tandem mass spectrometric analysis of trypsin-digested (TNK-tPA)-(scFv α fibrin) were performed and confirmed for the identification of the present molecular constructs (see, the above Example).

Illustrated below is the configuration of the prepared (TNK-tPA)-(scFv α fibrin) molecular construct.

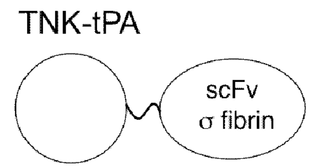


**Example 27: Assay of biological activity of fusion protein containing reteplase and scFv specific for human fibrin**

Reteplase is a recombinant human tissue plasminogen activator involved in breakdown of blood clots. To investigate the biological activity of the recombinant reteplase-containing proteins fused with scFv 102-10 specific for human fibrin, a chromogenic assay in fibrin-coated plate was performed (scheme 10).

**<<Scheme 10 Chromogenic assay of recombinant reteplase-containing proteins fused with scFv specific for human fibrin>>**

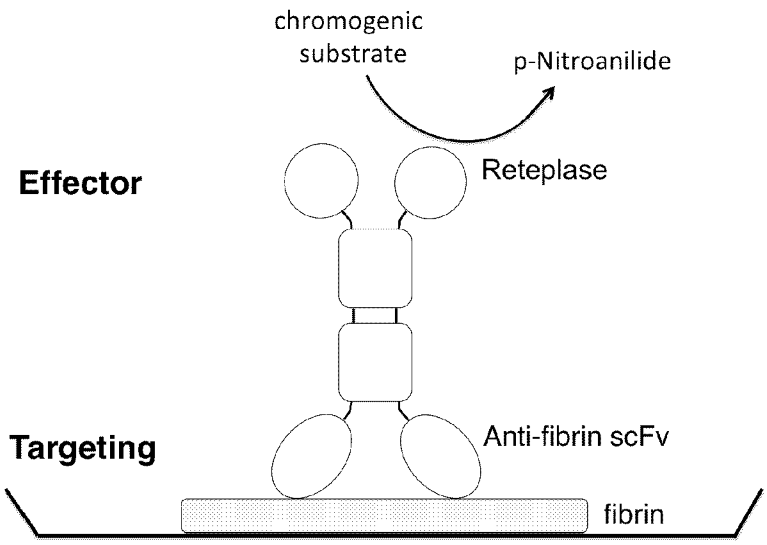


Briefly, to prepare fibrinogen plate, 100 μl of human fibrinogen (Sigma) in PBS was added 96-well flat-bottom plates (Nunc) at 10 μg/well; the plate was then sealed and allowed to stand at 4°C overnight. The fibrin plate was prepared as follows. The fibrinogen solution was removed, and then 100 μL of TBS containing 0.05 U/ml thrombin (Sigma), 2 mM $CaCl_2$ and 7 mM L-cysteine (Sigma) was added to the wells. The thrombin-treated plate was incubated at 37°C for 1 hour to allow fibrin formation. The thrombin solution was removed and blocked with 10% skim milk at room temperature for 1 hour.

Then, 100 μl of sample solution was added to the fibrinogen plate and the fibrin plate, which were then shaken at room temperature for 1 hour. After that, each plate was washed twice with PBST, followed by one PBS washing to remove nonspecific binding proteins. 25 μl of 10 μM human plasminogen (Cat. No.7549-1, Biovision) was added and incubated in each well of the fibrinogen and fibrin plates at 37°C for 30 minutes. 62.5 μl of 100 mM Tris buffer at pH 8.5 and 1 μl of 50 mM

chromogenic substrate D-Val-Leu-Lys-p-Nitroanilide dihydrochloride (Cat. No.V7127, Sigma), a synthetic plasmin substrate, were then added to the well and incubated at 25°C for 30 minutes. 31.5 μl of 10 % citric acid was added to each well to stop the reaction. The reteplase-containing proteins catalyze plasminogen to form plasmin, which in turn cleaves the chromogenic substrate to release yellow colored p-Nitroanilide; it was measured at 405 nm by a plate reader.

Figure 19 shows that recombinant 2-chain (reteplase)-hlgG4.Fc, 2-chain (reteplase)-hlgG4.Fc-(scFv α fibrin) and (reteplase)-(scFv α fibrin) showed protease activity as positive control proteins, recombinant reteplase and commercially available tPA protein (Cat. No.T0831, Sigma). The hlgG4.Fc and anti-fibrin 102-10 scFv were used as negative controls. The binding assay shows that both TPA and reteplase bind to fibrin. Moreover, the fusion protein of reteplase and scFv specific for fibrin, in particular the one with the $G_4S$ linker, exhibited better binding activity.

## Claims

**1.** A linker unit comprising a center core, a plurality of linking arms, a plurality of first elements, and optionally a coupling arm, wherein

the center core comprises a first polypeptide comprising a plurality of lysine (K) residues, wherein each K residue and its next K residue are separated by a filler sequence comprising glycine (G) and serine (S) residues, and the number of K residues ranges from 2 to 15;
the plurality of linking arms are respectively linked to the K residues of the center core, wherein each of the linking arms is a PEG chain having 2-20 repeats of EG units;
the plurality of first elements are respectively linked to the plurality of linking arms, wherein each of the first elements is a single-chain variable fragment (scFv) specific for fibrin; and
the amino acid residue at the N- or C-terminus of the center core has an azide or alkyne group; or the amino acid residue at the N- or C-terminus of the center core is a cysteine residue, and the thiol group of the cysteine residue is linked with the coupling arm having an azide, alkyne, tetrazine, cyclooctene, or cyclooctyne group at the free terminus of the coupling arm, the coupling arm is a PEG chain having 2-12 repeats of EG units;
wherein the linker unit is produced by linking the plurality of first elements to the plurality of linking arms via forming an amide bound therebetween, or via thiol-maleimide reaction, copper catalyzed azide-alkyne cycloaddition (CuAAC) reaction, strained-promoted azide-alkyne click chemistry (SPAAC) reaction, or inverse electron demand Diels-Alder (iEDDA) reaction.

**2.** The linker unit of claim 1, wherein
the amino acid residue having the azide group is L-azidohomoalanine (AHA), 4-azido-L-phenylalanine, 4-azido-D-phenylalanine, 3-azido-L-alanine, 3-azido-D-alanine, 4-azido-L-homoalanine, 4-azido-D-homoalanine, 5-azido-L-ornithine, 5-azido-d-ornithine, 6-azido-L-lysine, or 6-azido-D-lysine.

**3.** The linker unit of claim 1, wherein
the amino acid residue having the alkyne group is L-homopropargylglycine (L-HPG), D-homopropargylglycine (D-HPG), or beta-homopropargylglycine (β-HPG).

**4.** The linker unit of claim 1, wherein
the cyclooctene group is trans-cyclooctene (TCO); and the cyclooctyne group is dibenzocyclooctyne (DBCO), difluorinated cyclooctyne (DIFO), bicyclononyne (BCN) or dibenzocyclooctyne (DICO).

**5.** The linker unit of claim 1, wherein
the tetrazine group is 1,2,3,4-tetrazine, 1,2,3,5-tetrazine or 1,2,4,5-tetrazine, or derivatives thereof.

**6.** The linker unit of claim 1, further comprising a second element that is linked to the center core, and the linker unit is produced by linking the second element to the center core via any of the following reactions,

CuAAC reaction occurred between the azide or alkyne group and the second element;
SPAAC reaction occurred between the azide or cyclooctyne group and the second element; and
iEDDA reaction occurred between the cyclooctene or tetrazine group and the second element.

**7.** The linker unit of claim 6, wherein
the second element is a tissue plasminogen activator or an inhibitor of Factor Xa or thrombin.

**8.** The linker unit of claim 7, wherein
the tissue plasminogen activator is alteplase, reteplase, tenecteplase, or lanoteplase.

**9.** The linker unit of claim 7, wherein the inhibitor of Factor Xa is apixaban, edoxaban, or rivaroxaban; and the inhibitor of thrombin is argatroban or melagatran.

**10.** A molecular construct comprising a first linker unit and a second linker unit, wherein

the first linker unit is the linker unit of claim 1;
the second linker unit comprises

a second center core comprising a second polypeptide comprising a plurality of lysine (K) residues, wherein each K residue and its next K residue are separated by a filler sequence comprising glycine (G) and serine (S) residues, and the number of K residues ranges from 2 to 15,
a plurality of second linking arms respectively linked to the K residues of the second center core, wherein each of the second linking arms is a PEG chain having 2-20 repeats of EG units,
a plurality of second elements respectively linked to the plurality of second linking arms, and
optionally, a second coupling arm linked to the second center core, wherein the second coupling arm is a PEG chain having 2-12 repeats of EG units;
wherein the amino acid residue at the N- or C-terminus of the second center core has the azide or the alkyne group; or the amino acid residue at the N- or C-terminus of the second center core is a cysteine residue, and the thiol group of the cysteine residue is linked with the second coupling arm having the azide, the alkyne, the tetrazine, the cyclooctene, or the cyclooctyne group at the free terminus of the second coupling arm; and
the second linker unit is produced by linking the plurality of second elements to the plurality of second linking arms via forming an amide bound therebetween, or via thiol-maleimide reaction, copper catalyzed azide-alkyne cycloaddition (CuAAC) reaction, strained-promoted azide-alkyne click chemistry (SPAAC) reaction, or inverse electron demand Diels-Alder (iEDDA) reaction;
wherein the molecular construct is produced by coupling the first and second linker units via a chemical bond between the azide group, alkyne group, cyclooctene group, cyclooctyne group or tetrazine group of the center core or the coupling arm of the first linker unit and that of the second center core or the second coupling arm of the second linker unit; and
the second element is a tissue plasminogen activator or an inhibitor of Factor Xa or thrombin.

**11.** The molecular construct of claim 10, wherein
the tissue plasminogen activator is alteplase, reteplase, tenecteplase, or lanoteplase.

**12.** The molecular construct of claim 10, wherein

the inhibitor of Factor Xa is apixaban, edoxaban, or rivaroxaban; and
the inhibitor of thrombin is argatroban or melagatran.

## Patentansprüche

**1.** Linker-Einheit, die einen zentralen Kern, eine Vielzahl von Verbindungsarmen, eine Vielzahl von ersten Elementen und optional einen Koppelungsarm umfasst, wobei

der zentrale Kern ein erstes Polypeptid umfasst, das eine Vielzahl von Lysin (K)-Resten umfasst, wobei jeder K-Rest und sein nächster K-Rest durch eine Füllsequenz getrennt sind, die Glycin (G)- und Serin (S)-Reste umfasst, und die Anzahl der K-Reste zwischen 2 und 15 beträgt;
die Vielzahl von Verbindungsarmen jeweils mit den K-Resten des zentralen Kerns verbunden sind, wobei jeder der Verbindungsarme eine PEG-Kette mit 2-20 Wiederholungen von EG-Einheiten ist;
die Vielzahl von ersten Elementen entsprechend mit der Vielzahl von Verbindungsarmen verbunden sind, wobei jedes der ersten Elemente ein einkettiges variables Fragment (scFv) ist, das für Fibrin spezifisch ist; und
der Aminosäurerest an dem N- oder C-Terminus des zentralen Kerns eine Azid- oder Alkingruppe aufweist; oder der Aminosäurerest an dem N- oder C-Terminus des zentralen Kerns ein Cysteinrest ist und die Thiolgruppe des Cysteinrests mit dem Koppelungsarm verbunden ist, der eine Azid-, Alkin-, Tetrazin-, Cycloocten-, oder Cyclooctin-Gruppe an dem freien Ende des Koppelungsarms aufweist, wobei der Koppelungsarm eine PEG-Kette mit 2-12 Wiederholungen von EG-Einheiten ist;
wobei die Linker-Einheit durch Verknüpfung der Vielzahl von ersten Elementen mit der Vielzahl von Verbindungsarmen durch Bildung einer Amidbindung dazwischen, oder über eine Thiol-Maleimid-Reaktion, eine kupferkatalysierte Azid-Alkin-Cycloadditions (CuAAC)-Reaktion, spannungsunterstützte Azid-Alkin-Klick-Chemie (SPAAC)-Reaktion oder eine inverse Elektronenbedarfs-Diels-Alder (iEDDA)-Reaktion hergestellt wird.

**2.** Linker-Einheit nach Anspruch 1, bei welcher
der Aminosäurerest, der die Azid-Gruppe aufweist, L-Azidohomoalanin (AHA), 4-Azido-L-Phenylalanin, 4-Azido-D-Phenylalanin, 3-Azido-L-Alanin, 3-Azido-D-Alanin, 4-Azido-L-Homoalanin, 4-Azido-D-Homoalanin, 5-Azido-L-Ornithin, 5-Azido-D-Ornithin, 6-Azido -L-Lysin oder 6-Azido-D-Lysin ist.

**3.** Linker-Einheit nach Anspruch 1, bei welcher
der Aminosäurerest, der die Alkin-Gruppe aufweist, L-Homopropargylglycin (L-HPG), D-Homopropargylglycin (D-HPG) oder Beta-Homopropargylglycin (β-HPG) ist.

**4.** Linker-Einheit nach Anspruch 1, bei welcher
die Cycloocten-Gruppe trans-Cycloocten (TCO) ist; und die Cyclooctin-Gruppe Dibenzocyclooctin (DBCO), difluoriertes Cyclooctin (DIFO), Bicyclononyn (BCN) oder Dibenzocyclooctin (DICO) ist.

**5.** Linker-Einheit nach Anspruch 1, bei welcher
die Tetrazin-Gruppe 1,2,3,4-Tetrazin, 1,2,3,5-Tetrazin oder 1,2,4,5-Tetrazin oder ein Derivat davon ist.

**6.** Linker-Einheit nach Anspruch 1, welche ferner ein zweites Element umfasst, das mit dem zentralen Kern verbunden ist, und die Linker-Einheit durch Verbinden des zweiten Elements mit dem zentralen Kern über eine der folgenden Reaktionen hergestellt ist,

CuAAC - Reaktion, die zwischen der Azid- oder Alkingruppe und dem zweiten Element stattgefunden hat;
SPAAC - Reaktion, die zwischen der Azid- oder Cyclooctin-Gruppe und dem zweiten Element stattgefunden hat; und
iEDDA - Reaktion, die zwischen der Cycloocten- oder Tetrazin-Gruppe und dem zweiten Element stattgefunden hat.

**7.** Linker-Einheit nach Anspruch 6, bei welcher
das zweite Element ein Gewebeplasminogenaktivator oder ein Inhibitor von Faktor Xa oder Thrombin ist.

**8.** Linker-Einheit nach Anspruch 7, bei welcher
der Gewebeplasminogenaktivator Alteplase, Reteplase, Tenecteplase, oder Lanoteplase ist.

**9.** Linker-Einheit nach Anspruch 7, bei welcher der Inhibitor von Faktor Xa Apixaban, Edoxaban, oder Rivaroxaban ist; und
der Inhibitor von Thrombin Argatroban oder Melagatran ist.

**10.** Molekulares Konstrukt, welches eine erste Linker-Einheit und eine zweite Linker-Einheit umfasst, wobei

die erste Linker-Einheit die Linker-Einheit nach Anspruch 1 ist;
die zweite Linker-Einheit umfasst

einen zweiten zentralen Kern, der ein zweites Polypeptid umfasst, das eine Vielzahl von Lysin (K)-Resten umfasst, wobei jeder K-Rest und sein nächster K-Rest durch eine Füllsequenz getrennt sind, die Glycin (G)- und Serin (S)-Reste umfasst, und die Anzahl der K-Reste zwischen 2 und 15 beträgt,
eine Vielzahl von zweiten Verbindungsarmen, die jeweils mit den K-Resten des zweiten zentralen Kerns verbunden sind, wobei jeder der zweiten Verbindungsarme eine PEG-Kette mit 2-20 Wiederholungen von EG-Einheiten ist,
eine Vielzahl von zweiten Elementen, die entsprechend mit der Vielzahl von zweiten Verbindungsarmen verbunden sind, und
optional, einen zweiten Koppelungsarm, der mit dem zweiten zentralen Kern verbunden ist, wobei der zweite Koppelungsarm eine PEG-Kette mit 2-12 Wiederholungen von EG-Einheiten ist;
wobei der Aminosäurerest an dem N- oder C-Terminus des zweiten zentralen Kerns die Azid- oder Alkingruppe aufweist; oder der Aminosäurerest an dem N- oder C-Terminus des zweiten zentralen Kerns ein Cysteinrest ist und die Thiolgruppe des Cysteinrests mit dem zweiten Koppelungsarm verbunden ist, der die Azid-, die Alkin-, die Tetrazin-, die Cycloocten-, oder die Cyclooctin-Gruppe an dem freien Ende des zweiten Koppelungsarms aufweist; und

die zweite Linker-Einheit durch Verknüpfung der Vielzahl von zweiten Elementen mit der Vielzahl von zweiten Verbindungsarmen durch Bildung einer Amidbindung dazwischen, oder über eine Thiol-Maleimid-Reaktion, kupferkatalysierte Azid-Alkin-Cycloadditions (CuAAC)-Reaktion, spannungsunterstützte Azid-Alkin-Klick-Chemie (SPAAC)-Reaktion oder inverse Elektronenbedarfs-Diels-Alder (iEDDA)-Reaktion hergestellt wird;

wobei das molekulare Konstrukt durch Kopplung der ersten und der zweiten Linker-Einheit mittels einer chemischen Bindung zwischen der Azid-Gruppe, Alkin-Gruppe, Cycloocten-Gruppe, Cyclooctin-Gruppe oder Tetrazin-Gruppe des zentralen Kerns oder des Koppelungsarms der ersten Linker-Einheit und derjenigen des zweiten zentralen Kerns oder des zweiten Koppelungsarms der zweiten Linker-Einheit hergestellt wird; und

das zweite Element ein Gewebeplasminogenaktivator oder ein Inhibitor von Faktor Xa oder Thrombin ist.

**11.** Molekulares Konstrukt nach Anspruch 10, bei welchem
der Gewebeplasminogenaktivator Alteplase, Reteplase, Tenecteplase, oder Lanoteplase ist.

**12.** Molekulares Konstrukt nach Anspruch 10, bei welchem

der Inhibitor von Faktor Xa Apixaban, Edoxaban, oder Rivaroxaban ist; und
der Inhibitor von Thrombin Argatroban oder Melagatran ist.

## Revendications

**1.** Unité de liaison comprenant un noyau central, une pluralité de bras de liaison, une pluralité de premiers éléments et éventuellement un bras de couplage, dans laquelle

le noyau central comprend un premier polypeptide comprenant une pluralité de résidus de lysine (K), dans laquelle chaque résidu K et son résidu K suivant sont séparés par une séquence de remplissage comprenant des résidus de glycine (G) et de sérine (S), et le nombre de résidus K étant compris dans la plage de 2 à 15 ;
la pluralité de bras de liaison sont respectivement liés aux résidus K du noyau central, dans laquelle chacun des bras de liaison est une chaîne PEG présentant 2-20 répétitions de motifs EG ;
la pluralité de premiers éléments sont respectivement liés à la pluralité de bras de liaison, dans laquelle chacun des premiers éléments est un fragment variable à chaîne unique (scFv) spécifique de la fibrine ; et
le résidu d'acide aminé à l'extrémité N ou C terminale du noyau central présente un groupe azoture ou alcyne ; ou
le résidu d'acide aminé à l'extrémité N ou C terminale du noyau central est un résidu de cystéine, et le groupe thiol du résidu de cystéine est lié au bras de couplage présentant un groupe azoture, alcyne, tétrazine, cyclooctène ou cyclooctyne à l'extrémité libre du bras de couplage, le bras de couplage est une chaîne PEG présentant 2-12 répétitions de motifs EG ;
dans laquelle l'unité de liaison est produite en liant la pluralité de premiers éléments à la pluralité de bras de liaison via la formation d'une liaison amide entre ceux-ci, ou via une réaction thiol-maléimide, une réaction de cycloaddition azoture-alcyne catalysée par le cuivre (CuAAC), une réaction de chimie clic azoture-alcyne activée par la contrainte (SPAAC) ou une réaction de Diels-Alder à demande d'électrons inversée (iEDDA).

**2.** Unité de liaison selon la revendication 1, dans laquelle
le résidu d'acide aminé présentant le groupe azoture est L-azidohomoalanine (AHA), 4-azido-L-phénylalanine, 4-azido-D-phénylalanine, 3-azido-L-alanine, 3-azido-D-alanine, 4- azido-L-homoalanine, 4-azido-D-homoalanine, 5-azido-L-ornithine, 5-azido-d-ornithine, 6-azido-L-lysine ou 6-azido-D-lysine.

**3.** Unité de liaison selon la revendication 1, dans laquelle
le résidu d'acide aminé présentant le groupe alcyne est L-homopropargylglycine (L-HPG), D-homopropargylglycine (D-HPG) ou bêta-homopropargylglycine ($\beta$-HPG).

**4.** Unité de liaison selon la revendication 1, dans laquelle
le groupe cyclooctène est trans-cyclooctène (TCO) ; et le groupe cyclooctyne est dibenzocyclooctyne (DBCO),

cyclooctyne difluoré (DIFO), bicyclononyne (BCN) ou dibenzocyclooctyne (DICO).

5. Unité de liaison selon la revendication 1, dans laquelle le groupe tétrazine est 1,2,3,4-tétrazine, 1,2,3,5-tétrazine ou 1,2,4,5-tétrazine, ou des dérivés de celles-ci.

6. Unité de liaison selon la revendication 1, comprenant en outre un second élément qui est lié au noyau central, et l'unité de liaison est produite en liant le second élément au noyau central via l'une quelconque des réactions suivantes,

   une réaction CuAAC ayant eu lieu entre le groupe azoture ou alcyne et le second élément ;
   une réaction SPAAC ayant eu lieu entre le groupe azoture ou cyclooctyne et le second élément ; et
   une réaction iEDDA ayant eu lieu entre le groupe cyclooctène ou tétrazine et le second élément.

7. Unité de liaison selon la revendication 6, dans laquelle le second élément est un activateur tissulaire du plasminogène ou un inhibiteur du facteur Xa ou de la thrombine.

8. Unité de liaison selon la revendication 7, dans laquelle l'activateur tissulaire du plasminogène est l'altéplase, la rétéplase, la ténectéplase ou la lanotéplase.

9. Unité de liaison selon la revendication 7, dans laquelle l'inhibiteur du facteur Xa est l'apixaban, l'édoxaban ou le rivaroxaban ; et
   l'inhibiteur de la thrombine est l'argatroban ou le mélagatran.

10. Construction moléculaire comprenant une première unité de liaison et une seconde unité de liaison, dans laquelle

   la première unité de liaison est l'unité de liaison de la revendication 1 ;
   la seconde unité de liaison comprend

      un second noyau central comprenant un second polypeptide comprenant une pluralité de résidus de lysine (K), dans laquelle chaque résidu K et son résidu K suivant sont séparés par une séquence de remplissage comprenant des résidus de glycine (G) et de sérine (S), et le nombre de résidus K étant compris dans la plage de 2 à 15,
      une pluralité de seconds bras de liaison liés respectivement aux résidus K du second noyau central, dans laquelle chacun des seconds bras de liaison est une chaîne PEG présentant 2-20 répétitions de motifs EG,
      une pluralité de seconds éléments respectivement liés à la pluralité de seconds bras de liaison, et
      éventuellement, un second bras de couplage lié au second noyau central, dans laquelle le second bras de couplage est une chaîne PEG présentant 2-12 répétitions de motifs EG ;
      dans laquelle le résidu d'acide aminé à l'extrémité N ou C terminale du second noyau central présente le groupe azoture ou alcyne ; ou le résidu d'acide aminé à l'extrémité N ou C terminale du second noyau central est un résidu de cystéine, et le groupe thiol du résidu de cystéine est lié au second bras de couplage présentant le groupe azoture, alcyne, tétrazine, cyclooctène ou cyclooctyne à l'extrémité libre du second bras de couplage ; et
      la seconde unité de liaison est produite en liant la pluralité de seconds éléments à la pluralité de seconds bras de liaison via la formation d'une liaison amide entre ceux-ci, ou via une réaction thiol-maléimide, une réaction de cycloaddition azoture-alcyne catalysée par le cuivre (CuAAC), une réaction de chimie clic azoture-alcyne activée par la contrainte (SPAAC) ou une réaction de Diels-Alder à demande d'électrons inversée (iEDDA) ;
      dans laquelle la construction moléculaire est produite en couplant les première et seconde unités de liaison via une liaison chimique entre le groupe azoture, le groupe alcyne, le groupe cyclooctène, le groupe cyclooctyne ou le groupe tétrazine du noyau central ou du bras de couplage de la première unité de liaison et celui du second noyau central ou du second bras de couplage de la seconde unité de liaison ; et
      le second élément est un activateur tissulaire du plasminogène ou un inhibiteur du facteur Xa ou de la thrombine.

11. Construction moléculaire selon la revendication 10, dans laquelle l'activateur tissulaire du plasminogène est l'altéplase, la rétéplase, la ténectéplase ou la lanotéplase.

12. Construction moléculaire selon la revendication 10, dans laquelle

   l'inhibiteur du facteur Xa est l'apixaban, l'édoxaban ou le rivaroxaban ; et

l'inhibiteur de la thrombine est l'argatroban ou le mélagatran.

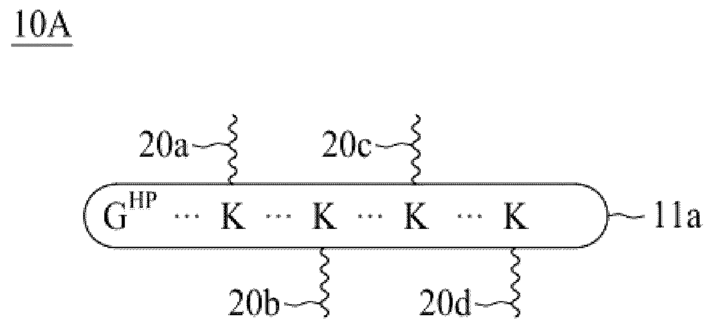


FIG. 1A

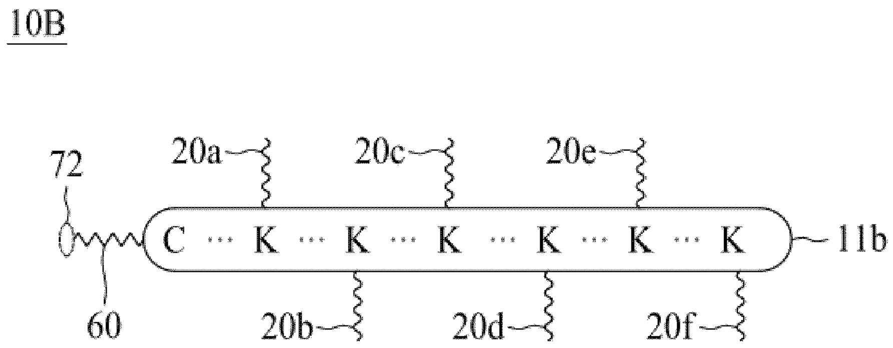


FIG. 1B

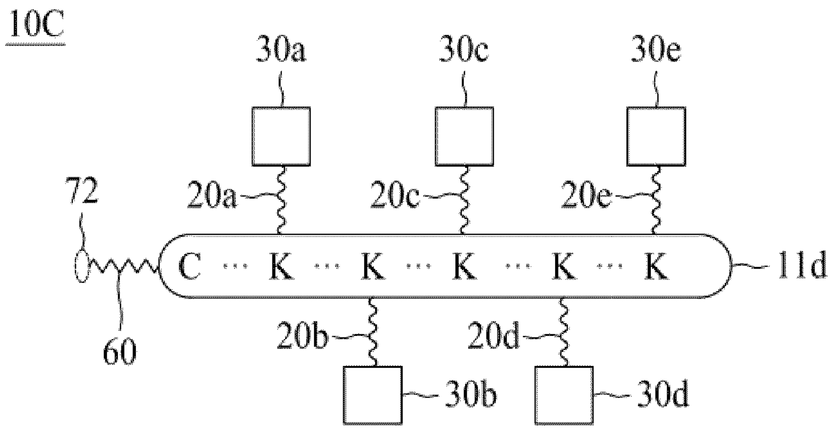


FIG. 1C

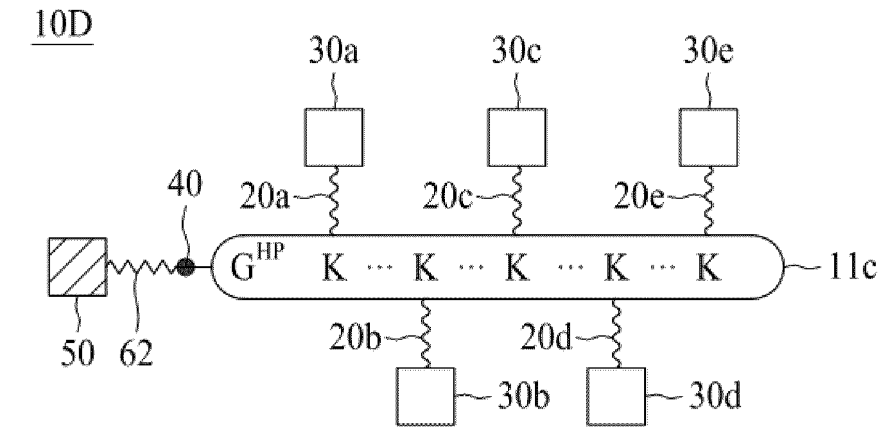


FIG. 1D

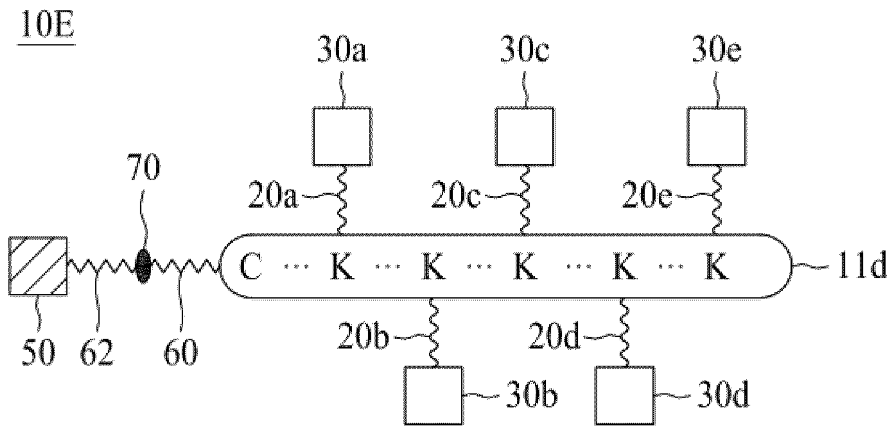


FIG. 1E

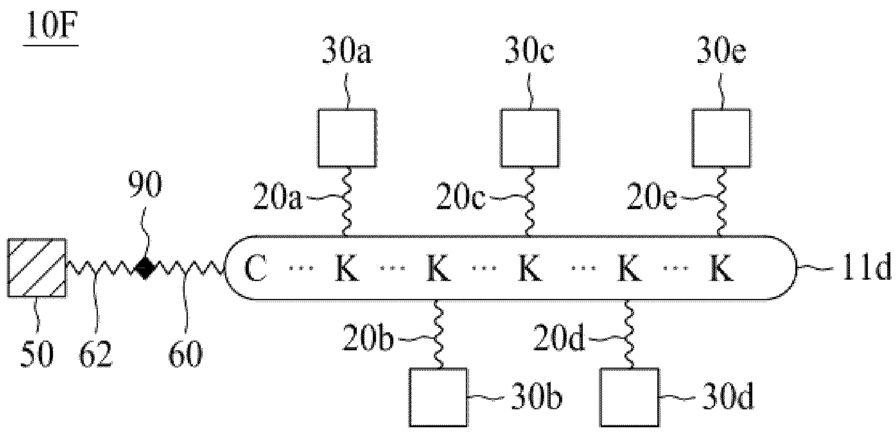


FIG. 1F

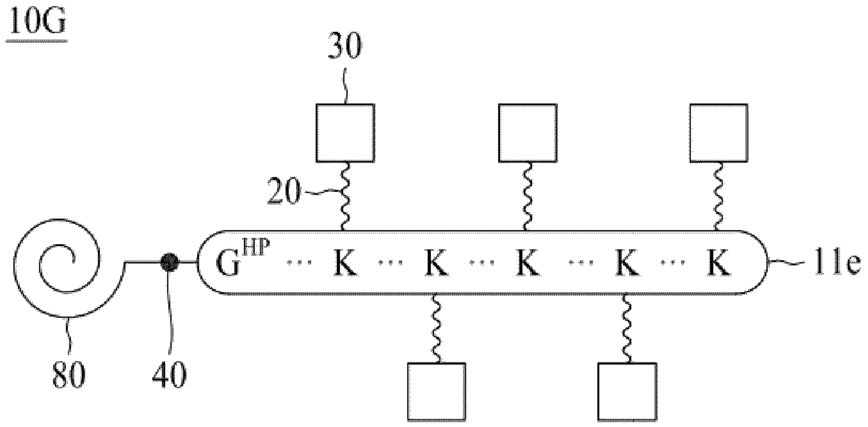


FIG. 1G

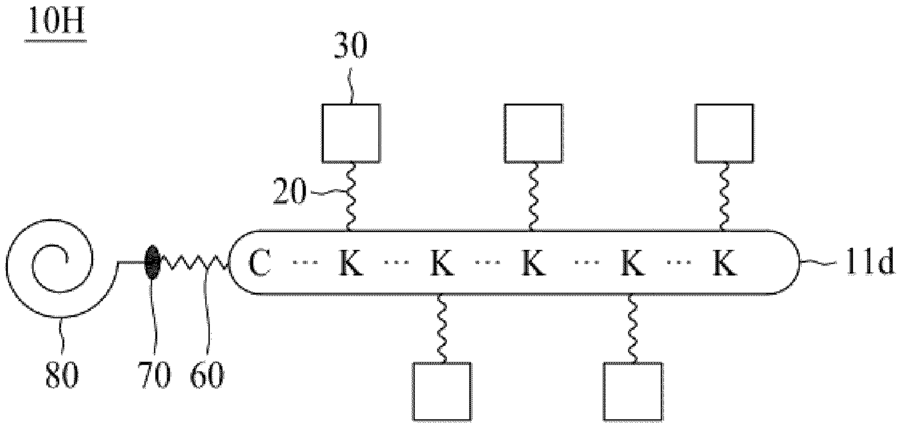


FIG. 1H

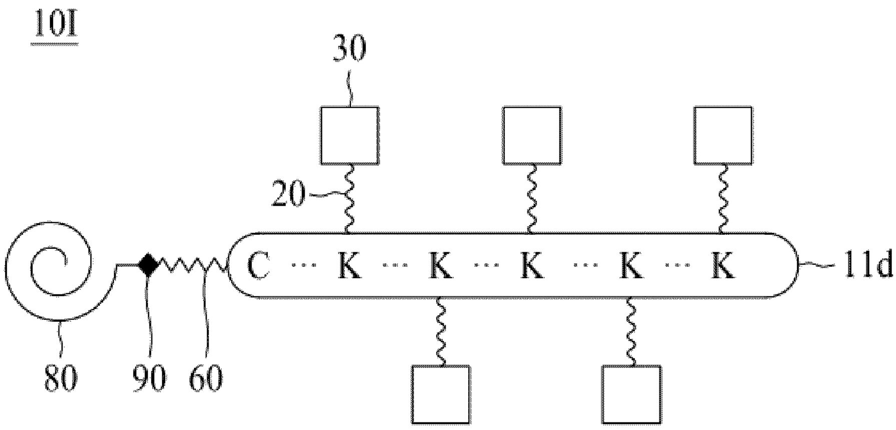


FIG. 1I

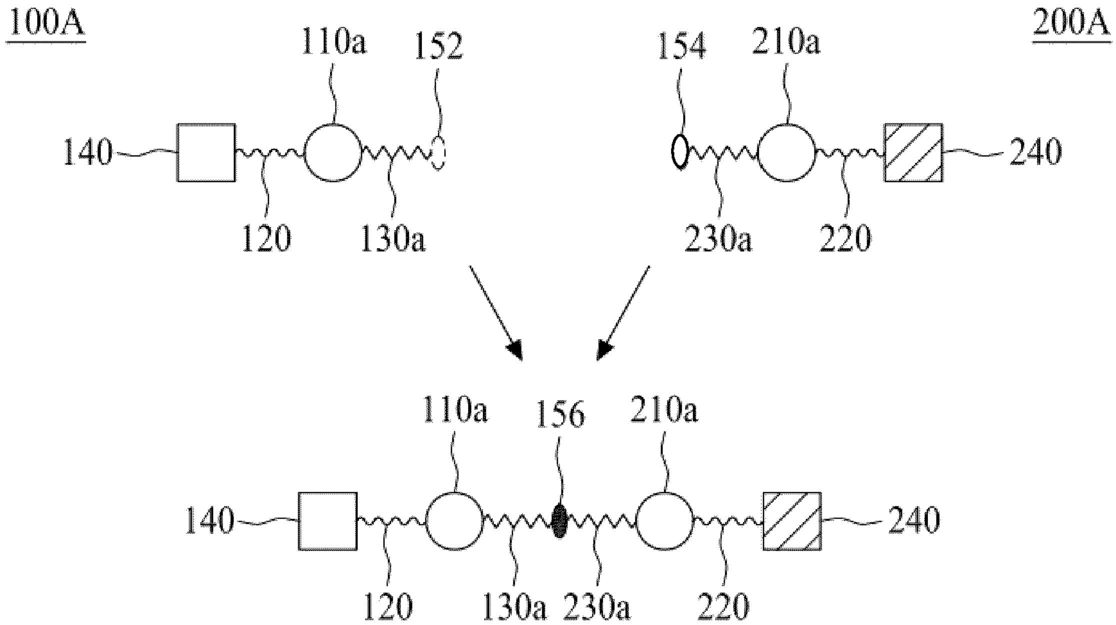
100A
110a
152
154
210a
200A
140
240
120
130a
230a
220
110a
156
210a
140
240
120
130a
230a
220

FIG. 2A

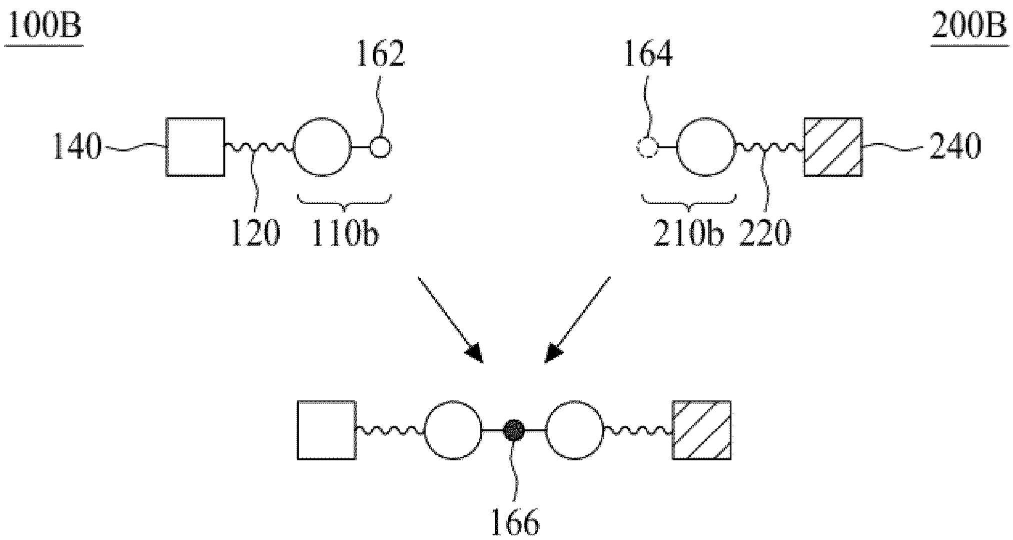
100B
162
164
200B
140
240
120
110b
210b
220
166

FIG. 2B

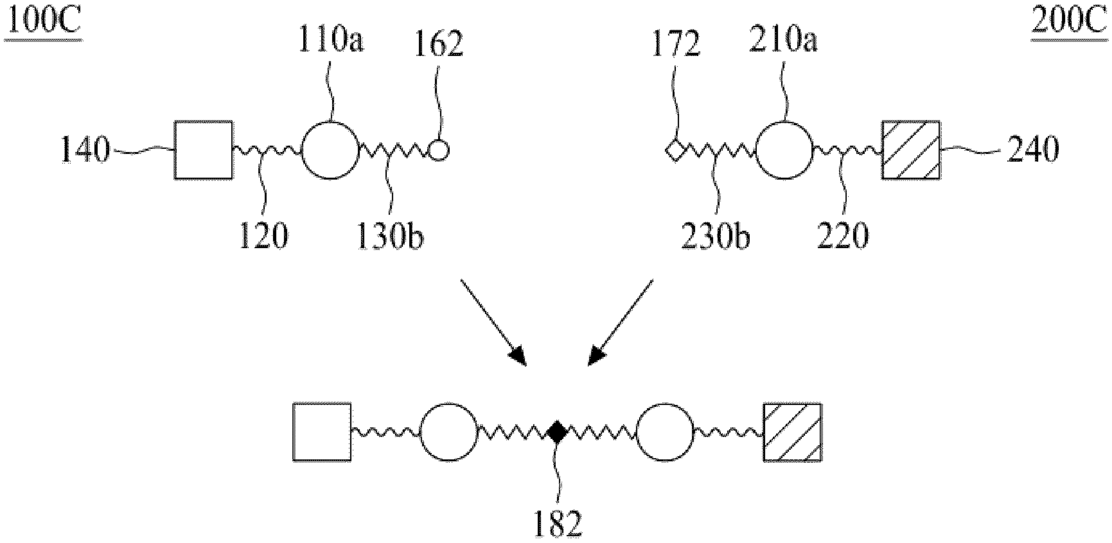
100C
110a
162
172
210a
200C
140
240
120
130b
230b
220
182

FIG. 2C

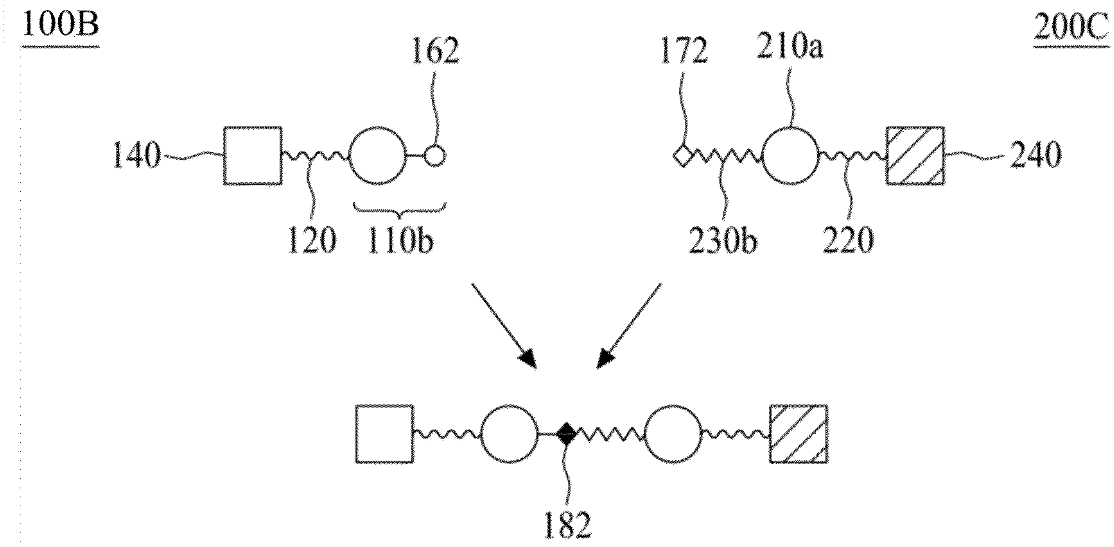
100B
162
172
210a
200C
140
240
120
110b
230b
220
182

FIG. 2D

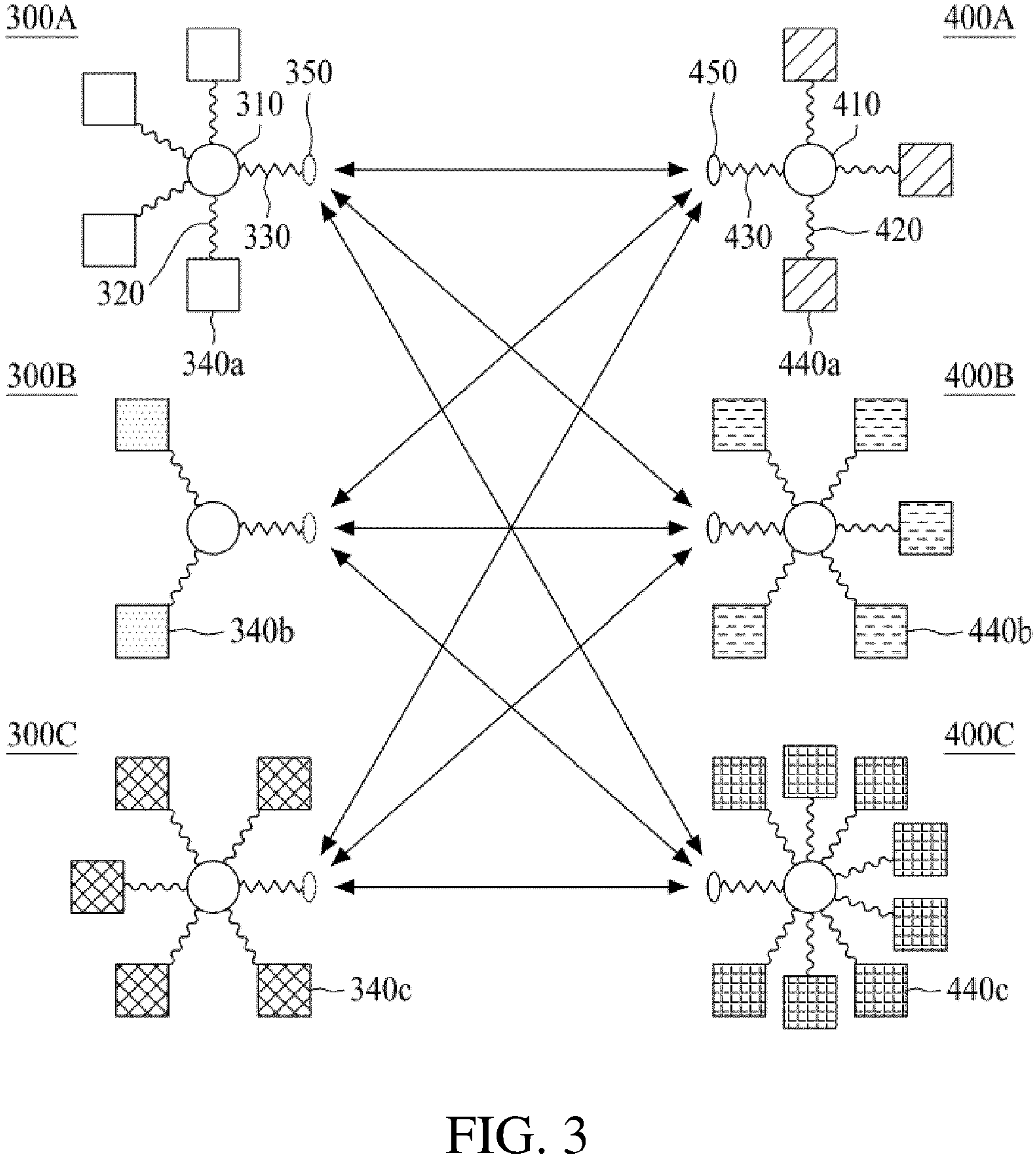
300A
350
310
330
320
340a
400A
450
410
430
420
440a
300B
340b
400B
440b
300C
340c
400C
440c

FIG. 3

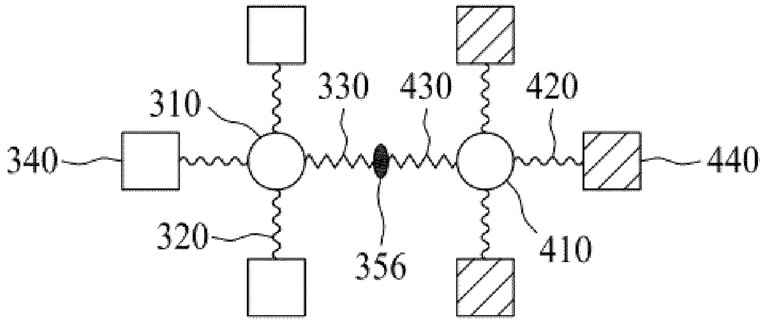
310
320
330
340
356
410
420
430
440

FIG. 4A

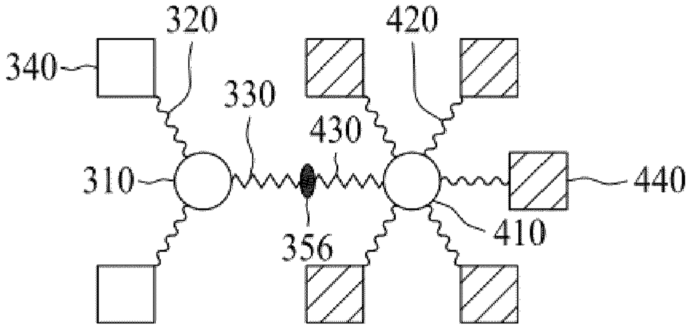
320
340
330
430
420
310
356
410
440

FIG. 4B

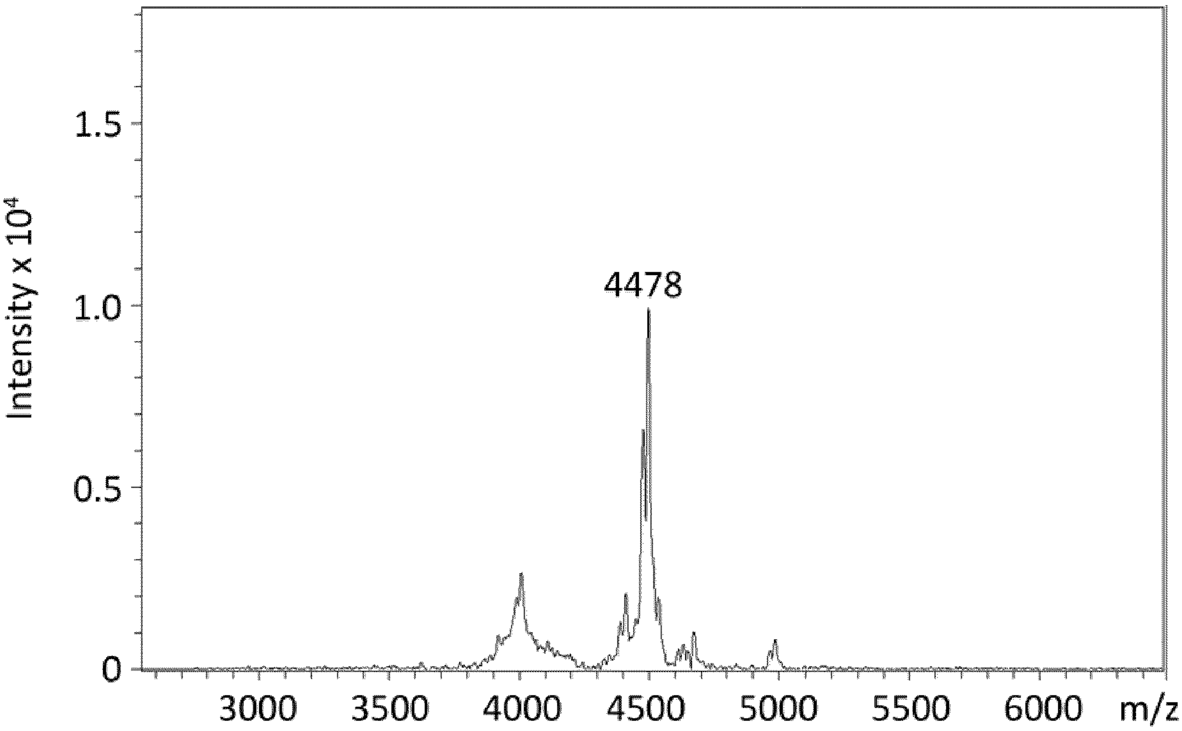
Intensity x 10^4
1.5
1.0
0.5
0
4478
3000
3500
4000
4500
5000
5500
6000
m/z

FIG. 5

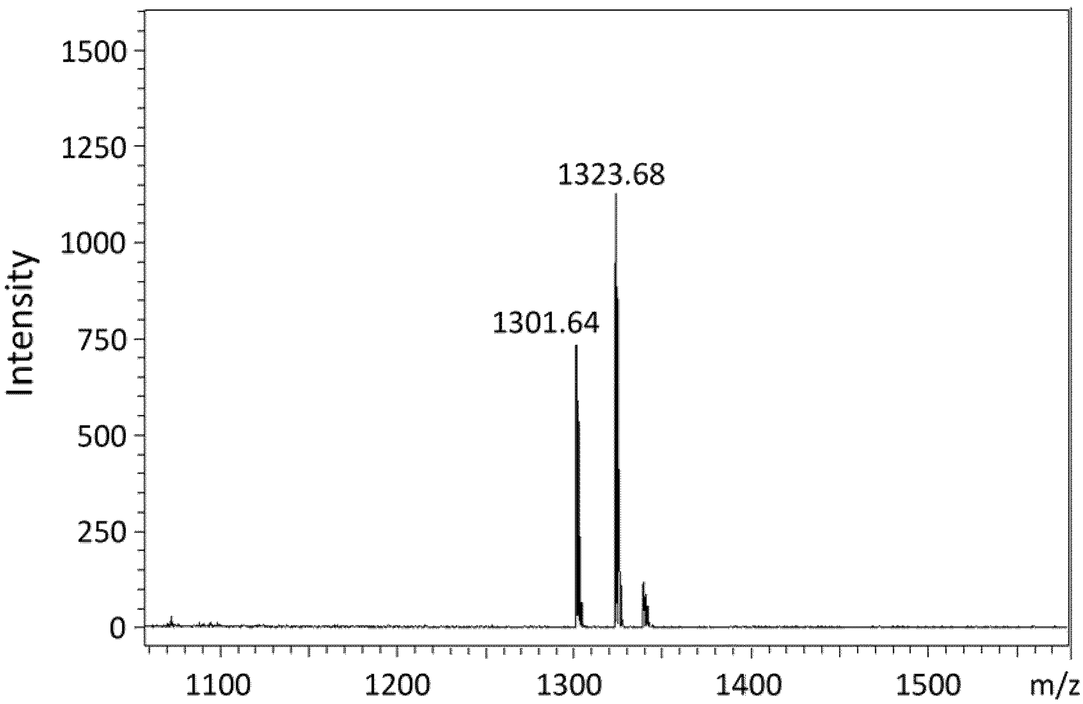
Intensity
1500
1250
1000
750
500
250
0
1323.68
1301.64
1100
1200
1300
1400
1500
m/z

FIG. 6

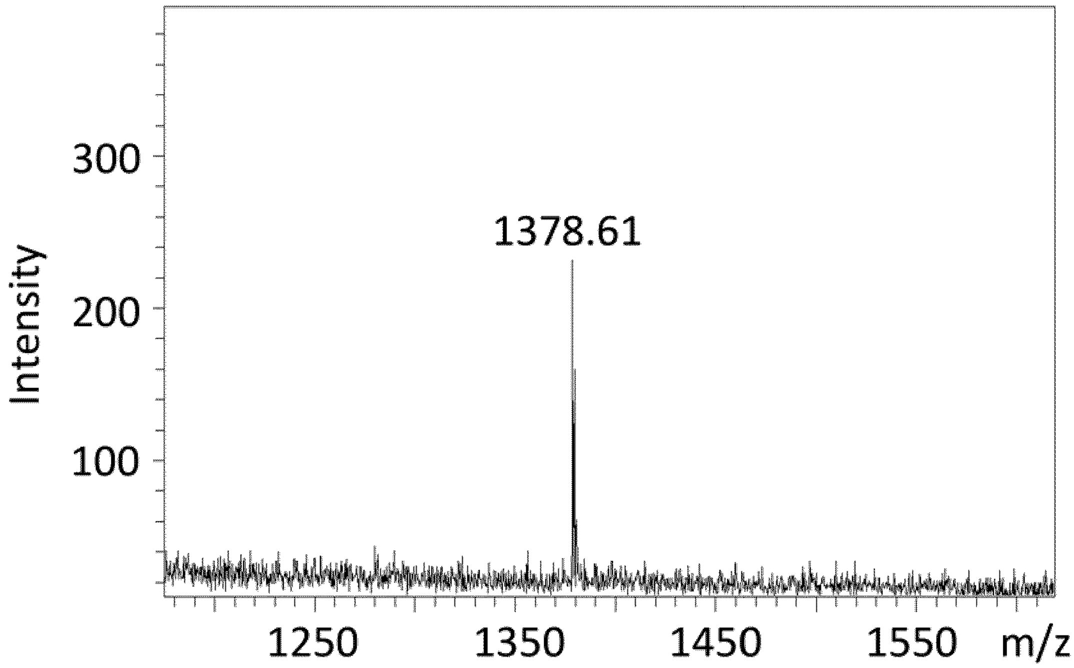
300
200
100
Intensity
1378.61
1250
1350
1450
1550
m/z

FIG. 7

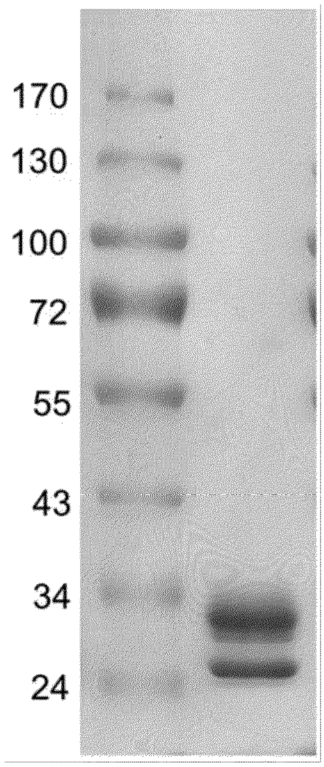
170
130
100
72
55
43
34
24

FIG. 8A

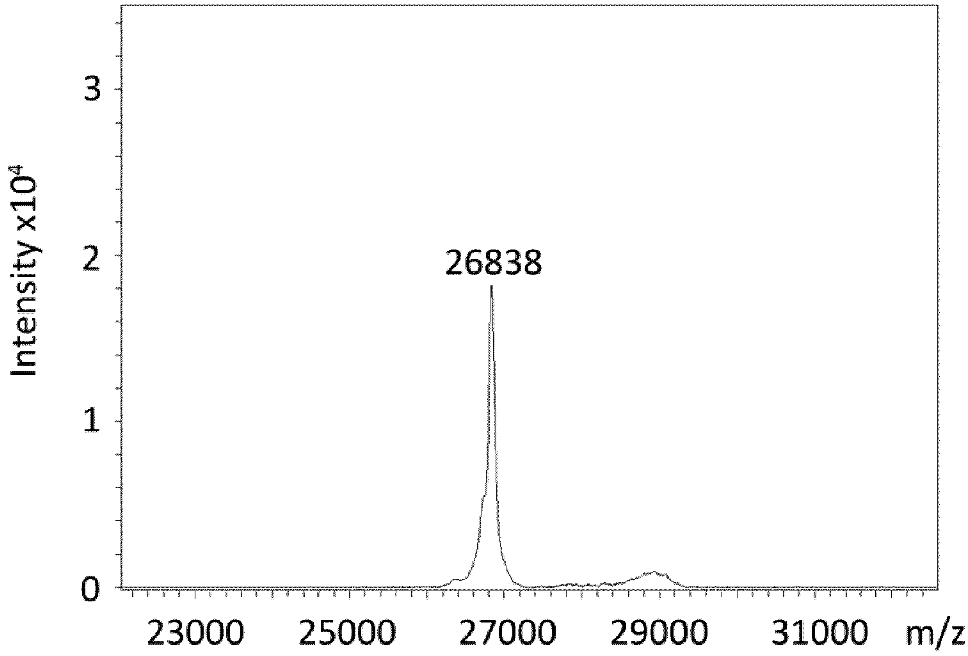
Intensity x10^4
3
2
1
0
26838
23000
25000
27000
29000
31000
m/z

FIG. 8B

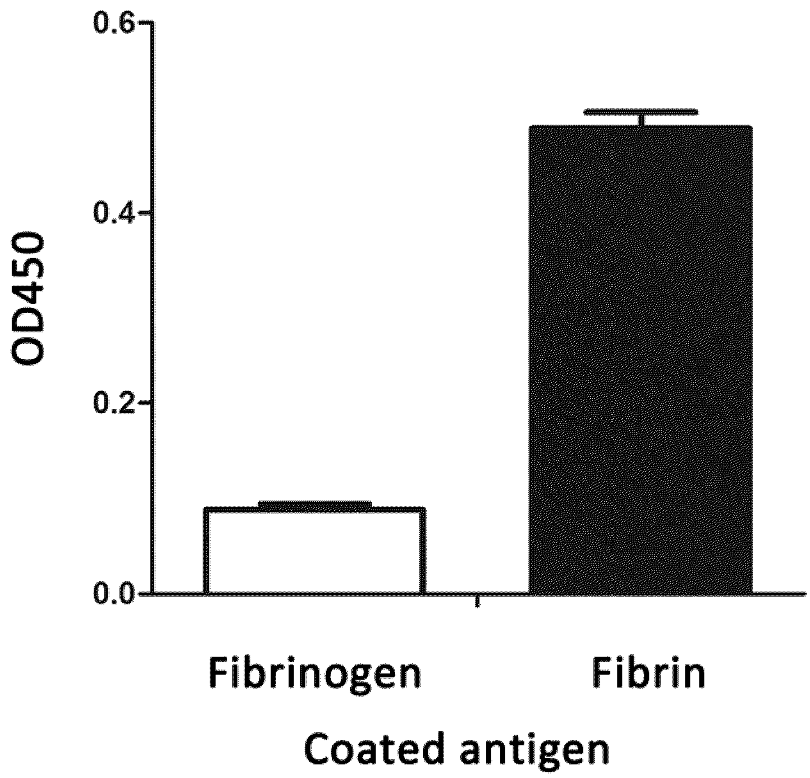
0.6
0.4
0.2
0.0
OD450
Fibrinogen
Fibrin
Coated antigen

FIG. 8C

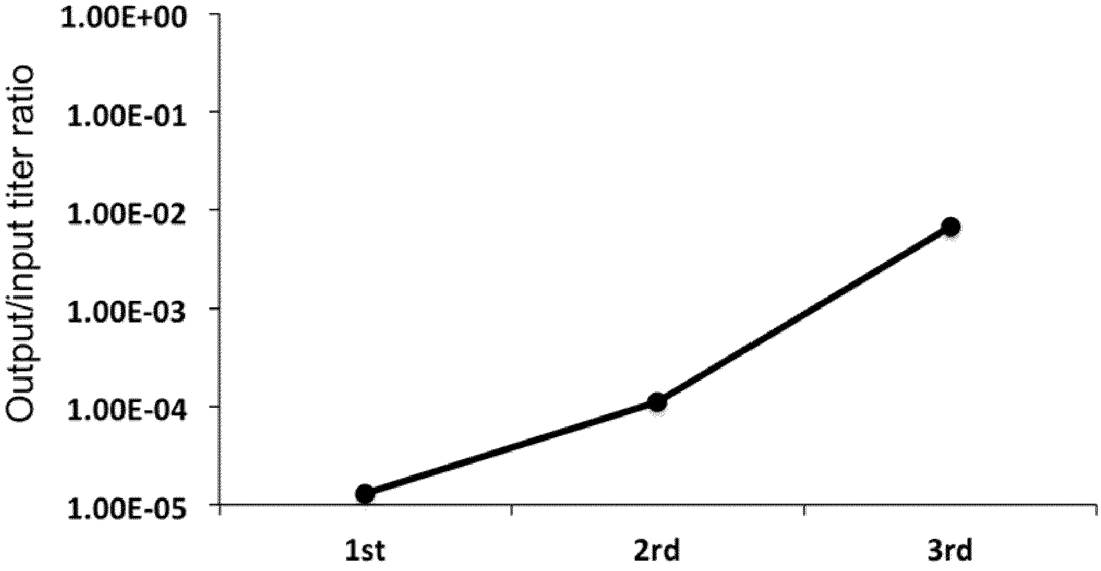
1.00E+00
1.00E-01
1.00E-02
1.00E-03
1.00E-04
1.00E-05
Output/input titer ratio
1st
2rd
3rd

FIG. 9A

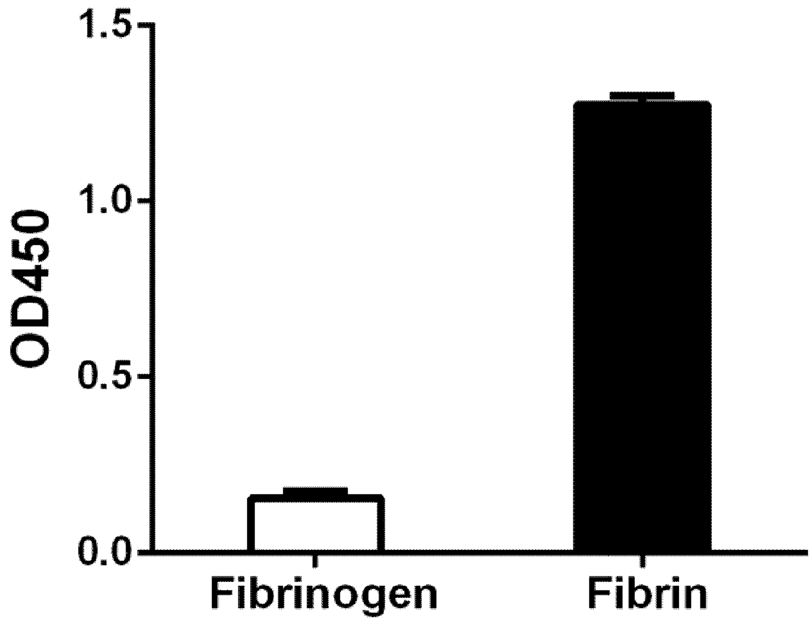
1.5
1.0
0.5
0.0
OD450
Fibrinogen
Fibrin

FIG. 9B

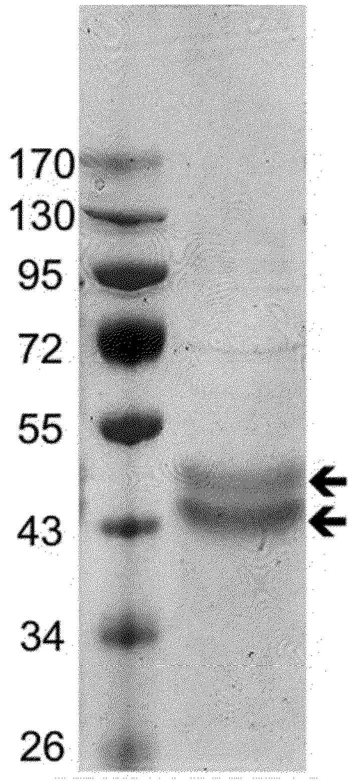


FIG. 10A

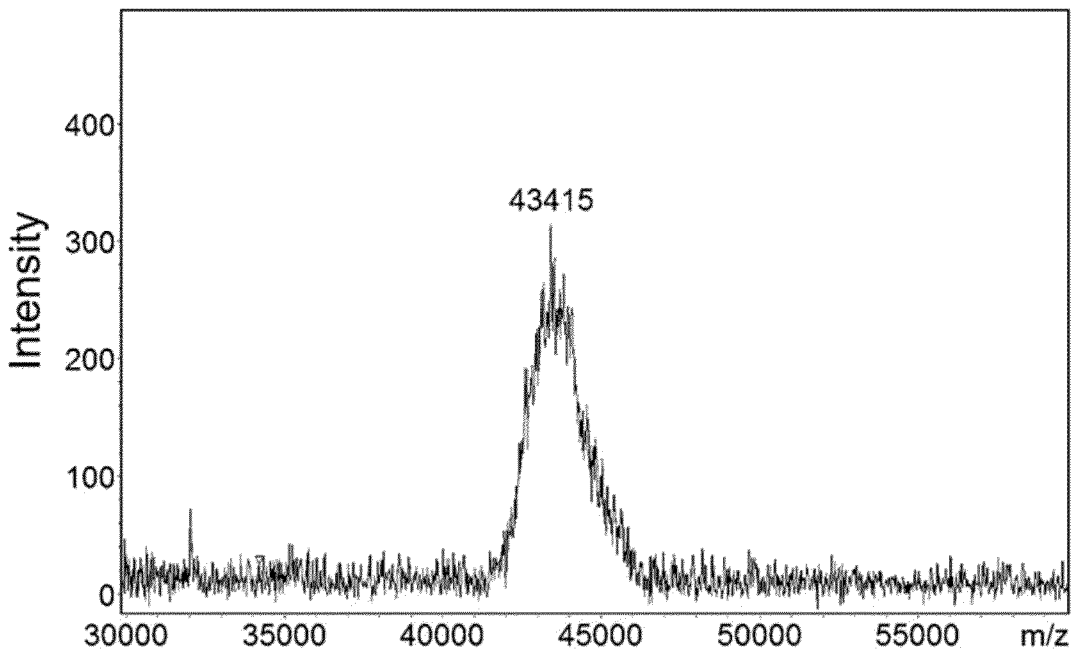


FIG. 10B

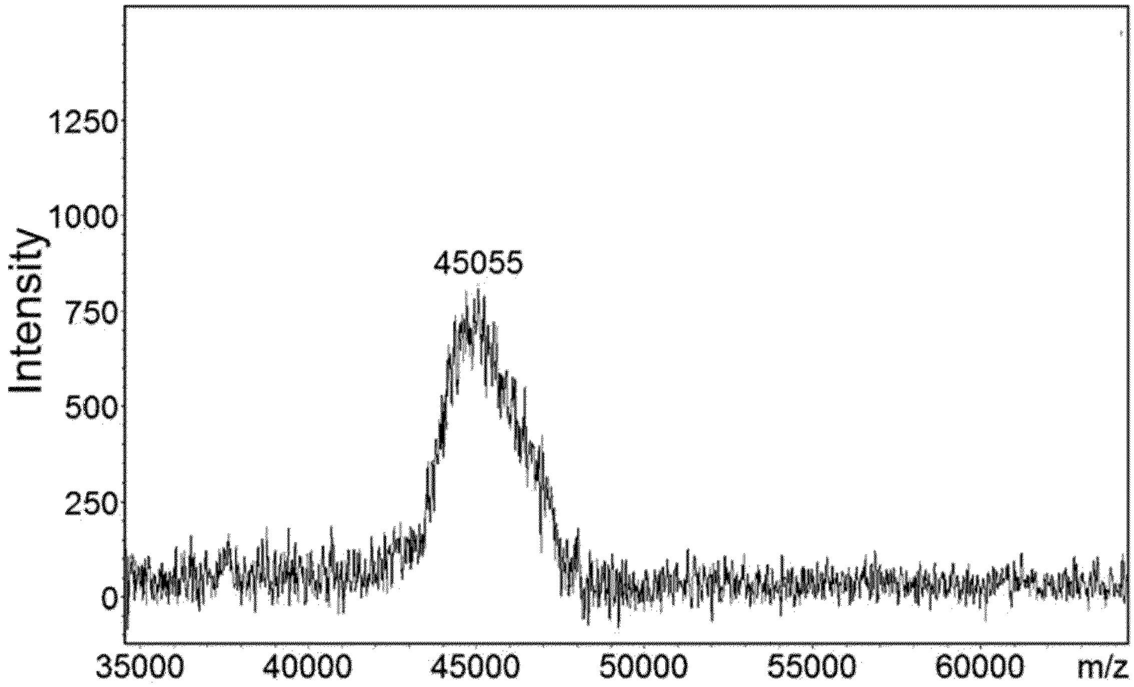
Intensity
1250
1000
750
500
250
0
45055
35000
40000
45000
50000
55000
60000
m/z

FIG. 11

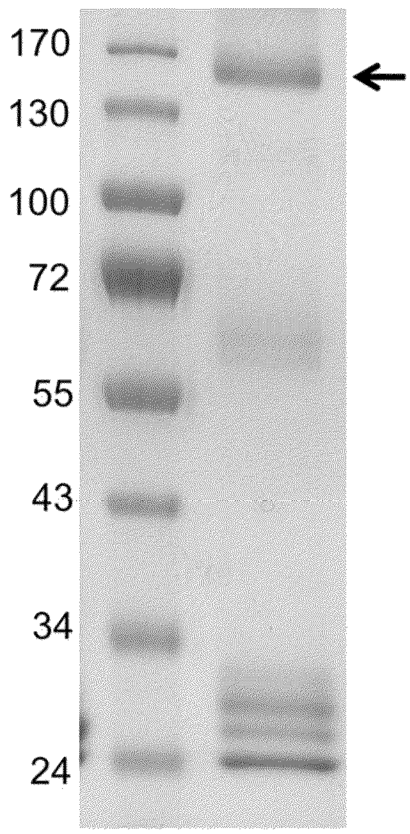
170
130
100
72
55
43
34
24

FIG. 12

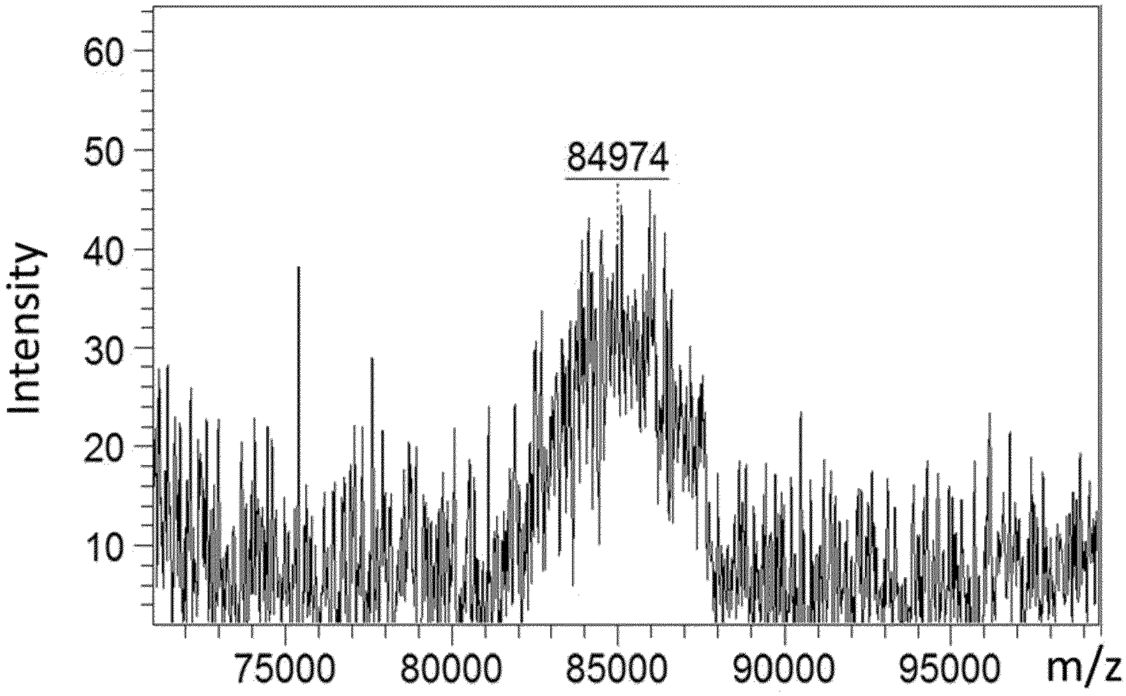
60
50
40
30
20
10
Intensity
84974
75000
80000
85000
90000
95000
m/z

FIG. 13

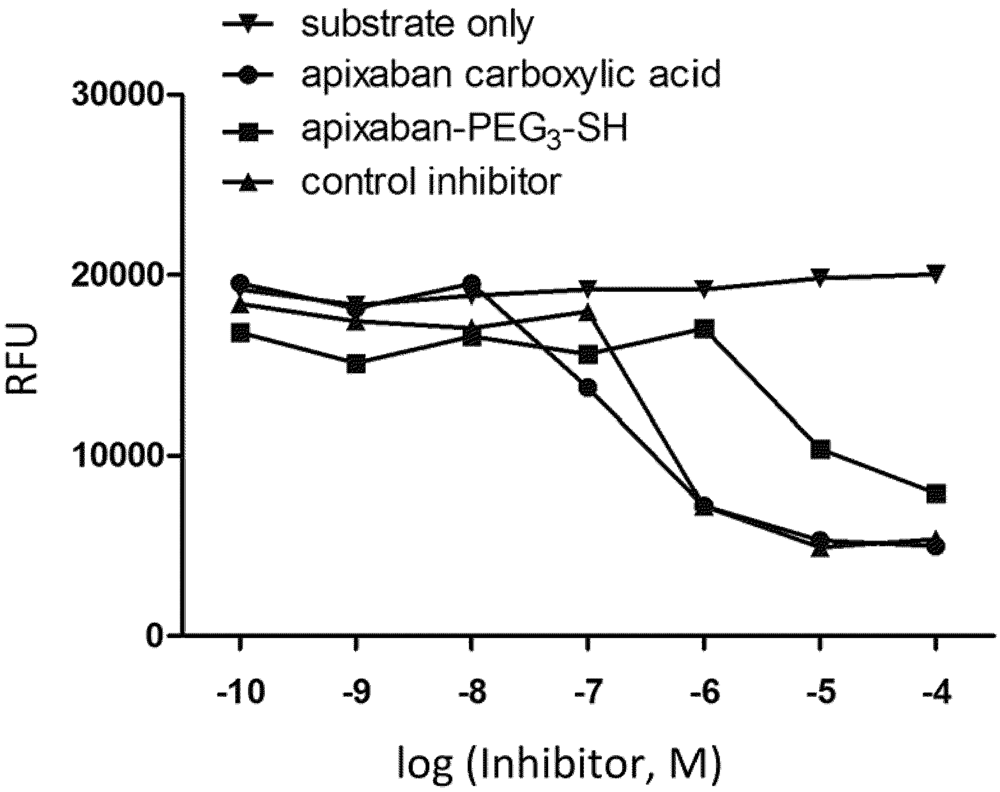
substrate only
apixaban carboxylic acid
apixaban-PEG3-SH
control inhibitor
30000
20000
10000
0
RFU
-10
-9
-8
-7
-6
-5
-4
log (Inhibitor, M)

FIG. 14

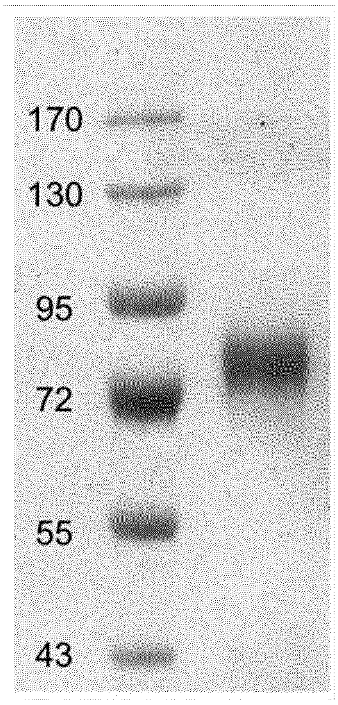


FIG. 15

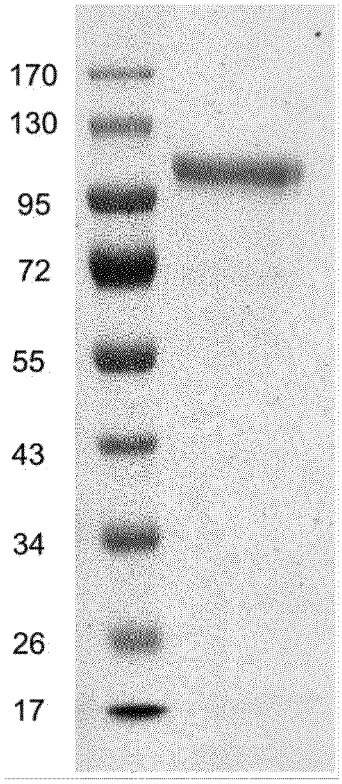


FIG. 16

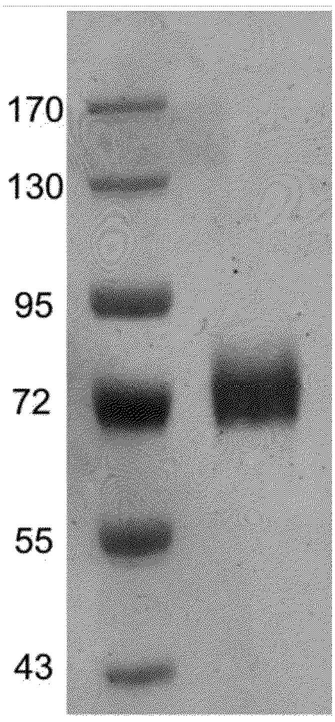


FIG. 17

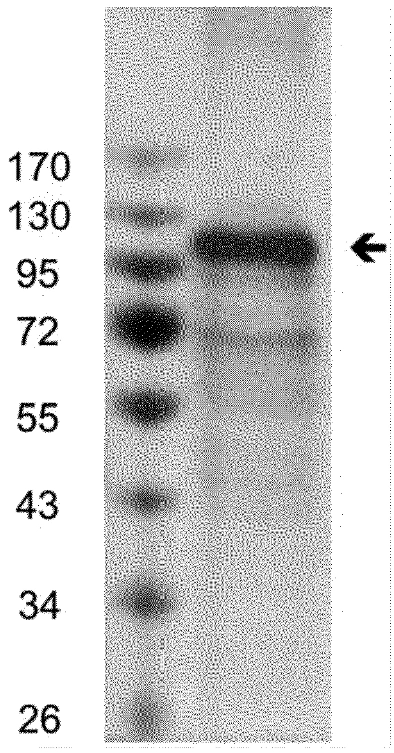


FIG. 18

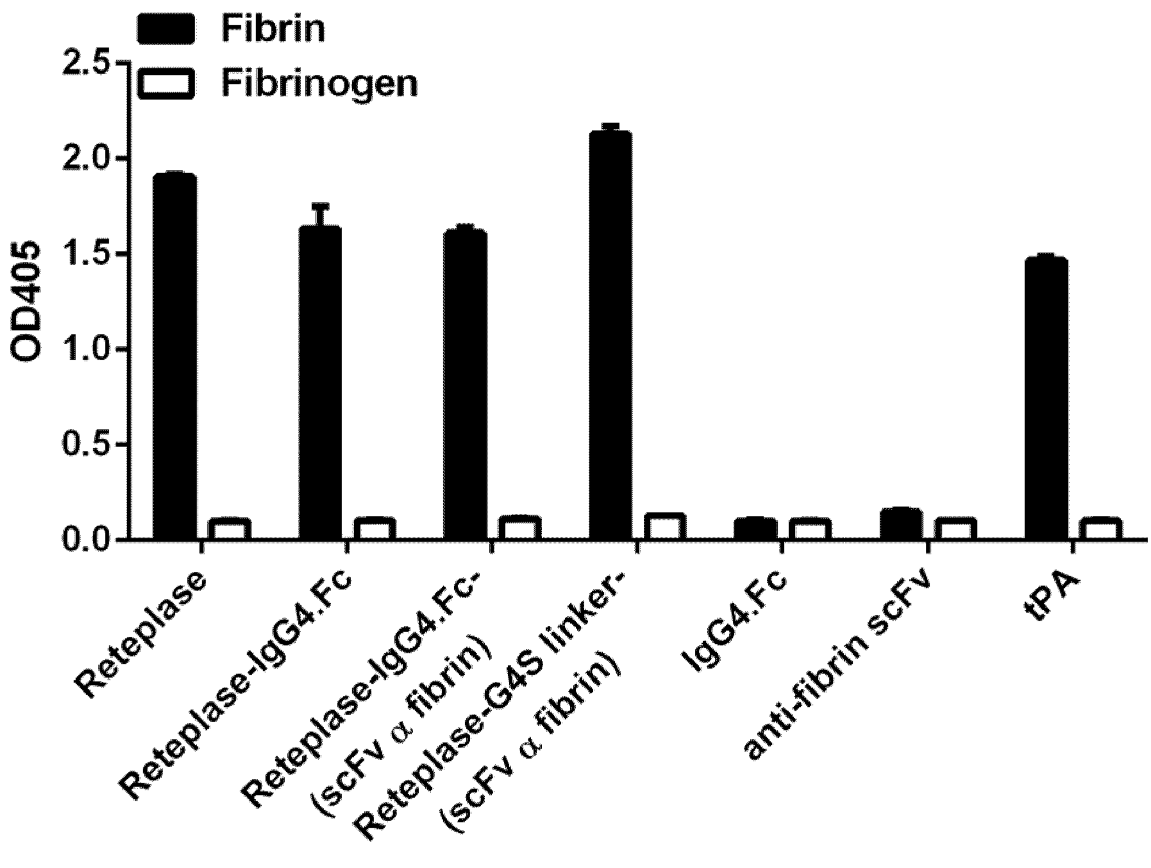
Fibrin
Fibrinogen
OD405
2.5
2.0
1.5
1.0
0.5
0.0
Reteplase
Reteplase-IgG4.Fc
Reteplase-IgG4.Fc-(scFv α fibrin)
Reteplase-G4S linker-(scFv α fibrin)
IgG4.Fc
anti-fibrin scFv
tPA

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011029008 A2
- WO 2011045668 A1
- WO 2008017122 A1
- WO 2013123591 A1
- JP 2012000072 A
- US 20160011217 A1


### Non-patent literature cited in the description


- **CHAUDHARY V K et al.** A rapid method of cloning functional variable-region antibody genes in Escherichia coli as single-chain immunotoxins. *Proc Natl Acad Sci U S A*, February 1990, vol. 87 (3), 1066-1070
- **PETER K et al.** Construction and functional evaluation of a single-chain antibody fusion protein with fibrin targeting and thrombin inhibition after activation by factor Xa. *CIRCULATION*, March 2000, vol. 101, 1158-1164